# EUROPEAN PATENT APPLICATION

(11) **EP 1 688 487 A1**
(43) Date of publication of application: **09.08.2006**
(21) Application number: 04819491.4
(22) Date of filing: 26.11.2004
(51) Int. Cl.: C12N 15/00, A61P 3/10

(54) **METHOD OF CONTROLLING TRANSCRIPTION OF INSULIN GENE**

(30) Priority: 28.11.2003 JP 2003398529
(71) Applicant: DAIICHI PHARMACEUTICAL CO., LTD., Chuo-ku, Tokyo 103-8234 (JP); Celestar Lexico-Sciences, Inc., Chiba-shi, Chiba 261-8501 (JP)
(72) Inventor: DOI, Hirofumi c/o CELESTAR LEXICO-SCIENCES, INC., Mihama-ku, Chiba-shi, Chiba 2618501 (JP); IMAI, Kensaku c/o CELESTAR LEXICO-SCIENCES, INC., Mihama-ku, Chiba-shi, Chiba 2618501 (JP); WADA, Naoya c/o DAIICHI PHARMACEUTICAL CO., LTD., Edogawa-ku, Tokyo 1348630 (JP)
(74) Representative: Sternagel, Fleischer, Godemeyer & Partner
(86) International application number: PCT/JP2004/018068
(87) International publication number: WO 2005/052150

(57) **Abstract**

A method for promoting insulin gene transcription, which comprises the step of inhibiting binding of IPF1 and any one of proteins selected from the following group: (i) HNF3G, (ii) PHF1, and (iii) DLX4; and a method for screening a substance that promotes insulin gene transcription, which comprises the step of bringing a test substance into contact with IPF1 and/or any one of proteins selected from the following group under a condition that allows the binding of IPF1 and said protein and then determining whether or not the test substance inhibits the binding of IPF1 and said protein by detecting presence or absence, or change of a signal and/or a marker generated by the binding of IPF1 and said protein in a system in which the signal and/or the marker can be detected: (i) HNF3G, (ii) PHF1, and (iii) DLX4.

SEQUENCE LISTING

<110> Daiichi Pharmaceutical Co., Ltd.

<120> Method of controlling transcription of insulin gene

<130> P21640EP

<140> EP 04819491.4
<141> 2004-11-26

<150> JP 398529/2003
<151> 2003-11-28

<160> 70

<170> PatentIn Ver. 2.1

<210> 1
<211> 852
<212> DNA
<213> Homo sapiens
<220>
<221> CDS
<222> (1)..(852)
<223>
<400> 1
atg aac ggc gag gag cag tac tac gcg gcc acg cag ctt tac aag gac 48
cca tgc gcg ttc cag cga ggc ccg gcg ccg gag ttc agc gcc agc ccc 96
cct gcg tgc ctg tac atg ggc cgc cag ccc ccg ccg ccg ccg ccg cac 144
ccg ttc cct ggc gcc ctg ggc gcg ctg gag cag ggc agc ccc ccg gac 192
atc tcc ccg tac gag gtg ccc ccc ctc gcc gac gac ccc gcg gtg gcg 240
cac ctt cac cac cac ctc ccg gct cag ctc gcg ctc ccc cac ccg ccc 288
gcc ggg ccc ttc ccg gag gga gcc gag ccg ggc gtc ctg gag gag ccc 336
aac cgc gtc cag ctg cct ttc cca tgg atg aag tct acc aaa gct cac 384
gcg tgg aaa ggc cag tgg gca ggc ggc gcc tac gct gcg gag ccg gag 432
gag aac aag cgg acg cgc acg gcc tac acg cgc gca cag ctg cta gag 480
ctg gag aag gag ttc cta ttc aac aag tac atc tca cgg ccg cgc cgg 528
gtg gag ctg gct gtc atg ttg aac ttg acc gag aga cac atc aag atc 576
tgg ttc caa aac cgc cgc atg aag tgg aaa aag gag gag gac aag aag 624
cgc ggc ggc ggg aca gct gtc ggg ggt ggc ggg gtc gcg gag cct gag 672
cag gac tgc gcc gtg acc tcc ggc gag gag ctt ctg gcg ctg ccg ccg 720
ccg ccg ccc ccc gga ggt gct gtg ccg ccc gct gcc ccc gtt gcc gcc 768
cga gag ggc cgc ctg ccg cct ggc ctt agc gcg tcg cca cag ccc tcc 816
agc gtc gcg cct cgg cgg ccg cag gaa cca cga tga 852

<210> 2
<211> 283
<212> PRT
<213> Homo sapiens
<400> 2

<210>  3
<211>  1053
<212>  DNA
<213>  Homo sapiens
<220>
<221>  CDS
<222>  (1)..(1053)
<223>
<400>  3
atg ctg ggc tca gtg aag atg gag gcc cat gac ctg gcc gag tgg agc       48
Met Leu Gly Ser Val Lys Met Glu Ala His Asp Leu Ala Glu Trp Ser
1               5                   10                  15

tac tac ccg gag gcg ggc gag gtc tac tcg ccg gtg acc cca gtg ccc       96
Tyr Tyr Pro Glu Ala Gly Glu Val Tyr Ser Pro Val Thr Pro Val Pro
            20                  25                  30

acc atg gcc ccc ctc aac tcc tac atg acc ctg aat cct cta agc tct      144
Thr Met Ala Pro Leu Asn Ser Tyr Met Thr Leu Asn Pro Leu Ser Ser
        35                  40                  45

ccc tat ccc cct ggg ggg ctc cct gcc tcc cca ctg ccc tca gga ccc      192
Pro Tyr Pro Pro Gly Gly Leu Pro Ala Ser Pro Leu Pro Ser Gly Pro
    50                  55                  60

ctg gca ccc cca gca cct gca gcc ccc ctg ggg ccc act ttc cca ggc      240
Leu Ala Pro Pro Ala Pro Ala Ala Pro Leu Gly Pro Thr Phe Pro Gly
65                  70                  75                  80

ctg ggt gtc agc ggt ggc agc agc agc tcc ggg tac ggg gcc ccg ggt      288

Leu Gly Val Ser Gly Gly Ser Ser Ser Ser Gly Tyr Gly Ala Pro Gly
                85                  90                  95

cct ggg ctg gtg cac ggg aag gag atg ccg aag ggg tat cgg cgg ccc      336
Pro Gly Leu Val His Gly Lys Glu Met Pro Lys Gly Tyr Arg Arg Pro
            100                 105                 110

ctg gca cac gcc aag cca ccg tat tcc tat atc tca ctc atc acc atg      384
Leu Ala His Ala Lys Pro Pro Tyr Ser Tyr Ile Ser Leu Ile Thr Met
        115                 120                 125

gcc atc cag cag gcg ccg ggc aag atg ctg acc ttg agt gaa atc tac      432
Ala Ile Gln Gln Ala Pro Gly Lys Met Leu Thr Leu Ser Glu Ile Tyr
    130                 135                 140

cag tgg atc atg gac ctc ttc cct tac tac cgg gag aat cag cag cgc      480
Gln Trp Ile Met Asp Leu Phe Pro Tyr Tyr Arg Glu Asn Gln Gln Arg
145                 150                 155                 160

tgg cag aac tcc att cgc cac tcg ctg tct ttc aac gac tgc ttc gtc      528
Trp Gln Asn Ser Ile Arg His Ser Leu Ser Phe Asn Asp Cys Phe Val
                165                 170                 175

aag gtg gcg cgt tcc cca gac aag cct ggc aag ggc tcc tac tgg gcc      576
Lys Val Ala Arg Ser Pro Asp Lys Pro Gly Lys Gly Ser Tyr Trp Ala
            180                 185                 190

cta cac ccc agc tca ggg aac atg ttt gag aat ggc tgc tac ctg cgc      624
Leu His Pro Ser Ser Gly Asn Met Phe Glu Asn Gly Cys Tyr Leu Arg
        195                 200                 205

cgc cag aaa cgc ttc aag ctg gag gag aag gtg aaa aaa ggg ggc agc      672
Arg Gln Lys Arg Phe Lys Leu Glu Glu Lys Val Lys Lys Gly Gly Ser
    210                 215                 220

ggg gct gcc acc acc acc agg aac ggg aca ggg tct gct gcc tcg acc      720
Gly Ala Ala Thr Thr Thr Arg Asn Gly Thr Gly Ser Ala Ala Ser Thr
225                 230                 235                 240

acc acc ccc gcg gcc aca gtc acc tcc ccg ccc cag ccc ccg cct cca      768
Thr Thr Pro Ala Ala Thr Val Thr Ser Pro Pro Gln Pro Pro Pro Pro
                245                 250                 255

gcc cct gag cct gag gcc cag ggc ggg gaa gat gtg ggg gct ctg gac      816
Ala Pro Glu Pro Glu Ala Gln Gly Gly Glu Asp Val Gly Ala Leu Asp
            260                 265                 270

tgt ggc tca ccc gct tcc tcc aca ccc tat ttc act ggc ctg gag ctc      864
Cys Gly Ser Pro Ala Ser Ser Thr Pro Tyr Phe Thr Gly Leu Glu Leu
        275                 280                 285

cca ggg gag ctg aag ctg gac gcg ccc tac aac ttc aac cac cct ttc      912
Pro Gly Glu Leu Lys Leu Asp Ala Pro Tyr Asn Phe Asn His Pro Phe
    290                 295                 300

tcc atc aac aac cta atg tca gaa cag aca cca gca cct ccc aaa ctg      960
Ser Ile Asn Asn Leu Met Ser Glu Gln Thr Pro Ala Pro Pro Lys Leu
305                 310                 315                 320

gac gtg ggg ttt ggg ggc tac ggg gct gaa ggt ggg gag cct gga gtc     1008
Asp Val Gly Phe Gly Gly Tyr Gly Ala Glu Gly Gly Glu Pro Gly Val
                325                 330                 335

tac tac cag ggc ctc tat tcc cgc tct ttg ctt aat gca tcc tag          1053
Tyr Tyr Gln Gly Leu Tyr Ser Arg Ser Leu Leu Asn Ala Ser
            340                 345                 350

<210>  4
<211>  350
<212>  PRT
<213>  Homo sapiens
<400>  4

Met Leu Gly Ser Val Lys Met Glu Ala His Asp Leu Ala Glu Trp Ser
1               5                   10                  15

Tyr Tyr Pro Glu Ala Gly Glu Val Tyr Ser Pro Val Thr Pro Val Pro
            20                  25                  30

Thr Met Ala Pro Leu Asn Ser Tyr Met Thr Leu Asn Pro Leu Ser Ser
        35                  40                  45

Pro Tyr Pro Pro Gly Gly Leu Pro Ala Ser Pro Leu Pro Ser Gly Pro
    50                  55                  60

Leu Ala Pro Pro Ala Pro Ala Ala Pro Leu Gly Pro Thr Phe Pro Gly
65                  70                  75                  80

Leu Gly Val Ser Gly Gly Ser Ser Ser Ser Gly Tyr Gly Ala Pro Gly
                85                  90                  95

Pro Gly Leu Val His Gly Lys Glu Met Pro Lys Gly Tyr Arg Arg Pro
            100                 105                 110

Leu Ala His Ala Lys Pro Pro Tyr Ser Tyr Ile Ser Leu Ile Thr Met
        115                 120                 125

Ala Ile Gln Gln Ala Pro Gly Lys Met Leu Thr Leu Ser Glu Ile Tyr
    130                 135                 140

Gln Trp Ile Met Asp Leu Phe Pro Tyr Tyr Arg Glu Asn Gln Gln Arg
145                 150                 155                 160

Trp Gln Asn Ser Ile Arg His Ser Leu Ser Phe Asn Asp Cys Phe Val
                165                 170                 175

Lys Val Ala Arg Ser Pro Asp Lys Pro Gly Lys Gly Ser Tyr Trp Ala
            180                 185                 190

Leu His Pro Ser Ser Gly Asn Met Phe Glu Asn Gly Cys Tyr Leu Arg
        195                 200                 205

Arg Gln Lys Arg Phe Lys Leu Glu Glu Lys Val Lys Lys Gly Gly Ser
    210                 215                 220

Gly Ala Ala Thr Thr Thr Arg Asn Gly Thr Gly Ser Ala Ala Ser Thr
225                 230                 235                 240

Thr Thr Pro Ala Ala Thr Val Thr Ser Pro Pro Gln Pro Pro Pro Pro
                245                 250                 255

Ala Pro Glu Pro Glu Ala Gln Gly Gly Glu Asp Val Gly Ala Leu Asp
            260                 265                 270

Cys Gly Ser Pro Ala Ser Ser Thr Pro Tyr Phe Thr Gly Leu Glu Leu
        275                 280                 285

Pro Gly Glu Leu Lys Leu Asp Ala Pro Tyr Asn Phe Asn His Pro Phe
    290                 295                 300

Ser Ile Asn Asn Leu Met Ser Glu Gln Thr Pro Ala Pro Pro Lys Leu
305                 310                 315                 320

Asp Val Gly Phe Gly Gly Tyr Gly Ala Glu Gly Gly Glu Pro Gly Val
                325                 330                 335

Tyr Tyr Gln Gly Leu Tyr Ser Arg Ser Leu Leu Asn Ala Ser
            340                 345                 350

<210>  5
<211>  1704
<212>  DNA
<213>  Homo sapiens
<220>
<221>  CDS
<222>  (1)..(1704)
<223>
<400>  5
atg gcg cag ccc ccc cgg ctg agc cgc tct ggt gcc tcc tca ctt tgg      48
Met Ala Gln Pro Pro Arg Leu Ser Arg Ser Gly Ala Ser Ser Leu Trp
1               5                   10                  15

gac cca gct tct cct gct ccc acc tct ggc ccc agg cct cgg ctt tgg      96
Asp Pro Ala Ser Pro Ala Pro Thr Ser Gly Pro Arg Pro Arg Leu Trp
            20                  25                  30

gag ggt caa gat gtg ctg gcc aga tgg act gat ggg ctg cta tac ttg      144
Glu Gly Gln Asp Val Leu Ala Arg Trp Thr Asp Gly Leu Leu Tyr Leu
        35                  40                  45

ggt acc atc aaa aag gtg gac agt gct agg gag gtg tgt ctg gtc cag      192
Gly Thr Ile Lys Lys Val Asp Ser Ala Arg Glu Val Cys Leu Val Gln
    50                  55                  60

ttt gag gat gat tcg cag ttt ctg gtt cta tgg aaa gac att agc cct      240
Phe Glu Asp Asp Ser Gln Phe Leu Val Leu Trp Lys Asp Ile Ser Pro
65                  70                  75                  80

gct gcc ctc cct gga gag gaa ctc ctc tgt tgt gtc tgt cgc tct gag      288
Ala Ala Leu Pro Gly Glu Glu Leu Leu Cys Cys Val Cys Arg Ser Glu
                85                  90                  95

act gtg gtc cct ggg aac cgg ttg gtc agc tgt gag aag tgt cgc cat      336
Thr Val Val Pro Gly Asn Arg Leu Val Ser Cys Glu Lys Cys Arg His
            100                 105                 110

gct tat cac cag gac tgc cat gtt ccc agg gct cca gcc cct gga gag      384
Ala Tyr His Gln Asp Cys His Val Pro Arg Ala Pro Ala Pro Gly Glu
        115                 120                 125

gga gag ggc aca tcc tgg gta tgc cgc cag tgt gtc ttt gcg atc gcc      432
Gly Glu Gly Thr Ser Trp Val Cys Arg Gln Cys Val Phe Ala Ile Ala
    130                 135                 140

acc aag agg gga ggt gcc ctg aag aag ggc ccc tat gcc cgg gcc atg      480
Thr Lys Arg Gly Gly Ala Leu Lys Lys Gly Pro Tyr Ala Arg Ala Met
145                 150                 155                 160

ctg ggt atg aag ctt tct ctg cca tat gga ctg aag ggg ctg gac tgg      528

Leu Gly Met Lys Leu Ser Leu Pro Tyr Gly Leu Lys Gly Leu Asp Trp
                165                 170                 175

gat gct gga cat ctg agc aac cga cag cag agt tac tgt tac tgt ggt 576
Asp Ala Gly His Leu Ser Asn Arg Gln Gln Ser Tyr Cys Tyr Cys Gly
            180                 185                 190

ggc cct ggg gag tgg aac ctg aaa atg ctg cag tgc cgg agc tgc ctg 624
Gly Pro Gly Glu Trp Asn Leu Lys Met Leu Gln Cys Arg Ser Cys Leu
        195                 200                 205

cag tgg ttc cat gag gcc tgc acc cag tgt ctg agc aag ccc ctc ctc 672
Gln Trp Phe His Glu Ala Cys Thr Gln Cys Leu Ser Lys Pro Leu Leu
    210                 215                 220

tat ggg gac agg ttc tat gaa ttt gaa tgc tgt gtg tgt cgc ggg ggc 720
Tyr Gly Asp Arg Phe Tyr Glu Phe Glu Cys Cys Val Cys Arg Gly Gly
225                 230                 235                 240

cct gag aaa gtc cgg aga cta cag ctt cgc tgg gtg gat gtg gcc cat 768
Pro Glu Lys Val Arg Arg Leu Gln Leu Arg Trp Val Asp Val Ala His
                245                 250                 255

ctt gtc ctg tat cac ctc agt gtt tgc tgt aag aag aaa tac ttt gat 816
Leu Val Leu Tyr His Leu Ser Val Cys Cys Lys Lys Lys Tyr Phe Asp
            260                 265                 270

ttt gat cgt gag atc ctc ccc ttc act tct gag aat tgg gac agt ttg 864
Phe Asp Arg Glu Ile Leu Pro Phe Thr Ser Glu Asn Trp Asp Ser Leu
        275                 280                 285

ctc ctg ggg gag ctt tca gac acc ccc aaa gga gaa cgt tct tcc aag 912
Leu Leu Gly Glu Leu Ser Asp Thr Pro Lys Gly Glu Arg Ser Ser Lys
    290                 295                 300

ctc ctc tct gct ctt aac agc cac aag gac cgt ttc att tca ggg aga 960
Leu Leu Ser Ala Leu Asn Ser His Lys Asp Arg Phe Ile Ser Gly Arg
305                 310                 315                 320

gag att aag aag agg aaa tgt ttg ttt ggt ctc cat gct cgg atg cct 1008
Glu Ile Lys Lys Arg Lys Cys Leu Phe Gly Leu His Ala Arg Met Pro
                325                 330                 335

ccc cct gtg gag ccc cct act gga gat gga gca ctc acc agc ttc cct 1056
Pro Pro Val Glu Pro Pro Thr Gly Asp Gly Ala Leu Thr Ser Phe Pro
            340                 345                 350

tca ggg cag ggc cct ggg gga ggg gtc tca cgt ccc ctg ggg aag cgc 1104
Ser Gly Gln Gly Pro Gly Gly Gly Val Ser Arg Pro Leu Gly Lys Arg
        355                 360                 365

cgg agg ccg gag cca gag ccc ctg agg agg agg cag aag ggg aaa gtg 1152
Arg Arg Pro Glu Pro Glu Pro Leu Arg Arg Arg Gln Lys Gly Lys Val
    370                 375                 380

gag gag ctg ggg cca ccc tca gca gtg cgc aat cag ccc gag ccc cag 1200
Glu Glu Leu Gly Pro Pro Ser Ala Val Arg Asn Gln Pro Glu Pro Gln
385                 390                 395                 400

gag cag agg gag cgg gct cat ctg cag agg gca ctg cag gcc tca gtg 1248
Glu Gln Arg Glu Arg Ala His Leu Gln Arg Ala Leu Gln Ala Ser Val
                405                 410                 415

tct cca cca tcc ccc agc cct aac cag agt tac cag ggc agc agc ggc     1296
Ser Pro Pro Ser Pro Ser Pro Asn Gln Ser Tyr Gln Gly Ser Ser Gly
            420                 425                 430

tac aac ttc cgg ccc aca gat gcc cgc tgc ctg ccc agc agc ccc atc     1344
Tyr Asn Phe Arg Pro Thr Asp Ala Arg Cys Leu Pro Ser Ser Pro Ile
        435                 440                 445

cgg atg ttt gct tcc ttc cac cct tct gcc agc acc gca ggg acc tct     1392
Arg Met Phe Ala Ser Phe His Pro Ser Ala Ser Thr Ala Gly Thr Ser
    450                 455                 460

ggg gac agt gga ccc cca gac agg tca ccc ctg gaa ctt cac att ggt     1440
Gly Asp Ser Gly Pro Pro Asp Arg Ser Pro Leu Glu Leu His Ile Gly
465                 470                 475                 480

ttc ccc aca gac atc cct aaa agt gcc ccc cac tcg atg act gcc tca     1488
Phe Pro Thr Asp Ile Pro Lys Ser Ala Pro His Ser Met Thr Ala Ser
                485                 490                 495

tct tcc tca gtt tca tcc cca tcc cca ggt ctt cct aga cgc tca gca     1536
Ser Ser Ser Val Ser Ser Pro Ser Pro Gly Leu Pro Arg Arg Ser Ala
            500                 505                 510

ccc cct tct ccc ctg tgc cgt agt ttg tct cct ggg act ggg gga gga     1584
Pro Pro Ser Pro Leu Cys Arg Ser Leu Ser Pro Gly Thr Gly Gly Gly
        515                 520                 525

gtc cga ggt ggg gtt ggt tac ctg tcc cga ggg gac cct gtc cgg gtc     1632
Val Arg Gly Gly Val Gly Tyr Leu Ser Arg Gly Asp Pro Val Arg Val
    530                 535                 540

ctt gct cgg aga gta cgg cct gat ggc tct gtg cag tac ctg gtt gag     1680
Leu Ala Arg Arg Val Arg Pro Asp Gly Ser Val Gln Tyr Leu Val Glu
545                 550                 555                 560

tgg gga gga ggg ggc atc ttc tga                                     1704
Trp Gly Gly Gly Gly Ile Phe
                565

<210>  6
<211>  567
<212>  PRT
<213>  Homo sapiens
<400>  6

Met Ala Gln Pro Pro Arg Leu Ser Arg Ser Gly Ala Ser Ser Leu Trp
1               5                   10                  15

Asp Pro Ala Ser Pro Ala Pro Thr Ser Gly Pro Arg Pro Arg Leu Trp
            20                  25                  30

Glu Gly Gln Asp Val Leu Ala Arg Trp Thr Asp Gly Leu Leu Tyr Leu
        35                  40                  45

Gly Thr Ile Lys Lys Val Asp Ser Ala Arg Glu Val Cys Leu Val Gln
    50                  55                  60

Phe Glu Asp Asp Ser Gln Phe Leu Val Leu Trp Lys Asp Ile Ser Pro
65                  70                  75                  80

Ala Ala Leu Pro Gly Glu Glu Leu Leu Cys Cys Val Cys Arg Ser Glu
                85                  90                  95

Thr Val Val Pro Gly Asn Arg Leu Val Ser Cys Glu Lys Cys Arg His
            100                 105                 110

Ala Tyr His Gln Asp Cys His Val Pro Arg Ala Pro Ala Pro Gly Glu
        115                 120                 125

Gly Glu Gly Thr Ser Trp Val Cys Arg Gln Cys Val Phe Ala Ile Ala
    130                 135                 140

Thr Lys Arg Gly Gly Ala Leu Lys Lys Gly Pro Tyr Ala Arg Ala Met
145                 150                 155                 160

Leu Gly Met Lys Leu Ser Leu Pro Tyr Gly Leu Lys Gly Leu Asp Trp
                165                 170                 175

Asp Ala Gly His Leu Ser Asn Arg Gln Gln Ser Tyr Cys Tyr Cys Gly
            180                 185                 190

Gly Pro Gly Glu Trp Asn Leu Lys Met Leu Gln Cys Arg Ser Cys Leu
        195                 200                 205

Gln Trp Phe His Glu Ala Cys Thr Gln Cys Leu Ser Lys Pro Leu Leu
    210                 215                 220

Tyr Gly Asp Arg Phe Tyr Glu Phe Glu Cys Cys Val Cys Arg Gly Gly
225                 230                 235                 240

Pro Glu Lys Val Arg Arg Leu Gln Leu Arg Trp Val Asp Val Ala His
                245                 250                 255

Leu Val Leu Tyr His Leu Ser Val Cys Cys Lys Lys Lys Tyr Phe Asp
            260                 265                 270

Phe Asp Arg Glu Ile Leu Pro Phe Thr Ser Glu Asn Trp Asp Ser Leu
        275                 280                 285

Leu Leu Gly Glu Leu Ser Asp Thr Pro Lys Gly Glu Arg Ser Ser Lys
    290                 295                 300

Leu Leu Ser Ala Leu Asn Ser His Lys Asp Arg Phe Ile Ser Gly Arg
305                 310                 315                 320

Glu Ile Lys Lys Arg Lys Cys Leu Phe Gly Leu His Ala Arg Met Pro
                325                 330                 335

Pro Pro Val Glu Pro Pro Thr Gly Asp Gly Ala Leu Thr Ser Phe Pro
            340                 345                 350

Ser Gly Gln Gly Pro Gly Gly Gly Val Ser Arg Pro Leu Gly Lys Arg
        355                 360                 365

Arg Arg Pro Glu Pro Glu Pro Leu Arg Arg Arg Gln Lys Gly Lys Val
    370                 375                 380

Glu Glu Leu Gly Pro Pro Ser Ala Val Arg Asn Gln Pro Glu Pro Gln
385                 390                 395                 400

Glu Gln Arg Glu Arg Ala His Leu Gln Arg Ala Leu Gln Ala Ser Val
                405                 410                 415

Ser Pro Pro Ser Pro Ser Pro Asn Gln Ser Tyr Gln Gly Ser Ser Gly
            420                 425                 430

Tyr Asn Phe Arg Pro Thr Asp Ala Arg Cys Leu Pro Ser Ser Pro Ile
        435                 440                 445

Arg Met Phe Ala Ser Phe His Pro Ser Ala Ser Thr Ala Gly Thr Ser
    450                 455                 460

Gly Asp Ser Gly Pro Pro Asp Arg Ser Pro Leu Glu Leu His Ile Gly
465                 470                 475                 480

Phe Pro Thr Asp Ile Pro Lys Ser Ala Pro His Ser Met Thr Ala Ser
                485                 490                 495

Ser Ser Ser Val Ser Ser Pro Ser Pro Gly Leu Pro Arg Arg Ser Ala
            500                 505                 510

Pro Pro Ser Pro Leu Cys Arg Ser Leu Ser Pro Gly Thr Gly Gly Gly
        515                 520                 525

Val Arg Gly Gly Val Gly Tyr Leu Ser Arg Gly Asp Pro Val Arg Val
    530                 535                 540

Leu Ala Arg Arg Val Arg Pro Asp Gly Ser Val Gln Tyr Leu Val Glu
545                 550                 555                 560

Trp Gly Gly Gly Gly Ile Phe
                565

<210> 7
<211> 723
<212> DNA
<213> Homo sapiens
<220>
<221> CDS
<222> (1)..(723)
<223>
<400> 7
atg acc tct ttg ccc tgc ccc ctc ccc ggc cgg gac gcc tcc aaa gct 48
Met Thr Ser Leu Pro Cys Pro Leu Pro Gly Arg Asp Ala Ser Lys Ala
1               5                   10                  15

gtc ttc cca gac ctc gcc cct gtc ccg tcg gta gcg gct gcc tac ccg 96
Val Phe Pro Asp Leu Ala Pro Val Pro Ser Val Ala Ala Ala Tyr Pro
            20                  25                  30

ctt ggc ttg tcc cct aca acc gca gcc tcc ccc aat ttg tcc tac tcc 144
Leu Gly Leu Ser Pro Thr Thr Ala Ala Ser Pro Asn Leu Ser Tyr Ser
        35                  40                  45

agg ccg tat ggc cac ctc ctg tct tac ccc tac acc gag cca gcg aac 192
Arg Pro Tyr Gly His Leu Leu Ser Tyr Pro Tyr Thr Glu Pro Ala Asn
    50                  55                  60

ccc gga gac tcc tac ctg tcc tgc cag caa ccc gcg gcg ctc tct cag 240
Pro Gly Asp Ser Tyr Leu Ser Cys Gln Gln Pro Ala Ala Leu Ser Gln
65                  70                  75                  80

ccc ctc tgc gga cct gca gag cac cct cag gaa ctc gag gca gac tcg 288
Pro Leu Cys Gly Pro Ala Glu His Pro Gln Glu Leu Glu Ala Asp Ser
                85                  90                  95

gag aag ccg cgg ctg tcc ccg gaa ccc tcc gag cgg cgc cct cag gcc 336
Glu Lys Pro Arg Leu Ser Pro Glu Pro Ser Glu Arg Arg Pro Gln Ala

            100                 105                 110

ccc gcc aaa aag ctc cgc aag ccg agg acc atc tac tcc agc ctg cag 384
Pro Ala Lys Lys Leu Arg Lys Pro Arg Thr Ile Tyr Ser Ser Leu Gln
        115                 120                 125

ctg cag cac cta aac cag cgt ttc cag cac acg cag tac ctg gcg ctg 432
Leu Gln His Leu Asn Gln Arg Phe Gln His Thr Gln Tyr Leu Ala Leu
    130                 135                 140

ccc gag agg gcc cag ctg gca gcg cag ctc ggc ctc acc cag acc cag 480
Pro Glu Arg Ala Gln Leu Ala Ala Gln Leu Gly Leu Thr Gln Thr Gln
145                 150                 155                 160

gta aag atc tgg ttt cag aac aaa cgc tcc aag tat aag aag ctc ctg 528
Val Lys Ile Trp Phe Gln Asn Lys Arg Ser Lys Tyr Lys Lys Leu Leu
                165                 170                 175

aag cag aat tct ggg ggg cag gaa ggg gac ttc cct ggg agg acc ttc 576
Lys Gln Asn Ser Gly Gly Gln Glu Gly Asp Phe Pro Gly Arg Thr Phe
            180                 185                 190

tct gtg tct ccc tgc tcc cca ccc ctc ccc tcc ctc tgg gat cta ccc 624
Ser Val Ser Pro Cys Ser Pro Pro Leu Pro Ser Leu Trp Asp Leu Pro
        195                 200                 205

aag gca ggg acc ctg ccc acc agt ggc tat ggc aac agc ttt gga gcc 672
Lys Ala Gly Thr Leu Pro Thr Ser Gly Tyr Gly Asn Ser Phe Gly Ala
    210                 215                 220

tgg tat cag cat cac tcc tca gat gtc ctg gct tcg cct cag atg atg 720
Trp Tyr Gln His His Ser Ser Asp Val Leu Ala Ser Pro Gln Met Met
225                 230                 235                 240

tga 723

<210> 8
<211> 240
<212> PRT
<213> Homo sapiens

<400> 8

Met Thr Ser Leu Pro Cys Pro Leu Pro Gly Arg Asp Ala Ser Lys Ala
1               5                   10                  15

Val Phe Pro Asp Leu Ala Pro Val Pro Ser Val Ala Ala Ala Tyr Pro
            20                  25                  30

Leu Gly Leu Ser Pro Thr Thr Ala Ala Ser Pro Asn Leu Ser Tyr Ser
        35                  40                  45

Arg Pro Tyr Gly His Leu Leu Ser Tyr Pro Tyr Thr Glu Pro Ala Asn
    50                  55                  60

Pro Gly Asp Ser Tyr Leu Ser Cys Gln Gln Pro Ala Ala Leu Ser Gln
65                  70                  75                  80

Pro Leu Cys Gly Pro Ala Glu His Pro Gln Glu Leu Glu Ala Asp Ser
                85                  90                  95

Glu Lys Pro Arg Leu Ser Pro Glu Pro Ser Glu Arg Arg Pro Gln Ala
            100                 105                 110

Pro Ala Lys Lys Leu Arg Lys Pro Arg Thr Ile Tyr Ser Ser Leu Gln
        115                 120                 125

Leu Gln His Leu Asn Gln Arg Phe Gln His Thr Gln Tyr Leu Ala Leu
    130                 135                 140

Pro Glu Arg Ala Gln Leu Ala Ala Gln Leu Gly Leu Thr Gln Thr Gln
145                 150                 155                 160

Val Lys Ile Trp Phe Gln Asn Lys Arg Ser Lys Tyr Lys Lys Leu Leu
                165                 170                 175

Lys Gln Asn Ser Gly Gly Gln Glu Gly Asp Phe Pro Gly Arg Thr Phe
            180                 185                 190

Ser Val Ser Pro Cys Ser Pro Pro Leu Pro Ser Leu Trp Asp Leu Pro
        195                 200                 205

Lys Ala Gly Thr Leu Pro Thr Ser Gly Tyr Gly Asn Ser Phe Gly Ala
    210                 215                 220

Trp Tyr Gln His His Ser Ser Asp Val Leu Ala Ser Pro Gln Met Met
225                 230                 235                 240

<210>  9
<211>  2004
<212>  DNA
<213>  Homo sapiens
<220>
<221>  CDS
<222>  (1)..(2004)
<223>
<400>  9
atg cat cac caa cag cga atg gct gcc tta ggg acg gac aaa gag ctg        48
Met His His Gln Gln Arg Met Ala Ala Leu Gly Thr Asp Lys Glu Leu
1               5                   10                  15

agt gat tta ctg gat ttc agt gcg atg ttt tca cct cct gtg agc agt        96
Ser Asp Leu Leu Asp Phe Ser Ala Met Phe Ser Pro Pro Val Ser Ser
            20                  25                  30

ggg aaa aat gga cca act tct ttg gca agt gga cat ttt act ggc tca       144
Gly Lys Asn Gly Pro Thr Ser Leu Ala Ser Gly His Phe Thr Gly Ser
        35                  40                  45

aat gta gaa gac aga agt agc tca ggg tcc tgg ggg aat gga gga cat       192
Asn Val Glu Asp Arg Ser Ser Ser Gly Ser Trp Gly Asn Gly Gly His
    50                  55                  60

cca agc ccg tcc agg aac tat gga gat ggg act ccc tat gac cac atg       240
Pro Ser Pro Ser Arg Asn Tyr Gly Asp Gly Thr Pro Tyr Asp His Met
65                  70                  75                  80

acc agc agg gac ctt ggg tca cat gac aat ctc tct cca cct ttt gtc       288
Thr Ser Arg Asp Leu Gly Ser His Asp Asn Leu Ser Pro Pro Phe Val
                85                  90                  95

aat tcc aga ata caa agt aaa aca gaa agg ggc tca tac tca tct tat       336
Asn Ser Arg Ile Gln Ser Lys Thr Glu Arg Gly Ser Tyr Ser Ser Tyr
            100                 105                 110

ggg aga gaa tca aac tta cag ggt tgc cac cag cag agt ctc ctt gga       384

Gly Arg Glu Ser Asn Leu Gln Gly Cys His Gln Gln Ser Leu Leu Gly
        115                 120                 125

ggt gac atg gat atg ggc aac cca gga acc ctt tcg ccc acc aaa cct        432
Gly Asp Met Asp Met Gly Asn Pro Gly Thr Leu Ser Pro Thr Lys Pro
    130                 135                 140

ggt tcc cag tac tat cag tat tct agc aat aat ccc cga agg agg cct        480
Gly Ser Gln Tyr Tyr Gln Tyr Ser Ser Asn Asn Pro Arg Arg Arg Pro
145                 150                 155                 160

ctt cac agt agt gcc atg gag gta cag aca aag aaa gtt cga aaa gtt        528
Leu His Ser Ser Ala Met Glu Val Gln Thr Lys Lys Val Arg Lys Val
                165                 170                 175

cct cca ggt ttg cca tct tca gtc tat gct cca tca gca agc act gcc        576
Pro Pro Gly Leu Pro Ser Ser Val Tyr Ala Pro Ser Ala Ser Thr Ala
            180                 185                 190

gac tac aat agg gac tcg cca ggc tat cct tcc tcc aaa cca gca acc        624
Asp Tyr Asn Arg Asp Ser Pro Gly Tyr Pro Ser Ser Lys Pro Ala Thr
        195                 200                 205

agc act ttc cct agc tcc ttc ttc atg caa gat ggc cat cac agc agt        672
Ser Thr Phe Pro Ser Ser Phe Phe Met Gln Asp Gly His His Ser Ser
    210                 215                 220

gac cct tgg agc tcc tcc agt ggg atg aat cag cct ggc tat gca gga        720
Asp Pro Trp Ser Ser Ser Ser Gly Met Asn Gln Pro Gly Tyr Ala Gly
225                 230                 235                 240

atg ttg ggc aac tct tct cat att cca cag tcc agc agc tac tgt agc        768
Met Leu Gly Asn Ser Ser His Ile Pro Gln Ser Ser Ser Tyr Cys Ser
                245                 250                 255

ctg cat cca cat gaa cgt ttg agc tat cca tca cac tcc tca gca gac        816
Leu His Pro His Glu Arg Leu Ser Tyr Pro Ser His Ser Ser Ala Asp
            260                 265                 270

atc aat tcc agt ctt cct ccg atg tcc act ttc cat cgt agt ggt aca        864
Ile Asn Ser Ser Leu Pro Pro Met Ser Thr Phe His Arg Ser Gly Thr
        275                 280                 285

aac cat tac agc acc tct tcc tgt acg cct cct gcc aac ggg aca gac        912
Asn His Tyr Ser Thr Ser Ser Cys Thr Pro Pro Ala Asn Gly Thr Asp
    290                 295                 300

agt ata atg gca aat aga gga agc ggg gca gcc ggc agc tcc cag act        960
Ser Ile Met Ala Asn Arg Gly Ser Gly Ala Ala Gly Ser Ser Gln Thr
305                 310                 315                 320

gga gat gct ctg ggg aaa gca ctt gct tcg atc tat tct cca gat cac       1008
Gly Asp Ala Leu Gly Lys Ala Leu Ala Ser Ile Tyr Ser Pro Asp His
                325                 330                 335

act aac aac agc ttt tca tca aac cct tca act cct gtt ggc tct cct       1056
Thr Asn Asn Ser Phe Ser Ser Asn Pro Ser Thr Pro Val Gly Ser Pro
            340                 345                 350

cca tct ctc tca gca ggc aca gct gtt tgg tct aga aat gga gga cag       1104
Pro Ser Leu Ser Ala Gly Thr Ala Val Trp Ser Arg Asn Gly Gly Gln
        355                 360                 365

gcc tca tcg tct cct aat tat gaa gga ccc tta cac tct ttg caa agc     1152
Ala Ser Ser Ser Pro Asn Tyr Glu Gly Pro Leu His Ser Leu Gln Ser
    370                 375                 380

cga att gaa gat cgt tta gaa aga ctg gat gat gct att cat gtt ctc     1200
Arg Ile Glu Asp Arg Leu Glu Arg Leu Asp Asp Ala Ile His Val Leu
385                 390                 395                 400

cgg aac cat gca gtg ggc cca tcc aca gct atg cct ggt ggt cat ggg     1248
Arg Asn His Ala Val Gly Pro Ser Thr Ala Met Pro Gly Gly His Gly
                405                 410                 415

gac atg cat gga atc att gga cct tct cat aat gga gcc atg ggt ggt     1296
Asp Met His Gly Ile Ile Gly Pro Ser His Asn Gly Ala Met Gly Gly
            420                 425                 430

ctg ggc tca ggg tat gga acc ggc ctt ctt tca gcc aac aga cat tca     1344
Leu Gly Ser Gly Tyr Gly Thr Gly Leu Leu Ser Ala Asn Arg His Ser
        435                 440                 445

ctc atg gtg ggg acc cat cgt gaa gat ggc gtg gcc ctg aga ggc agc     1392
Leu Met Val Gly Thr His Arg Glu Asp Gly Val Ala Leu Arg Gly Ser
    450                 455                 460

cat tct ctt ctg cca aac cag gtt ccg gtt cca cag ctt cct gtc cag     1440
His Ser Leu Leu Pro Asn Gln Val Pro Val Pro Gln Leu Pro Val Gln
465                 470                 475                 480

tct gcg act tcc cct gac ctg aac cca ccc cag gac cct tac aga ggc     1488
Ser Ala Thr Ser Pro Asp Leu Asn Pro Pro Gln Asp Pro Tyr Arg Gly
                485                 490                 495

atg cca cca gga cta cag ggg cag agt gtc tcc tct ggc agc tct gag     1536
Met Pro Pro Gly Leu Gln Gly Gln Ser Val Ser Ser Gly Ser Ser Glu
            500                 505                 510

atc aaa tcc gat gac gag ggt gat gag aac ctg caa gac acg aaa tct     1584
Ile Lys Ser Asp Asp Glu Gly Asp Glu Asn Leu Gln Asp Thr Lys Ser
        515                 520                 525

tcg gag gac aag aaa tta gat gac gac aag aag gat atc aaa tca att     1632
Ser Glu Asp Lys Lys Leu Asp Asp Asp Lys Lys Asp Ile Lys Ser Ile
    530                 535                 540

act agc aat aat gac gat gag gac ctg aca cca gag cag aag gca gag     1680
Thr Ser Asn Asn Asp Asp Glu Asp Leu Thr Pro Glu Gln Lys Ala Glu
545                 550                 555                 560

cgt gag aag gag cgg agg atg gcc aac aat gcc cga gag cgt ctg cgg     1728
Arg Glu Lys Glu Arg Arg Met Ala Asn Asn Ala Arg Glu Arg Leu Arg
                565                 570                 575

gtc cgt gac atc aac gag gct ttc aaa gag ctc ggc cgc atg gtg cag     1776
Val Arg Asp Ile Asn Glu Ala Phe Lys Glu Leu Gly Arg Met Val Gln
            580                 585                 590

ctc cac ctc aag agt gac aag ccc cag acc aag ctc ctg atc ctc cac     1824
Leu His Leu Lys Ser Asp Lys Pro Gln Thr Lys Leu Leu Ile Leu His
        595                 600                 605

cag gcg gtg gcc gtc atc ctc agt ctg gag cag caa gtc cga gaa agg     1872
Gln Ala Val Ala Val Ile Leu Ser Leu Glu Gln Gln Val Arg Glu Arg
    610                 615                 620

aat ctg aat ccg aaa gct gcg tgt ctg aaa aga agg gag gaa gag aag 1920
Asn Leu Asn Pro Lys Ala Ala Cys Leu Lys Arg Arg Glu Glu Glu Lys
625                 630                 635                 640

gtg tcc tca gag cct ccc cct ctc tcc ttg gcc ggc cca cac cct gga 1968
Val Ser Ser Glu Pro Pro Pro Leu Ser Leu Ala Gly Pro His Pro Gly
                645                 650                 655

atg gga gac gca tcg aat cac atg gga cag atg taa 2004
Met Gly Asp Ala Ser Asn His Met Gly Gln Met
            660                 665

<210> 10
<211> 667
<212> PRT
<213> Homo sapiens
<400> 10

Met His His Gln Gln Arg Met Ala Ala Leu Gly Thr Asp Lys Glu Leu
1               5                   10                  15

Ser Asp Leu Leu Asp Phe Ser Ala Met Phe Ser Pro Pro Val Ser Ser
            20                  25                  30

Gly Lys Asn Gly Pro Thr Ser Leu Ala Ser Gly His Phe Thr Gly Ser
        35                  40                  45

Asn Val Glu Asp Arg Ser Ser Ser Gly Ser Trp Gly Asn Gly Gly His
    50                  55                  60

Pro Ser Pro Ser Arg Asn Tyr Gly Asp Gly Thr Pro Tyr Asp His Met
65                  70                  75                  80

Thr Ser Arg Asp Leu Gly Ser His Asp Asn Leu Ser Pro Pro Phe Val
                85                  90                  95

Asn Ser Arg Ile Gln Ser Lys Thr Glu Arg Gly Ser Tyr Ser Ser Tyr
            100                 105                 110

Gly Arg Glu Ser Asn Leu Gln Gly Cys His Gln Gln Ser Leu Leu Gly
        115                 120                 125

Gly Asp Met Asp Met Gly Asn Pro Gly Thr Leu Ser Pro Thr Lys Pro
    130                 135                 140

Gly Ser Gln Tyr Tyr Gln Tyr Ser Ser Asn Asn Pro Arg Arg Arg Pro
145                 150                 155                 160

Leu His Ser Ser Ala Met Glu Val Gln Thr Lys Lys Val Arg Lys Val
                165                 170                 175

Pro Pro Gly Leu Pro Ser Ser Val Tyr Ala Pro Ser Ala Ser Thr Ala
            180                 185                 190

Asp Tyr Asn Arg Asp Ser Pro Gly Tyr Pro Ser Ser Lys Pro Ala Thr
        195                 200                 205

Ser Thr Phe Pro Ser Ser Phe Phe Met Gln Asp Gly His His Ser Ser
    210                 215                 220

Asp Pro Trp Ser Ser Ser Ser Gly Met Asn Gln Pro Gly Tyr Ala Gly
225                 230                 235                 240

Met Leu Gly Asn Ser Ser His Ile Pro Gln Ser Ser Ser Tyr Cys Ser
                245                 250                 255

Leu His Pro His Glu Arg Leu Ser Tyr Pro Ser His Ser Ser Ala Asp
            260                 265                 270

Ile Asn Ser Ser Leu Pro Pro Met Ser Thr Phe His Arg Ser Gly Thr
        275                 280                 285

Asn His Tyr Ser Thr Ser Ser Cys Thr Pro Pro Ala Asn Gly Thr Asp
    290                 295                 300

Ser Ile Met Ala Asn Arg Gly Ser Gly Ala Ala Gly Ser Ser Gln Thr
305                 310                 315                 320

Gly Asp Ala Leu Gly Lys Ala Leu Ala Ser Ile Tyr Ser Pro Asp His
                325                 330                 335

Thr Asn Asn Ser Phe Ser Ser Asn Pro Ser Thr Pro Val Gly Ser Pro
            340                 345                 350

Pro Ser Leu Ser Ala Gly Thr Ala Val Trp Ser Arg Asn Gly Gly Gln
        355                 360                 365

Ala Ser Ser Ser Pro Asn Tyr Glu Gly Pro Leu His Ser Leu Gln Ser
    370                 375                 380

Arg Ile Glu Asp Arg Leu Glu Arg Leu Asp Asp Ala Ile His Val Leu
385                 390                 395                 400

Arg Asn His Ala Val Gly Pro Ser Thr Ala Met Pro Gly Gly His Gly
                405                 410                 415

Asp Met His Gly Ile Ile Gly Pro Ser His Asn Gly Ala Met Gly Gly
            420                 425                 430

Leu Gly Ser Gly Tyr Gly Thr Gly Leu Leu Ser Ala Asn Arg His Ser
        435                 440                 445

Leu Met Val Gly Thr His Arg Glu Asp Gly Val Ala Leu Arg Gly Ser
    450                 455                 460

His Ser Leu Leu Pro Asn Gln Val Pro Val Pro Gln Leu Pro Val Gln
465                 470                 475                 480

Ser Ala Thr Ser Pro Asp Leu Asn Pro Pro Gln Asp Pro Tyr Arg Gly
                485                 490                 495

Met Pro Pro Gly Leu Gln Gly Gln Ser Val Ser Ser Gly Ser Ser Glu
            500                 505                 510

Ile Lys Ser Asp Asp Glu Gly Asp Glu Asn Leu Gln Asp Thr Lys Ser
        515                 520                 525

Ser Glu Asp Lys Lys Leu Asp Asp Asp Lys Lys Asp Ile Lys Ser Ile
    530                 535                 540

Thr Ser Asn Asn Asp Asp Glu Asp Leu Thr Pro Glu Gln Lys Ala Glu
545                 550                 555                 560

Arg Glu Lys Glu Arg Arg Met Ala Asn Asn Ala Arg Glu Arg Leu Arg
                565                 570                 575

Val Arg Asp Ile Asn Glu Ala Phe Lys Glu Leu Gly Arg Met Val Gln
            580                 585                 590

Leu His Leu Lys Ser Asp Lys Pro Gln Thr Lys Leu Leu Ile Leu His
        595                 600                 605

Gln Ala Val Ala Val Ile Leu Ser Leu Glu Gln Gln Val Arg Glu Arg
    610                 615                 620

Asn Leu Asn Pro Lys Ala Ala Cys Leu Lys Arg Arg Glu Glu Glu Lys
625                 630                 635                 640

Val Ser Ser Glu Pro Pro Pro Leu Ser Leu Ala Gly Pro His Pro Gly
                645                 650                 655

Met Gly Asp Ala Ser Asn His Met Gly Gln Met
            660                 665

<210> 11
<211> 2085
<212> DNA
<213> Homo sapiens
<220>
<221> CDS
<222> (1)..(2085)
<223>
<400> 11
atg ccg gag ttc ctg gaa gac ccc tcg gtc ctg aca aaa gac aag ttg 48
Met Pro Glu Phe Leu Glu Asp Pro Ser Val Leu Thr Lys Asp Lys Leu
1               5                   10                  15

aag agt gag ttg gtc gcc aac aat gtg acg ctg ccg gcc ggg gag cag 96
Lys Ser Glu Leu Val Ala Asn Asn Val Thr Leu Pro Ala Gly Glu Gln
            20                  25                  30

cgc aaa gac gtg tac gtc cag ctc tac ctg cag cac ctc acg gct cgc 144
Arg Lys Asp Val Tyr Val Gln Leu Tyr Leu Gln His Leu Thr Ala Arg
        35                  40                  45

aac cgg ccg ccg ctc ccc gcc ggc acc aac agc aag ggg ccc ccg gac 192
Asn Arg Pro Pro Leu Pro Ala Gly Thr Asn Ser Lys Gly Pro Pro Asp
    50                  55                  60

ttc tcc agt gac gaa gag cgc gag ccc acc ccg gtc ctc ggc tct ggg 240
Phe Ser Ser Asp Glu Glu Arg Glu Pro Thr Pro Val Leu Gly Ser Gly
65                  70                  75                  80

gcc gcc gcc gcg ggc cgg agc cga gca gcc gtc ggc agg aaa gcc aca 288
Ala Ala Ala Ala Gly Arg Ser Arg Ala Ala Val Gly Arg Lys Ala Thr
                85                  90                  95

aaa aaa act gat aaa ccc aga caa gaa gat aaa gat gat cta gat gta 336
Lys Lys Thr Asp Lys Pro Arg Gln Glu Asp Lys Asp Asp Leu Asp Val
            100                 105                 110

aca gag ctc act aat gaa gat ctt ttg gat cag ctt gtg aaa tac gga 384
Thr Glu Leu Thr Asn Glu Asp Leu Leu Asp Gln Leu Val Lys Tyr Gly
        115                 120                 125

gtg aat cct ggt cct att gtg gga aca acc agg aag cta tat gag aaa 432
Val Asn Pro Gly Pro Ile Val Gly Thr Thr Arg Lys Leu Tyr Glu Lys
    130                 135                 140

aag ctt ttg aaa ctg agg gaa caa gga aca gaa tca aga tct tct act     480
Lys Leu Leu Lys Leu Arg Glu Gln Gly Thr Glu Ser Arg Ser Ser Thr
145                 150                 155                 160

cct ctg cca aca att tct tct tca gca gaa aat aca agg cag aat gga     528
Pro Leu Pro Thr Ile Ser Ser Ser Ala Glu Asn Thr Arg Gln Asn Gly
                165                 170                 175

agt aat gat tct gac aga tac agt gac aat gaa gaa gga aag aag aaa     576
Ser Asn Asp Ser Asp Arg Tyr Ser Asp Asn Glu Glu Gly Lys Lys Lys
            180                 185                 190

gaa cac aag aaa gtg aag tcc act agg gat att gtt cct ttt tct gaa     624
Glu His Lys Lys Val Lys Ser Thr Arg Asp Ile Val Pro Phe Ser Glu
        195                 200                 205

ctt gga act act ccc tct ggt ggt gga ttt ttt cag ggt att tct ttt     672
Leu Gly Thr Thr Pro Ser Gly Gly Gly Phe Phe Gln Gly Ile Ser Phe
    210                 215                 220

cct gaa atc tcc acc cgt cct cct ttg ggc agt acc gaa cta cag gca     720
Pro Glu Ile Ser Thr Arg Pro Pro Leu Gly Ser Thr Glu Leu Gln Ala
225                 230                 235                 240

gct aag aaa gta cat act tct aag gga gac cta cct agg gag cct ctt     768
Ala Lys Lys Val His Thr Ser Lys Gly Asp Leu Pro Arg Glu Pro Leu
                245                 250                 255

gtt gcc aca aac ttg cct ggc agg gga cag ttg cag aag tta gcc tct     816
Val Ala Thr Asn Leu Pro Gly Arg Gly Gln Leu Gln Lys Leu Ala Ser
            260                 265                 270

gaa agg aat ttg ttt att tca tgc aag tct agc cat gat agg tgt tta     864
Glu Arg Asn Leu Phe Ile Ser Cys Lys Ser Ser His Asp Arg Cys Leu
        275                 280                 285

gag aaa agt tct tcg tca tct tct cag cct gaa cac agt gcc atg ttg     912
Glu Lys Ser Ser Ser Ser Ser Ser Gln Pro Glu His Ser Ala Met Leu
    290                 295                 300

gtc tct act gca gct tct cct tca ctg att aaa gaa acc acc act ggt     960
Val Ser Thr Ala Ala Ser Pro Ser Leu Ile Lys Glu Thr Thr Thr Gly
305                 310                 315                 320

tac tat aaa gac ata gta gaa aat att tgc ggt aga gag aaa agt gga    1008
Tyr Tyr Lys Asp Ile Val Glu Asn Ile Cys Gly Arg Glu Lys Ser Gly
                325                 330                 335

att caa cca tta tgt cct gag agg tcc cat att tca gat caa tcg cct    1056
Ile Gln Pro Leu Cys Pro Glu Arg Ser His Ile Ser Asp Gln Ser Pro
            340                 345                 350

ctc tcc agt aaa agg aaa gca cta gaa gag tct gag agc tca caa cta    1104
Leu Ser Ser Lys Arg Lys Ala Leu Glu Glu Ser Glu Ser Ser Gln Leu
        355                 360                 365

att tct ccg cca ctt gcc cag gca atc aga gat tat gtc aat tct ctg    1152
Ile Ser Pro Pro Leu Ala Gln Ala Ile Arg Asp Tyr Val Asn Ser Leu
    370                 375                 380

ttg gtc cag ggt ggg gta ggt agt ttg cct gga act tct aac tct atg    1200
Leu Val Gln Gly Gly Val Gly Ser Leu Pro Gly Thr Ser Asn Ser Met

385                 390                 395                 400

ccc cca ctg gat gta gaa aac ata cag aag aga att gat cag tct aag      1248
Pro Pro Leu Asp Val Glu Asn Ile Gln Lys Arg Ile Asp Gln Ser Lys
                405                 410                 415

ttt caa gaa act gaa ttc ctg tct cct cca aga aaa gtc cct aga ctg      1296
Phe Gln Glu Thr Glu Phe Leu Ser Pro Pro Arg Lys Val Pro Arg Leu
            420                 425                 430

agt gag aag tca gtg gag gaa agg gat tca ggt tcc ttt gtg gca ttt      1344
Ser Glu Lys Ser Val Glu Glu Arg Asp Ser Gly Ser Phe Val Ala Phe
        435                 440                 445

cag aac ata cct gga tcc gaa ctg atg tct tct ttt gcc aaa act gtt      1392
Gln Asn Ile Pro Gly Ser Glu Leu Met Ser Ser Phe Ala Lys Thr Val
    450                 455                 460

gtc tct cat tca ctc act acc tta ggt cta gaa gtg gct aag caa tca      1440
Val Ser His Ser Leu Thr Thr Leu Gly Leu Glu Val Ala Lys Gln Ser
465                 470                 475                 480

cag cat gat aaa ata gat gcc tca gaa cta tct ttt ccc ttc cat gaa      1488
Gln His Asp Lys Ile Asp Ala Ser Glu Leu Ser Phe Pro Phe His Glu
                485                 490                 495

tct att tta aaa gta att gaa gaa gaa tgg cag caa gtt gac agg cag      1536
Ser Ile Leu Lys Val Ile Glu Glu Glu Trp Gln Gln Val Asp Arg Gln
            500                 505                 510

ctg cct tca ctg gca tgc aaa tat cca gtt tct tcc agg gag gca aca      1584
Leu Pro Ser Leu Ala Cys Lys Tyr Pro Val Ser Ser Arg Glu Ala Thr
        515                 520                 525

cag ata tta tca gtt cca aaa gta gat gat gaa atc cta ggg ttt att      1632
Gln Ile Leu Ser Val Pro Lys Val Asp Asp Glu Ile Leu Gly Phe Ile
    530                 535                 540

tct gaa gcc act cca cta gga ggt att caa gca gcc tcc act gag tct      1680
Ser Glu Ala Thr Pro Leu Gly Gly Ile Gln Ala Ala Ser Thr Glu Ser
545                 550                 555                 560

tgc aat cag cag ttg gac tta gca ctc tgt aga gca tat gaa gct gca      1728
Cys Asn Gln Gln Leu Asp Leu Ala Leu Cys Arg Ala Tyr Glu Ala Ala
                565                 570                 575

gca tca gca ttg cag att gca act cac act gcc ttt gta gct aag gct      1776
Ala Ser Ala Leu Gln Ile Ala Thr His Thr Ala Phe Val Ala Lys Ala
            580                 585                 590

atg cag gca gac att agt caa gct gca cag att ctt agc tca gat cct      1824
Met Gln Ala Asp Ile Ser Gln Ala Ala Gln Ile Leu Ser Ser Asp Pro
        595                 600                 605

agt cgt acc cac caa gcg ctt ggg att ctg agc aaa aca tat gat gca      1872
Ser Arg Thr His Gln Ala Leu Gly Ile Leu Ser Lys Thr Tyr Asp Ala
    610                 615                 620

gcc tca tat att tgt gaa gct gca ttt gat gaa gtg aag atg gct gcc      1920
Ala Ser Tyr Ile Cys Glu Ala Ala Phe Asp Glu Val Lys Met Ala Ala
625                 630                 635                 640

cat acc atg gga aat gcc act gta ggt cgt cga tac ctc tgg ctg aag      1968

His Thr Met Gly Asn Ala Thr Val Gly Arg Arg Tyr Leu Trp Leu Lys
                645                 650                 655

gat tgc aaa att aat tta gct tct aag aat aag ctg gct tcc act ccc      2016
Asp Cys Lys Ile Asn Leu Ala Ser Lys Asn Lys Leu Ala Ser Thr Pro
            660                 665                 670

ttt aaa ggt gga aca tta ttt gga gga gaa gta tgc aaa gta att aaa      2064
Phe Lys Gly Gly Thr Leu Phe Gly Gly Glu Val Cys Lys Val Ile Lys
        675                 680                 685

aag cgt gga aat aaa cac tag                                          2085
Lys Arg Gly Asn Lys His
    690

<210>  12
<211>  694
<212>  PRT
<213>  Homo sapiens
<400>  12
Met Pro Glu Phe Leu Glu Asp Pro Ser Val Leu Thr Lys Asp Lys Leu
1               5                   10                  15

Lys Ser Glu Leu Val Ala Asn Asn Val Thr Leu Pro Ala Gly Glu Gln
            20                  25                  30

Arg Lys Asp Val Tyr Val Gln Leu Tyr Leu Gln His Leu Thr Ala Arg
        35                  40                  45

Asn Arg Pro Pro Leu Pro Ala Gly Thr Asn Ser Lys Gly Pro Pro Asp
    50                  55                  60

Phe Ser Ser Asp Glu Glu Arg Glu Pro Thr Pro Val Leu Gly Ser Gly
65                  70                  75                  80

Ala Ala Ala Ala Gly Arg Ser Arg Ala Ala Val Gly Arg Lys Ala Thr
                85                  90                  95

Lys Lys Thr Asp Lys Pro Arg Gln Glu Asp Lys Asp Asp Leu Asp Val
            100                 105                 110

Thr Glu Leu Thr Asn Glu Asp Leu Leu Asp Gln Leu Val Lys Tyr Gly
        115                 120                 125

Val Asn Pro Gly Pro Ile Val Gly Thr Thr Arg Lys Leu Tyr Glu Lys
    130                 135                 140

Lys Leu Leu Lys Leu Arg Glu Gln Gly Thr Glu Ser Arg Ser Ser Thr
145                 150                 155                 160

Pro Leu Pro Thr Ile Ser Ser Ser Ala Glu Asn Thr Arg Gln Asn Gly
                165                 170                 175

Ser Asn Asp Ser Asp Arg Tyr Ser Asp Asn Glu Glu Gly Lys Lys Lys
            180                 185                 190

Glu His Lys Lys Val Lys Ser Thr Arg Asp Ile Val Pro Phe Ser Glu
        195                 200                 205

Leu Gly Thr Thr Pro Ser Gly Gly Gly Phe Phe Gln Gly Ile Ser Phe
    210                 215                 220

Pro Glu Ile Ser Thr Arg Pro Pro Leu Gly Ser Thr Glu Leu Gln Ala
225                 230                 235                 240

Ala Lys Lys Val His Thr Ser Lys Gly Asp Leu Pro Arg Glu Pro Leu
                245                 250                 255

Val Ala Thr Asn Leu Pro Gly Arg Gly Gln Leu Gln Lys Leu Ala Ser
            260                 265                 270

Glu Arg Asn Leu Phe Ile Ser Cys Lys Ser Ser His Asp Arg Cys Leu
        275                 280                 285

Glu Lys Ser Ser Ser Ser Ser Ser Gln Pro Glu His Ser Ala Met Leu
    290                 295                 300

Val Ser Thr Ala Ala Ser Pro Ser Leu Ile Lys Glu Thr Thr Thr Gly
305                 310                 315                 320

Tyr Tyr Lys Asp Ile Val Glu Asn Ile Cys Gly Arg Glu Lys Ser Gly
                325                 330                 335

Ile Gln Pro Leu Cys Pro Glu Arg Ser His Ile Ser Asp Gln Ser Pro
            340                 345                 350

Leu Ser Ser Lys Arg Lys Ala Leu Glu Glu Ser Glu Ser Ser Gln Leu
        355                 360                 365

Ile Ser Pro Pro Leu Ala Gln Ala Ile Arg Asp Tyr Val Asn Ser Leu
    370                 375                 380

Leu Val Gln Gly Gly Val Gly Ser Leu Pro Gly Thr Ser Asn Ser Met
385                 390                 395                 400

Pro Pro Leu Asp Val Glu Asn Ile Gln Lys Arg Ile Asp Gln Ser Lys
                405                 410                 415

Phe Gln Glu Thr Glu Phe Leu Ser Pro Pro Arg Lys Val Pro Arg Leu
            420                 425                 430

Ser Glu Lys Ser Val Glu Glu Arg Asp Ser Gly Ser Phe Val Ala Phe
        435                 440                 445

Gln Asn Ile Pro Gly Ser Glu Leu Met Ser Ser Phe Ala Lys Thr Val
    450                 455                 460

Val Ser His Ser Leu Thr Thr Leu Gly Leu Glu Val Ala Lys Gln Ser
465                 470                 475                 480

Gln His Asp Lys Ile Asp Ala Ser Glu Leu Ser Phe Pro Phe His Glu
                485                 490                 495

Ser Ile Leu Lys Val Ile Glu Glu Glu Trp Gln Gln Val Asp Arg Gln
            500                 505                 510

Leu Pro Ser Leu Ala Cys Lys Tyr Pro Val Ser Ser Arg Glu Ala Thr
        515                 520                 525

Gln Ile Leu Ser Val Pro Lys Val Asp Asp Glu Ile Leu Gly Phe Ile
    530                 535                 540

Ser Glu Ala Thr Pro Leu Gly Gly Ile Gln Ala Ala Ser Thr Glu Ser
545                 550                 555                 560

Cys Asn Gln Gln Leu Asp Leu Ala Leu Cys Arg Ala Tyr Glu Ala Ala
                565                 570                 575

Ala Ser Ala Leu Gln Ile Ala Thr His Thr Ala Phe Val Ala Lys Ala
            580                 585                 590

Met Gln Ala Asp Ile Ser Gln Ala Ala Gln Ile Leu Ser Ser Asp Pro
        595                 600                 605

Ser Arg Thr His Gln Ala Leu Gly Ile Leu Ser Lys Thr Tyr Asp Ala
    610                 615                 620

Ala Ser Tyr Ile Cys Glu Ala Ala Phe Asp Glu Val Lys Met Ala Ala
625                 630                 635                 640

His Thr Met Gly Asn Ala Thr Val Gly Arg Arg Tyr Leu Trp Leu Lys
                645                 650                 655

Asp Cys Lys Ile Asn Leu Ala Ser Lys Asn Lys Leu Ala Ser Thr Pro
            660                 665                 670

Phe Lys Gly Gly Thr Leu Phe Gly Gly Glu Val Cys Lys Val Ile Lys
        675                 680                 685

Lys Arg Gly Asn Lys His
    690

<210>  13
<211>  21
<212>  PRT
<213>  Homo sapiens
<220>
<221>  MISC_FEATURE
<223>  Partial sequence of IPF1, showing high score in the local alignme
       nt between IPF1 and HNF3G, No.1
<400>  13
Pro Pro Gly Leu Ser Ala Ser Pro Gln Pro Ser Ser Val Ala Pro Arg
1               5                   10                  15

Arg Pro Gln Glu Pro
            20

<210>  14
<211>  8
<212>  PRT
<213>  Homo sapiens
<220>
<221>  MISC_FEATURE
<223>  Partial sequence of IPF1, showing high score in the local alignme
       nt between IPF1 and HNF3G, No.2
<400>  14

Pro Glu Gly Ala Glu Pro Gly Val
1               5

<210>  15
<211>  12
<212>  PRT
<213>  Homo sapiens
<220>
<221>  MISC_FEATURE
<223>  Partial sequence of IPF1, showing high score in the local alignme
       nt between IPF1 and HNF3G, No.3

<400>  15
Pro Pro Pro His Pro Phe Pro Gly Ala Leu Gly Ala
1               5                   10

<210>  16
<211>  22
<212>  PRT
<213>  Homo sapiens
<220>
<221>  MISC_FEATURE
<223>  Partial sequence of HNF3G, showing high score in the local alignm
       ent between IPF1 and HNF3G, No.1
<400>  16
Pro Gly Gly Leu Pro Ala Ser Pro Leu Pro Ser Gly Pro Leu Ala Pro
1               5                   10                  15

Pro Ala Pro Ala Ala Pro
            20

<210>  17
<211>  8
<212>  PRT
<213>  Homo sapiens
<220>
<221>  MISC_FEATURE
<223>  Partial sequence of HNF3G, showing high score in the local alignm
       ent between IPF1 and HNF3G, No.2
<400>  17
Ala Glu Gly Gly Glu Pro Gly Val
1               5

<210>  18
<211>  12
<212>  PRT
<213>  Homo sapiens
<220>
<221>  MISC_FEATURE
<223>  Partial sequence of HNF3G, showing high score in the local alignm
       ent between IPF1 and HNF3G, No.3
<400>  18
Pro Leu Ser Ser Pro Tyr Pro Gly Gly Leu Pro Ala
1               5                   10

<210>  19
<211>  23
<212>  PRT
<213>  Homo sapiens
<220>
<221>  MISC_FEATURE
<223>  Partial sequence of IPF1, showing high score in the local alignme
       nt between IPF1 and PHF1, No.1
<400>  19
His Pro Phe Pro Gly Ala Leu Gly Ala Leu Glu Gln Gly Ser Pro Pro
1               5                   10                  15

Asp Ile Ser Pro Tyr Glu Val
            20

<210>  20
<211>  9
<212>  PRT
<213>  Homo sapiens

<220>
<221>  MISC_FEATURE
<223>  Partial sequence of IPF1, showing high score in the local alignme
       nt between IPF1 and PHF1, No.2
<400>  20
Ala Val Thr Ser Gly Glu Glu Leu Leu
1               5

<210>  21
<211>  23
<212>  PRT
<213>  Homo sapiens
<220>
<221>  MISC_FEATURE
<223>  Partial sequence of PHF1, showing high score in the local alignme
       nt between IPF1 and PHF1, No.1
<400>  21
His Pro Ser Ala Ser Thr Ala Gly Thr Ser Gly Asp Ser Gly Pro Pro
1               5                   10                  15

Asp Arg Ser Pro Leu Glu Leu
            20

<210>  22
<211>  9
<212>  PRT
<213>  Homo sapiens
<220>
<221>  MISC_FEATURE
<223>  Partial sequence of PHF1, showing high score in the local alignme
       nt between IPF1 and PHF1, No.2
<400>  22
Ala Ala Leu Pro Gly Glu Glu Leu Leu
1               5

<210>  23
<211>  57
<212>  PRT
<213>  Homo sapiens
<220>
<221>  MISC_FEATURE
<223>  Partial sequence of IPF1, showing high score in the local alignme
       nt between IPF1 and DLX4, No.1
<400>  23
Lys Arg Thr Arg Thr Ala Tyr Thr Arg Ala Gln Leu Leu Glu Leu Glu
1               5                   10                  15

Lys Glu Phe Leu Phe Asn Lys Tyr Ile Ser Arg Pro Arg Arg Val Glu
            20                  25                  30

Leu Ala Val Met Leu Asn Leu Thr Glu Arg His Ile Lys Ile Trp Phe
        35                  40                  45

Gln Asn Arg Arg Met Lys Trp Lys Lys
    50                  55

<210>  24
<211>  15
<212>  PRT
<213>  Homo sapiens
<220>
<221>  MISC_FEATURE
<223>  Partial sequence of IPF1, showing high score in the local alignme

nt between IPF1 and DLX4, No.2
<400> 24
Ser Pro Gln Pro Ser Ser Val Ala Pro Arg Arg Pro Gln Glu Pro
1               5                   10                  15

<210> 25
<211> 57
<212> PRT
<213> Homo sapiens
<220>
<221> MISC_FEATURE
<223> Partial sequence of DLX4, showing high score in the local alignme
      nt between IPF1 and DLX4, No.1
<400> 25
Arg Lys Pro Arg Thr Ile Tyr Ser Ser Leu Gln Leu Gln His Leu Asn
1               5                   10                  15

Gln Arg Phe Gln His Thr Gln Tyr Leu Ala Leu Pro Glu Arg Ala Gln
            20                  25                  30

Leu Ala Ala Gln Leu Gly Leu Thr Gln Thr Gln Val Lys Ile Trp Phe
        35                  40                  45

Gln Asn Lys Arg Ser Lys Tyr Lys Lys
    50                  55

<210> 26
<211> 12
<212> PRT
<213> Homo sapiens
<220>
<221> MISC_FEATURE
<223> Partial sequence of DLX4, showing high score in the local alignme
      nt between IPF1 and DLX4, No.2
<400> 26
Ser Pro Glu Pro Ser Glu Arg Arg Pro Gln Ala Pro
1               5                   10

<210> 27
<211> 15
<212> PRT
<213> Homo sapiens
<220>
<221> MISC_FEATURE
<223> Partial sequence of IPF1, showing high score in the local alignme
      nt between IPF1 and TCF4, No.1
<400> 27
His His His Leu Pro Ala Gln Leu Ala Leu Pro His Pro Pro Ala
1               5                   10                  15

<210> 28
<211> 16
<212> PRT
<213> Homo sapiens
<220>
<221> MISC_FEATURE
<223> Partial sequence of IPF1, showing high score in the local alignme
      nt between IPF1 and TCF4, No.2
<400> 28
Arg Leu Pro Pro Gly Leu Ser Ala Ser Pro Gln Pro Ser Ser Val Ala
1               5                   10                  15

<210> 29

<211>  26
<212>  PRT
<213>  Homo sapiens
<220>
<221>  MISC_FEATURE
<223>  Partial sequence of IPF1, showing high score in the local alignme
       nt between IPF1 and TCF4, No.3
<400>  29
Gly Pro Ala Pro Glu Phe Ser Ala Ser Pro Pro Ala Cys Leu Tyr Met
1               5                   10                  15

Gly Arg Gln Pro Pro Pro Pro Pro Pro His
            20                  25

<210>  30
<211>  15
<212>  PRT
<213>  Homo sapiens
<220>
<221>  MISC_FEATURE
<223>  Partial sequence of TCF4 showing high score in the local alignmen
       t between IPF1 and TCF4, No.1
<400>  30
His Ser Leu Leu Pro Asn Gln Val Pro Val Pro Gln Leu Pro Val
1               5                   10                  15

<210>  31
<211>  16
<212>  PRT
<213>  Homo sapiens
<220>
<221>  MISC_FEATURE
<223>  Partial sequence of TCF4 showing high score in the local alignmen
       t between IPF1 and TCF4, No.2
<400>  31
Lys Val Pro Pro Gly Leu Pro Ser Ser Val Tyr Ala Pro Ser Ala Ser
1               5                   10                  15

<210>  32
<211>  26
<212>  PRT
<213>  Homo sapiens
<220>
<221>  MISC_FEATURE
<223>  Partial sequence of TCF4 showing high score in the local alignmen
       t between IPF1 and TCF4, No.3
<400>  32
Gly Ser Pro Pro Ser Leu Ser Ala Gly Thr Ala Val Trp Ser Arg Asn
1               5                   10                  15

Gly Gly Gln Ala Ser Ser Ser Pro Asn Tyr
            20                  25

<210>  33
<211>  14
<212>  PRT
<213>  Homo sapiens
<220>
<221>  MISC_FEATURE
<223>  Partial sequence of IPF1 showing high score in the local alignmen
       t between IPF1 and TMPO
<400>  33
Phe Gln Arg Gly Pro Ala Pro Glu Phe Ser Ala Ser Pro Pro

1                 5                   10

<210>  34
<211>  14
<212>  PRT
<213>  Homo sapiens
<220>
<221>  MISC_FEATURE
<223>  Partial sequence of TMPO showing high score in the local alignmen
       t between IPF1 and TMPO
<400>  34
Phe Gln Gly Ile Ser Phe Pro Glu Ile Ser Thr Arg Pro Pro
1               5                   10

<210>  35
<211>  37
<212>  DNA
<213>  Artificial Sequence
<220>
<223>  Designed oligonucleotide based on sequence of SEQ ID NO: 3 for use
        as a primer (Human HNF3G)
<400>  35
gctagcctgg gctcagtgaa gatggaggcc catgacc                              37

<210>  36
<211>  37
<212>  DNA
<213>  Artificial Sequence
<220>
<223>  Designed oligonucleotide based on sequence of SEQ ID NO: 3 for use
        as a reverse primer (Human HNF3G)
<400>  36
ctcgagctag gatgcattaa gcaaagagcg ggaatag                              37

<210>  37
<211>  26
<212>  DNA
<213>  Artificial Sequence
<220>
<223>  Designed oligonucleotide based on sequence of SEQ_ID NO:5 for use
       as a primer (Human PHF1)
<400>  37
gcgctagcgc gcagcccccc cggctg                                          26

<210>  38
<211>  26
<212>  DNA
<213>  Artificial Sequence
<220>
<223>  Designed oligonucleotide based on sequence of SEQ_ID NO:5 for use
       as a reverse primer (Human PHF1)

<400>  38
gcctcgagtc agaagatgcc ccctcc                                          26

<210>  39
<211>  29
<212>  DNA
<213>  Artificial Sequence
<220>
<223>  Designed oligonucleotide based on sequence of SEQ ID NO: 7 for use
        as a primer (Human DLX4)
<400>  39

acctgcagag caccctcagg aactcgagg                                    29

<210>  40
<211>  29
<212>  DNA
<213>  Artificial Sequence
<220>
<223>  Designed oligonucleotide based on sequence of SEQ ID NO: 7 for use
        as a reverse primer (Human DLX4)
<400>  40
gacagtctca actccagcca atggctctc                                    29

<210>  41
<211>  31
<212>  DNA
<213>  Artificial Sequence
<220>
<223>  Designed oligonucleotide based on mouse HNF3G gene for use as a pr
       imer
<400>  41
tgcctgtaga gagaccgaag cactcggttc c                                 31

<210>  42
<211>  30
<212>  DNA
<213>  Artificial Sequence
<220>
<223>  Designed oligonucleotide based on mouse HNF3G gene for use as a re
       verse primer
<400>  42
agctaatggg tcatatcaca gtaatagacc                                   30

<210>  43
<211>  30
<212>  DNA
<213>  Artificial Sequence
<220>
<223>  Designed oligonucleotide based on mouse PHF1 gene for use as a pri
       mer
<400>  43
gagactcatc caggtctctt gagtctgacc                                   30

<210>  44
<211>  30
<212>  DNA
<213>  Artificial Sequence
<220>
<223>  Designed oligonucleotide based on mouse PHF1 gene for use as a rev
       erse primer
<400>  44
tccaggtaca tcccagctgt aggtgaggtc                                   30

<210>  45
<211>  29
<212>  DNA
<213>  Artificial Sequence
<220>
<223>  Designed oligonucleotide based on mouse DLX4 gene for use as a pri
       mer
<400>  45
gctggcagaa agagtagaca gcgtgcggg                                    29

<210>  46

<211>  27
<212>  DNA
<213>  Artificial Sequence
<220>
<223>  Designed oligonucleotide based on mouse DLX4 gene for use as a rev
       erse primer
<400>  46
agcaggtgct cgggtcctgg gcttgac                                          27

<210>  47
<211>  11
<212>  PRT
<213>  Homo sapiens
<220>
<221>  MISC_FEATURE
<223>  Partial sequence of IPF1 showing high score in the local alignmen
       t between IPF1 and HNF3G, No.4
<400>  47
Pro Pro Gly Leu Ser Ala Ser Pro Gln Pro Ser
1               5                   10

<210>  48
<211>  7
<212>  PRT
<213>  Homo sapiens
<220>
<221>  MISC_FEATURE
<223>  Partial sequence of IPF1 showing high score in the local alignmen
       t between IPF1 and HNF3G, No.5
<400>  48
Glu Gly Ala Glu Pro Gly Val
1               5

<210>  49
<211>  8
<212>  PRT
<213>  Homo sapiens
<220>
<221>  MISC_FEATURE
<223>  Partial sequence of IPF1 showing high score in the local alignmen
       t between IPF1 and HNF3G, No.6
<400>  49
Pro Phe Pro Gly Ala Leu Gly Ala
1               5

<210>  50
<211>  11
<212>  PRT
<213>  Homo sapiens
<220>
<221>  MISC_FEATURE
<223>  Partial sequence of HNF3G showing high score in the local alignme
       nt between IPF1 and HNF3G, No.4
<400>  50
Pro Gly Gly Leu Pro Ala Ser Pro Leu Pro Ser
1               5                   10

<210>  51
<211>  7
<212>  PRT
<213>  Homo sapiens

<220>

<221>  MISC_FEATURE
<223>  Partial sequence of HNF3G showing high score in the local alignme
       nt between IPF1 and HNF3G, No.5
<400>  51
Glu Gly Gly Glu Pro Gly Val
1               5

<210>  52
<211>  8
<212>  PRT
<213>  Homo sapiens
<220>
<221>  MISC_FEATURE
<223>  Partial sequence of HNF3G showing high score in the local alignme
       nt between IPF1 and HNF3G, No.6
<400>  52
Pro Tyr Pro Gly Gly Leu Pro Ala
1               5

<210>  53
<211>  8
<212>  PRT
<213>  Homo sapiens
<220>
<221>  MISC_FEATURE
<223>  Partial sequence of IPF1 showing high score in the local alignmen
       t between IPF1 and PHF1, No.3
<400>  53
Pro Pro Asp Ile Ser Pro Tyr Glu
1               5

<210>  54
<211>  5
<212>  PRT
<213>  Homo sapiens
<220>
<221>  MISC_FEATURE
<223>  Partial sequence of IPF1 showing high score in the local alignmen
       t between IPF1 and PHF1, No.4
<400>  54
Gly Glu Glu Leu Leu
1               5

<210>  55
<211>  8
<212>  PRT
<213>  Homo sapiens
<220>
<221>  MISC_FEATURE
<223>  Partial sequence of PHF1 showing high score in the local alignmen
       t between IPF1 and PHF1, No.3
<400>  55
Pro Pro Asp Arg Ser Pro Leu Glu
1               5

<210>  56
<211>  5
<212>  PRT
<213>  Homo sapiens
<220>
<221>  MISC_FEATURE
<223>  Partial sequence of PHF1 showing high score in the local alignmen
       t between IPF1 and PHF1, No.4

<400>  56
Gly Glu Glu Leu Leu
1               5

<210>  57
<211>  14
<212>  PRT
<213>  Homo sapiens
<220>
<221>  MISC_FEATURE
<223>  Partial sequence of IPF1 showing high score in the local alignmen
       t between IPF1 and DLX4, No.3
<400>  57
Ile Lys Ile Trp Phe Gln Asn Arg Arg Met Lys Trp Lys Lys
1               5                   10

<210>  58
<211>  5
<212>  PRT
<213>  Homo sapiens
<220>
<221>  MISC_FEATURE
<223>  Partial sequence of IPF1 showing high score in the local alignmen
       t between IPF1 and DLX4, No.4
<400>  58
Ser Pro Gln Pro Ser
1               5

<210>  59
<211>  6
<212>  PRT
<213>  Homo sapiens
<220>
<221>  MISC_FEATURE
<223>  Partial sequence of IPF1 showing high score in the local alignmen
       t between IPF1 and DLX4, No.5
<400>  59
Arg Arg Pro Gln Glu Pro
1               5

<210>  60
<211>  14
<212>  PRT
<213>  Homo sapiens
<220>
<221>  MISC_FEATURE
<223>  Partial sequence of DLX4 showing high score in the local alignmen
       t between IPF1 and DLX4, No.3
<400>  60
Val Lys Ile Trp Phe Gln Asn Lys Arg Ser Lys Tyr Lys Lys
1               5                   10

<210>  61
<211>  5
<212>  PRT
<213>  Homo sapiens
<220>
<221>  MISC_FEATURE
<223>  Partial sequence of DLX4 showing high score in the local alignmen
       t between IPF1 and DLX4, No.4
<400>  61
Ser Pro Glu Pro Ser
1               5

<210>  62
<211>  6
<212>  PRT
<213>  Homo sapiens
<220>
<221>  MISC_FEATURE
<223>  Partial sequence of DLX4 showing high score in the local alignmen
       t between IPF1 and DLX4, No.5
<400>  62
Arg Arg Pro Gln Ala Pro
1               5

<210>  63
<211>  7
<212>  PRT
<213>  Homo sapiens
<220>
<221>  MISC_FEATURE
<223>  Partial sequence of IPF1 showing high score in the local alignmen
       t between IPF1 and TCF4, No.4
<400>  63
His His His Leu Pro Ala Gln
1               5

<210>  64
<211>  7
<212>  PRT
<213>  Homo sapiens
<220>
<221>  MISC_FEATURE
<223>  Partial sequence of IPF1 showing high score in the local alignmen
       t between IPF1 and TCF4, No.5
<400>  64
Pro Pro Gly Leu Ser Ala Ser
1               5

<210>  65
<211>  8
<212>  PRT
<213>  Homo sapiens
<220>
<221>  MISC_FEATURE
<223>  Partial sequence of IPF1 showing high score in the local alignmen
       t between IPF1 and TCF4, No.6
<400>  65
Gly Pro Ala Pro Glu Phe Ser Ala
1               5

<210>  66
<211>  7
<212>  PRT
<213>  Homo sapiens
<220>
<221>  MISC_FEATURE
<223>  Partial sequence of TCF4 showing high score in the local alignmen
       t between IPF1 and TCF4, No.4
<400>  66
His Ser Leu Leu Pro Asn Gln
1               5

<210>  67
<211>  7

<212>  PRT
<213>  Homo sapiens
<220>
<221>  MISC_FEATURE
<223>  Partial sequence of TCF4 showing high score in the local alignmen
       t between IPF1 and TCF4, No.5
<400>  67
Pro Pro Gly Leu Pro Ser Ser
1               5

<210>  68
<211>  8
<212>  PRT
<213>  Homo sapiens
<220>
<221>  MISC_FEATURE
<223>  Partial sequence of TCF4 showing high score in the local alignmen
       t between IPF1 and TCF4, No.6
<400>  68
Gly Ser Pro Pro Ser Leu Ser Ala
1               5

<210>  69
<211>  14
<212>  PRT
<213>  Homo sapiens
<220>
<221>  MISC_FEATURE
<223>  Partial sequence of IPF1 showing high score in the local alignmen
       t between IPF1 and TMPO, No.2
<400>  69
Phe Gln Arg Gly Pro Ala Pro Glu Phe Ser Ala Ser Pro Pro
1               5                   10

<210>  70
<211>  14
<212>  PRT
<213>  Homo sapiens
<220>
<221>  MISC_FEATURE
<223>  Partial sequence of TMPO showing high score in the local alignmen
       t between IPF1 and TMPO, No.2
<400>  70
Phe Gln Gly Ile Ser Phe Pro Glu Ile Ser Thr Arg Pro Pro
1               5                   10

SEQUENCE LISTING

<110> Celestar Lexico-Sciences, Inc.

Daiichi Pharmaceutical Co., Ltd.

<120> An inhibitor of interaction between IPF1 and IPF1-binding proteins

<130> A41830M

<160> 70

<170> PatentIn version 3.1

<210> 1

<211> 852

<212> DNA

<213> Homo sapiens

<220>

<221> CDS

<222> (1)..(852)

<223>

<400> 1
atg aac ggc gag gag cag tac tac gcg gcc acg cag ctt tac aag gac 48
cca tgc gcg ttc cag cga ggc ccg gcg ccg gag ttc agc gcc agc ccc 96
cct gcg tgc ctg tac atg ggc cgc cag ccc ccg ccg ccg ccg ccg cac 144
ccg ttc cct ggc gcc ctg ggc gcg ctg gag cag ggc agc ccc ccg gac 192
atc tcc ccg tac gag gtg ccc ccc ctc gcc gac gac ccc gcg gtg gcg 240
cac ctt cac cac cac ctc ccg gct cag ctc gcg ctc ccc cac ccg ccc 288
gcc ggg ccc ttc ccg gag gga gcc gag ccg ggc gtc ctg gag gag ccc 336
aac cgc gtc cag ctg cct ttc cca tgg atg aag tct acc aaa gct cac 384
gcg tgg aaa ggc cag tgg gca ggc ggc gcc tac gct gcg gag ccg gag 432
gag aac aag cgg acg cgc acg gcc tac acg cgc gca cag ctg cta gag 480
ctg gag aag gag ttc cta ttc aac aag tac atc tca cgg ccg cgc cgg     528
gtg gag ctg gct gtc atg ttg aac ttg acc gag aga cac atc aag atc     576
tgg ttc caa aac cgc cgc atg aag tgg aaa aag gag gag gac aag aag     624
cgc ggc ggc ggg aca gct gtc ggg ggt ggc ggg gtc gcg gag cct gag     672
cag gac tgc gcc gtg acc tcc ggc gag gag ctt ctg gcg ctg ccg ccg     720
ccg ccg ccc ccc gga ggt gct gtg ccg ccc gct gcc ccc gtt gcc gcc     768
cga gag ggc cgc ctg ccg cct ggc ctt agc gcg tcg cca cag ccc tcc 816
agc gtc gcg cct cgg cgg ccg cag gaa cca cga tga 852
<210> 2
<211> 283
<212> PRT
<213> Homo sapiens
<400> 2

<210>  3
<211>  1053
<212>  DNA
<213>  Homo sapiens
<220>
<221>  CDS
<222>  (1)..(1053)
<223>
<400>  3
atg ctg ggc tca gtg aag atg gag gcc cat gac ctg gcc gag tgg agc        48
Met Leu Gly Ser Val Lys Met Glu Ala His Asp Leu Ala Glu Trp Ser
1               5                   10                  15

tac tac ccg gag gcg ggc gag gtc tac tcg ccg gtg acc cca gtg ccc        96

Tyr Tyr Pro Glu Ala Gly Glu Val Tyr Ser Pro Val Thr Pro Val Pro
            20                  25                  30

acc atg gcc ccc ctc aac tcc tac atg acc ctg aat cct cta agc tct 144
Thr Met Ala Pro Leu Asn Ser Tyr Met Thr Leu Asn Pro Leu Ser Ser
        35                  40                  45

ccc tat ccc cct ggg ggg ctc cct gcc tcc cca ctg ccc tca gga ccc 192
Pro Tyr Pro Pro Gly Gly Leu Pro Ala Ser Pro Leu Pro Ser Gly Pro
    50                  55                  60

ctg gca ccc cca gca cct gca gcc ccc ctg ggg ccc act ttc cca ggc 240
Leu Ala Pro Pro Ala Pro Ala Ala Pro Leu Gly Pro Thr Phe Pro Gly
65                  70                  75                  80

ctg ggt gtc agc ggt ggc agc agc agc tcc ggg tac ggg gcc ccg ggt 288
Leu Gly Val Ser Gly Gly Ser Ser Ser Ser Gly Tyr Gly Ala Pro Gly
                85                  90                  95

cct ggg ctg gtg cac ggg aag gag atg ccg aag ggg tat cgg cgg ccc 336
Pro Gly Leu Val His Gly Lys Glu Met Pro Lys Gly Tyr Arg Arg Pro
            100                 105                 110

ctg gca cac gcc aag cca ccg tat tcc tat atc tca ctc atc acc atg 384
Leu Ala His Ala Lys Pro Pro Tyr Ser Tyr Ile Ser Leu Ile Thr Met
        115                 120                 125

gcc atc cag cag gcg ccg ggc aag atg ctg acc ttg agt gaa atc tac 432
Ala Ile Gln Gln Ala Pro Gly Lys Met Leu Thr Leu Ser Glu Ile Tyr
    130                 135                 140

cag tgg atc atg gac ctc ttc cct tac tac cgg gag aat cag cag cgc 480
Gln Trp Ile Met Asp Leu Phe Pro Tyr Tyr Arg Glu Asn Gln Gln Arg
145                 150                 155                 160

tgg cag aac tcc att cgc cac tcg ctg tct ttc aac gac tgc ttc gtc 528
Trp Gln Asn Ser Ile Arg His Ser Leu Ser Phe Asn Asp Cys Phe Val
                165                 170                 175

aag gtg gcg cgt tcc cca gac aag cct ggc aag ggc tcc tac tgg gcc 576
Lys Val Ala Arg Ser Pro Asp Lys Pro Gly Lys Gly Ser Tyr Trp Ala
            180                 185                 190

cta cac ccc agc tca ggg aac atg ttt gag aat ggc tgc tac ctg cgc 624
Leu His Pro Ser Ser Gly Asn Met Phe Glu Asn Gly Cys Tyr Leu Arg
        195                 200                 205

cgc cag aaa cgc ttc aag ctg gag gag aag gtg aaa aaa ggg ggc agc 672
Arg Gln Lys Arg Phe Lys Leu Glu Glu Lys Val Lys Lys Gly Gly Ser
    210                 215                 220

ggg gct gcc acc acc acc agg aac ggg aca ggg tct gct gcc tcg acc 720
Gly Ala Ala Thr Thr Thr Arg Asn Gly Thr Gly Ser Ala Ala Ser Thr
225                 230                 235                 240

acc acc ccc gcg gcc aca gtc acc tcc ccg ccc cag ccc ccg cct cca 768
Thr Thr Pro Ala Ala Thr Val Thr Ser Pro Pro Gln Pro Pro Pro Pro
                245                 250                 255

gcc cct gag cct gag gcc cag ggc ggg gaa gat gtg ggg gct ctg gac 816
Ala Pro Glu Pro Glu Ala Gln Gly Gly Glu Asp Val Gly Ala Leu Asp
            260                 265                 270

tgt ggc tca ccc gct tcc tcc aca ccc tat ttc act ggc ctg gag ctc 864
Cys Gly Ser Pro Ala Ser Ser Thr Pro Tyr Phe Thr Gly Leu Glu Leu
        275                 280                 285

cca ggg gag ctg aag ctg gac gcg ccc tac aac ttc aac cac cct ttc 912
Pro Gly Glu Leu Lys Leu Asp Ala Pro Tyr Asn Phe Asn His Pro Phe
    290                 295                 300

tcc atc aac aac cta atg tca gaa cag aca cca gca cct ccc aaa ctg 960
Ser Ile Asn Asn Leu Met Ser Glu Gln Thr Pro Ala Pro Pro Lys Leu
305                 310                 315                 320

gac gtg ggg ttt ggg ggc tac ggg gct gaa ggt ggg gag cct gga gtc 1008
Asp Val Gly Phe Gly Gly Tyr Gly Ala Glu Gly Gly Glu Pro Gly Val
                325                 330                 335

tac tac cag ggc ctc tat tcc cgc tct ttg ctt aat gca tcc tag 1053
Tyr Tyr Gln Gly Leu Tyr Ser Arg Ser Leu Leu Asn Ala Ser

            340                 345                 350

<210> 4

<211> 350

<212> PRT

<213> Homo sapiens

<400> 4

Met Leu Gly Ser Val Lys Met Glu Ala His Asp Leu Ala Glu Trp Ser
1               5                   10                  15

Tyr Tyr Pro Glu Ala Gly Glu Val Tyr Ser Pro Val Thr Pro Val Pro
            20                  25                  30

Thr Met Ala Pro Leu Asn Ser Tyr Met Thr Leu Asn Pro Leu Ser Ser
        35                  40                  45

Pro Tyr Pro Pro Gly Gly Leu Pro Ala Ser Pro Leu Pro Ser Gly Pro
    50                  55                  60

Leu Ala Pro Pro Ala Pro Ala Ala Pro Leu Gly Pro Thr Phe Pro Gly
65                  70                  75                  80

Leu Gly Val Ser Gly Gly Ser Ser Ser Ser Gly Tyr Gly Ala Pro Gly
                85                  90                  95

Pro Gly Leu Val His Gly Lys Glu Met Pro Lys Gly Tyr Arg Arg Pro

            100                 105                 110

Leu Ala His Ala Lys Pro Pro Tyr Ser Tyr Ile Ser Leu Ile Thr Met

        115                 120                 125

Ala Ile Gln Gln Ala Pro Gly Lys Met Leu Thr Leu Ser Glu Ile Tyr

    130                 135                 140

Gln Trp Ile Met Asp Leu Phe Pro Tyr Tyr Arg Glu Asn Gln Gln Arg

145                 150                 155                 160

Trp Gln Asn Ser Ile Arg His Ser Leu Ser Phe Asn Asp Cys Phe Val

                165                 170                 175

Lys Val Ala Arg Ser Pro Asp Lys Pro Gly Lys Gly Ser Tyr Trp Ala

            180                 185                 190

Leu His Pro Ser Ser Gly Asn Met Phe Glu Asn Gly Cys Tyr Leu Arg

        195                 200                 205

Arg Gln Lys Arg Phe Lys Leu Glu Glu Lys Val Lys Lys Gly Gly Ser

    210                 215                 220

Gly Ala Ala Thr Thr Thr Arg Asn Gly Thr Gly Ser Ala Ala Ser Thr

225                 230                 235                 240

Thr Thr Pro Ala Ala Thr Val Thr Ser Pro Pro Gln Pro Pro Pro Pro

                245                 250                 255

Ala Pro Glu Pro Glu Ala Gln Gly Gly Glu Asp Val Gly Ala Leu Asp

            260                 265                 270

Cys Gly Ser Pro Ala Ser Ser Thr Pro Tyr Phe Thr Gly Leu Glu Leu

        275                 280                 285

Pro Gly Glu Leu Lys Leu Asp Ala Pro Tyr Asn Phe Asn His Pro Phe

    290                 295                 300

Ser Ile Asn Asn Leu Met Ser Glu Gln Thr Pro Ala Pro Pro Lys Leu

305                 310                 315                 320

Asp Val Gly Phe Gly Gly Tyr Gly Ala Glu Gly Gly Glu Pro Gly Val

                325                 330                 335

Tyr Tyr Gln Gly Leu Tyr Ser Arg Ser Leu Leu Asn Ala Ser

            340                 345                 350

<210> 5

<211> 1704

<212> DNA

<213> Homo sapiens

<220>

<221> CDS

<222> (1)..(1704)

<223>

<400> 5

atg gcg cag ccc ccc cgg ctg agc cgc tct ggt gcc tcc tca ctt tgg 48

Met Ala Gln Pro Pro Arg Leu Ser Arg Ser Gly Ala Ser Ser Leu Trp

1               5                   10                  15

gac cca gct tct cct gct ccc acc tct ggc ccc agg cct cgg ctt tgg 96

Asp Pro Ala Ser Pro Ala Pro Thr Ser Gly Pro Arg Pro Arg Leu Trp

            20                  25                  30

gag ggt caa gat gtg ctg gcc aga tgg act gat ggg ctg cta tac ttg 144

Glu Gly Gln Asp Val Leu Ala Arg Trp Thr Asp Gly Leu Leu Tyr Leu

        35                  40                  45

ggt acc atc aaa aag gtg gac agt gct agg gag gtg tgt ctg gtc cag 192

Gly Thr Ile Lys Lys Val Asp Ser Ala Arg Glu Val Cys Leu Val Gln

    50                  55                  60

ttt gag gat gat tcg cag ttt ctg gtt cta tgg aaa gac att agc cct 240

Phe Glu Asp Asp Ser Gln Phe Leu Val Leu Trp Lys Asp Ile Ser Pro

65                  70                  75                  80

gct gcc ctc cct gga gag gaa ctc ctc tgt tgt gtc tgt cgc tct gag 288

Ala Ala Leu Pro Gly Glu Glu Leu Leu Cys Cys Val Cys Arg Ser Glu

                85                  90                  95

act gtg gtc cct ggg aac cgg ttg gtc agc tgt gag aag tgt cgc cat 336

Thr Val Val Pro Gly Asn Arg Leu Val Ser Cys Glu Lys Cys Arg His
            100                 105                 110

gct tat cac cag gac tgc cat gtt ccc agg gct cca gcc cct gga gag 384
Ala Tyr His Gln Asp Cys His Val Pro Arg Ala Pro Ala Pro Gly Glu
        115                 120                 125

gga gag ggc aca tcc tgg gta tgc cgc cag tgt gtc ttt gcg atc gcc 432
Gly Glu Gly Thr Ser Trp Val Cys Arg Gln Cys Val Phe Ala Ile Ala
    130                 135                 140

acc aag agg gga ggt gcc ctg aag aag ggc ccc tat gcc cgg gcc atg 480
Thr Lys Arg Gly Gly Ala Leu Lys Lys Gly Pro Tyr Ala Arg Ala Met
145                 150                 155                 160

ctg ggt atg aag ctt tct ctg cca tat gga ctg aag ggg ctg gac tgg 528
Leu Gly Met Lys Leu Ser Leu Pro Tyr Gly Leu Lys Gly Leu Asp Trp
                165                 170                 175

gat gct gga cat ctg agc aac cga cag cag agt tac tgt tac tgt ggt 576
Asp Ala Gly His Leu Ser Asn Arg Gln Gln Ser Tyr Cys Tyr Cys Gly
            180                 185                 190

ggc cct ggg gag tgg aac ctg aaa atg ctg cag tgc cgg agc tgc ctg 624
Gly Pro Gly Glu Trp Asn Leu Lys Met Leu Gln Cys Arg Ser Cys Leu
        195                 200                 205

cag tgg ttc cat gag gcc tgc acc cag tgt ctg agc aag ccc ctc ctc 672
Gln Trp Phe His Glu Ala Cys Thr Gln Cys Leu Ser Lys Pro Leu Leu
    210                 215                 220

tat ggg gac agg ttc tat gaa ttt gaa tgc tgt gtg tgt cgc ggg ggc 720
Tyr Gly Asp Arg Phe Tyr Glu Phe Glu Cys Cys Val Cys Arg Gly Gly
225                 230                 235                 240

cct gag aaa gtc cgg aga cta cag ctt cgc tgg gtg gat gtg gcc cat 768
Pro Glu Lys Val Arg Arg Leu Gln Leu Arg Trp Val Asp Val Ala His
                245                 250                 255

ctt gtc ctg tat cac ctc agt gtt tgc tgt aag aag aaa tac ttt gat 816
Leu Val Leu Tyr His Leu Ser Val Cys Cys Lys Lys Lys Tyr Phe Asp
            260                 265                 270

ttt gat cgt gag atc ctc ccc ttc act tct gag aat tgg gac agt ttg 864
Phe Asp Arg Glu Ile Leu Pro Phe Thr Ser Glu Asn Trp Asp Ser Leu
        275                 280                 285

ctc ctg ggg gag ctt tca gac acc ccc aaa gga gaa cgt tct tcc aag 912
Leu Leu Gly Glu Leu Ser Asp Thr Pro Lys Gly Glu Arg Ser Ser Lys
    290                 295                 300

ctc ctc tct gct ctt aac agc cac aag gac cgt ttc att tca ggg aga 960
Leu Leu Ser Ala Leu Asn Ser His Lys Asp Arg Phe Ile Ser Gly Arg
305                 310                 315                 320

gag att aag aag agg aaa tgt ttg ttt ggt ctc cat gct cgg atg cct 1008
Glu Ile Lys Lys Arg Lys Cys Leu Phe Gly Leu His Ala Arg Met Pro
                325                 330                 335

ccc cct gtg gag ccc cct act gga gat gga gca ctc acc agc ttc cct 1056
Pro Pro Val Glu Pro Pro Thr Gly Asp Gly Ala Leu Thr Ser Phe Pro
            340                 345                 350

tca ggg cag ggc cct ggg gga ggg gtc tca cgt ccc ctg ggg aag cgc 1104
Ser Gly Gln Gly Pro Gly Gly Gly Val Ser Arg Pro Leu Gly Lys Arg
        355                 360                 365

cgg agg ccg gag cca gag ccc ctg agg agg agg cag aag ggg aaa gtg 1152
Arg Arg Pro Glu Pro Glu Pro Leu Arg Arg Arg Gln Lys Gly Lys Val
    370                 375                 380

gag gag ctg ggg cca ccc tca gca gtg cgc aat cag ccc gag ccc cag 1200
Glu Glu Leu Gly Pro Pro Ser Ala Val Arg Asn Gln Pro Glu Pro Gln
385                 390                 395                 400

gag cag agg gag cgg gct cat ctg cag agg gca ctg cag gcc tca gtg 1248
Glu Gln Arg Glu Arg Ala His Leu Gln Arg Ala Leu Gln Ala Ser Val
                405                 410                 415

tct cca cca tcc ccc agc cct aac cag agt tac cag ggc agc agc ggc 1296
Ser Pro Pro Ser Pro Ser Pro Asn Gln Ser Tyr Gln Gly Ser Ser Gly

            420                 425                 430

tac aac ttc cgg ccc aca gat gcc cgc tgc ctg ccc agc agc ccc atc 1344
Tyr Asn Phe Arg Pro Thr Asp Ala Arg Cys Leu Pro Ser Ser Pro Ile
        435                 440                 445

cgg atg ttt gct tcc ttc cac cct tct gcc agc acc gca ggg acc tct 1392
Arg Met Phe Ala Ser Phe His Pro Ser Ala Ser Thr Ala Gly Thr Ser
    450                 455                 460

ggg gac agt gga ccc cca gac agg tca ccc ctg gaa ctt cac att ggt 1440
Gly Asp Ser Gly Pro Pro Asp Arg Ser Pro Leu Glu Leu His Ile Gly
465                 470                 475                 480

ttc ccc aca gac atc cct aaa agt gcc ccc cac tcg atg act gcc tca 1488
Phe Pro Thr Asp Ile Pro Lys Ser Ala Pro His Ser Met Thr Ala Ser
                485                 490                 495

tct tcc tca gtt tca tcc cca tcc cca ggt ctt cct aga cgc tca gca 1536
Ser Ser Ser Val Ser Ser Pro Ser Pro Gly Leu Pro Arg Arg Ser Ala
            500                 505                 510

ccc cct tct ccc ctg tgc cgt agt ttg tct cct ggg act ggg gga gga 1584
Pro Pro Ser Pro Leu Cys Arg Ser Leu Ser Pro Gly Thr Gly Gly Gly
        515                 520                 525

gtc cga ggt ggg gtt ggt tac ctg tcc cga ggg gac cct gtc cgg gtc 1632

Val Arg Gly Gly Val Gly Tyr Leu Ser Arg Gly Asp Pro Val Arg Val
    530                 535                 540

ctt gct cgg aga gta cgg cct gat ggc tct gtg cag tac ctg gtt gag 1680
Leu Ala Arg Arg Val Arg Pro Asp Gly Ser Val Gln Tyr Leu Val Glu
545                 550                 555                 560

tgg gga gga ggg ggc atc ttc tga 1704
Trp Gly Gly Gly Gly Ile Phe
                565

<210> 6
<211> 567
<212> PRT
<213> Homo sapiens
<400> 6

Met Ala Gln Pro Pro Arg Leu Ser Arg Ser Gly Ala Ser Ser Leu Trp
1               5                   10                  15

Asp Pro Ala Ser Pro Ala Pro Thr Ser Gly Pro Arg Pro Arg Leu Trp
            20                  25                  30

Glu Gly Gln Asp Val Leu Ala Arg Trp Thr Asp Gly Leu Leu Tyr Leu
        35                  40                  45

Gly Thr Ile Lys Lys Val Asp Ser Ala Arg Glu Val Cys Leu Val Gln

    50                  55                  60

Phe Glu Asp Asp Ser Gln Phe Leu Val Leu Trp Lys Asp Ile Ser Pro
65                  70                  75                  80

Ala Ala Leu Pro Gly Glu Glu Leu Leu Cys Cys Val Cys Arg Ser Glu
                85                  90                  95

Thr Val Val Pro Gly Asn Arg Leu Val Ser Cys Glu Lys Cys Arg His
            100                 105                 110

Ala Tyr His Gln Asp Cys His Val Pro Arg Ala Pro Ala Pro Gly Glu
        115                 120                 125

Gly Glu Gly Thr Ser Trp Val Cys Arg Gln Cys Val Phe Ala Ile Ala
    130                 135                 140

Thr Lys Arg Gly Gly Ala Leu Lys Lys Gly Pro Tyr Ala Arg Ala Met
145                 150                 155                 160

Leu Gly Met Lys Leu Ser Leu Pro Tyr Gly Leu Lys Gly Leu Asp Trp
                165                 170                 175

Asp Ala Gly His Leu Ser Asn Arg Gln Gln Ser Tyr Cys Tyr Cys Gly
            180                 185                 190

Gly Pro Gly Glu Trp Asn Leu Lys Met Leu Gln Cys Arg Ser Cys Leu

        195                 200                 205

Gln Trp Phe His Glu Ala Cys Thr Gln Cys Leu Ser Lys Pro Leu Leu

    210                 215                 220

Tyr Gly Asp Arg Phe Tyr Glu Phe Glu Cys Cys Val Cys Arg Gly Gly

225                 230                 235                 240

Pro Glu Lys Val Arg Arg Leu Gln Leu Arg Trp Val Asp Val Ala His

                245                 250                 255

Leu Val Leu Tyr His Leu Ser Val Cys Cys Lys Lys Lys Tyr Phe Asp

            260                 265                 270

Phe Asp Arg Glu Ile Leu Pro Phe Thr Ser Glu Asn Trp Asp Ser Leu

        275                 280                 285

Leu Leu Gly Glu Leu Ser Asp Thr Pro Lys Gly Glu Arg Ser Ser Lys

    290                 295                 300

Leu Leu Ser Ala Leu Asn Ser His Lys Asp Arg Phe Ile Ser Gly Arg

305                 310                 315                 320

Glu Ile Lys Lys Arg Lys Cys Leu Phe Gly Leu His Ala Arg Met Pro

                325                 330                 335

Pro Pro Val Glu Pro Pro Thr Gly Asp Gly Ala Leu Thr Ser Phe Pro

            340                 345                 350

Ser Gly Gln Gly Pro Gly Gly Gly Val Ser Arg Pro Leu Gly Lys Arg
        355                 360                 365

Arg Arg Pro Glu Pro Glu Pro Leu Arg Arg Arg Gln Lys Gly Lys Val
    370                 375                 380

Glu Glu Leu Gly Pro Pro Ser Ala Val Arg Asn Gln Pro Glu Pro Gln
385                 390                 395                 400

Glu Gln Arg Glu Arg Ala His Leu Gln Arg Ala Leu Gln Ala Ser Val
                405                 410                 415

Ser Pro Pro Ser Pro Ser Pro Asn Gln Ser Tyr Gln Gly Ser Ser Gly
            420                 425                 430

Tyr Asn Phe Arg Pro Thr Asp Ala Arg Cys Leu Pro Ser Ser Pro Ile
        435                 440                 445

Arg Met Phe Ala Ser Phe His Pro Ser Ala Ser Thr Ala Gly Thr Ser
    450                 455                 460

Gly Asp Ser Gly Pro Pro Asp Arg Ser Pro Leu Glu Leu His Ile Gly
465                 470                 475                 480

Phe Pro Thr Asp Ile Pro Lys Ser Ala Pro His Ser Met Thr Ala Ser

                485                 490                 495

Ser Ser Ser Val Ser Ser Pro Ser Pro Gly Leu Pro Arg Arg Ser Ala

            500                 505                 510

Pro Pro Ser Pro Leu Cys Arg Ser Leu Ser Pro Gly Thr Gly Gly Gly

        515                 520                 525

Val Arg Gly Gly Val Gly Tyr Leu Ser Arg Gly Asp Pro Val Arg Val

    530                 535                 540

Leu Ala Arg Arg Val Arg Pro Asp Gly Ser Val Gln Tyr Leu Val Glu

545                 550                 555                 560

Trp Gly Gly Gly Gly Ile Phe

                565

<210> 7

<211> 723

<212> DNA

<213> Homo sapiens

<220>

<221> CDS

<222> (1)..(723)

<223>

<400> 7

atg acc tct ttg ccc tgc ccc ctc ccc ggc cgg gac gcc tcc aaa gct 48

Met Thr Ser Leu Pro Cys Pro Leu Pro Gly Arg Asp Ala Ser Lys Ala
1               5                   10                  15

gtc ttc cca gac ctc gcc cct gtc ccg tcg gta gcg gct gcc tac ccg     96
Val Phe Pro Asp Leu Ala Pro Val Pro Ser Val Ala Ala Ala Tyr Pro
            20                  25                  30

ctt ggc ttg tcc cct aca acc gca gcc tcc ccc aat ttg tcc tac tcc     144
Leu Gly Leu Ser Pro Thr Thr Ala Ala Ser Pro Asn Leu Ser Tyr Ser
        35                  40                  45

agg ccg tat ggc cac ctc ctg tct tac ccc tac acc gag cca gcg aac     192
Arg Pro Tyr Gly His Leu Leu Ser Tyr Pro Tyr Thr Glu Pro Ala Asn
    50                  55                  60

ccc gga gac tcc tac ctg tcc tgc cag caa ccc gcg gcg ctc tct cag     240
Pro Gly Asp Ser Tyr Leu Ser Cys Gln Gln Pro Ala Ala Leu Ser Gln
65                  70                  75                  80

ccc ctc tgc gga cct gca gag cac cct cag gaa ctc gag gca gac tcg     288
Pro Leu Cys Gly Pro Ala Glu His Pro Gln Glu Leu Glu Ala Asp Ser
                85                  90                  95

gag aag ccg cgg ctg tcc ccg gaa ccc tcc gag cgg cgc cct cag gcc     336
Glu Lys Pro Arg Leu Ser Pro Glu Pro Ser Glu Arg Arg Pro Gln Ala
            100                 105                 110

ccc gcc aaa aag ctc cgc aag ccg agg acc atc tac tcc agc ctg cag 384
Pro Ala Lys Lys Leu Arg Lys Pro Arg Thr Ile Tyr Ser Ser Leu Gln
        115                 120                 125

ctg cag cac cta aac cag cgt ttc cag cac acg cag tac ctg gcg ctg 432
Leu Gln His Leu Asn Gln Arg Phe Gln His Thr Gln Tyr Leu Ala Leu
    130                 135                 140

ccc gag agg gcc cag ctg gca gcg cag ctc ggc ctc acc cag acc cag 480
Pro Glu Arg Ala Gln Leu Ala Ala Gln Leu Gly Leu Thr Gln Thr Gln
145                 150                 155                 160

gta aag atc tgg ttt cag aac aaa cgc tcc aag tat aag aag ctc ctg 528
Val Lys Ile Trp Phe Gln Asn Lys Arg Ser Lys Tyr Lys Lys Leu Leu
                165                 170                 175

aag cag aat tct ggg ggg cag gaa ggg gac ttc cct ggg agg acc ttc 576
Lys Gln Asn Ser Gly Gly Gln Glu Gly Asp Phe Pro Gly Arg Thr Phe
            180                 185                 190

tct gtg tct ccc tgc tcc cca ccc ctc ccc tcc ctc tgg gat cta ccc 624
Ser Val Ser Pro Cys Ser Pro Pro Leu Pro Ser Leu Trp Asp Leu Pro
        195                 200                 205

aag gca ggg acc ctg ccc acc agt ggc tat ggc aac agc ttt gga gcc 672
Lys Ala Gly Thr Leu Pro Thr Ser Gly Tyr Gly Asn Ser Phe Gly Ala
    210                 215                 220

tgg tat cag cat cac tcc tca gat gtc ctg gct tcg cct cag atg atg          720
Trp Tyr Gln His His Ser Ser Asp Val Leu Ala Ser Pro Gln Met Met
225                 230                 235                 240

tga                                                                     723

<210>  8
<211>  240
<212>  PRT
<213>  Homo sapiens

<400>  8

Met Thr Ser Leu Pro Cys Pro Leu Pro Gly Arg Asp Ala Ser Lys Ala
1               5                   10                  15

Val Phe Pro Asp Leu Ala Pro Val Pro Ser Val Ala Ala Ala Tyr Pro
            20                  25                  30

Leu Gly Leu Ser Pro Thr Thr Ala Ala Ser Pro Asn Leu Ser Tyr Ser
        35                  40                  45

Arg Pro Tyr Gly His Leu Leu Ser Tyr Pro Tyr Thr Glu Pro Ala Asn
    50                  55                  60

Pro Gly Asp Ser Tyr Leu Ser Cys Gln Gln Pro Ala Ala Leu Ser Gln

65                  70                  75                  80

Pro Leu Cys Gly Pro Ala Glu His Pro Gln Glu Leu Glu Ala Asp Ser
                85                  90                  95

Glu Lys Pro Arg Leu Ser Pro Glu Pro Ser Glu Arg Arg Pro Gln Ala
            100                 105                 110

Pro Ala Lys Lys Leu Arg Lys Pro Arg Thr Ile Tyr Ser Ser Leu Gln
        115                 120                 125

Leu Gln His Leu Asn Gln Arg Phe Gln His Thr Gln Tyr Leu Ala Leu
    130                 135                 140

Pro Glu Arg Ala Gln Leu Ala Ala Gln Leu Gly Leu Thr Gln Thr Gln
145                 150                 155                 160

Val Lys Ile Trp Phe Gln Asn Lys Arg Ser Lys Tyr Lys Lys Leu Leu
                165                 170                 175

Lys Gln Asn Ser Gly Gly Gln Glu Gly Asp Phe Pro Gly Arg Thr Phe
            180                 185                 190

Ser Val Ser Pro Cys Ser Pro Pro Leu Pro Ser Leu Trp Asp Leu Pro
        195                 200                 205

Lys Ala Gly Thr Leu Pro Thr Ser Gly Tyr Gly Asn Ser Phe Gly Ala

210                 215                 220

Trp Tyr Gln His His Ser Ser Asp Val Leu Ala Ser Pro Gln Met Met
225                 230                 235                 240

<210>  9
<211>  2004
<212>  DNA
<213>  Homo sapiens
<220>
<221>  CDS
<222>  (1)..(2004)
<223>
<400>  9
atg cat cac caa cag cga atg gct gcc tta ggg acg gac aaa gag ctg       48
Met His His Gln Gln Arg Met Ala Ala Leu Gly Thr Asp Lys Glu Leu
1               5                   10                  15

agt gat tta ctg gat ttc agt gcg atg ttt tca cct cct gtg agc agt       96
Ser Asp Leu Leu Asp Phe Ser Ala Met Phe Ser Pro Pro Val Ser Ser
            20                  25                  30

ggg aaa aat gga cca act tct ttg gca agt gga cat ttt act ggc tca      144
Gly Lys Asn Gly Pro Thr Ser Leu Ala Ser Gly His Phe Thr Gly Ser
        35                  40                  45

aat gta gaa gac aga agt agc tca ggg tcc tgg ggg aat gga gga cat      192

Asn Val Glu Asp Arg Ser Ser Ser Gly Ser Trp Gly Asn Gly Gly His

    50                  55                  60

cca agc ccg tcc agg aac tat gga gat ggg act ccc tat gac cac atg 240

Pro Ser Pro Ser Arg Asn Tyr Gly Asp Gly Thr Pro Tyr Asp His Met

65                  70                  75                  80

acc agc agg gac ctt ggg tca cat gac aat ctc tct cca cct ttt gtc 288

Thr Ser Arg Asp Leu Gly Ser His Asp Asn Leu Ser Pro Pro Phe Val

                85                  90                  95

aat tcc aga ata caa agt aaa aca gaa agg ggc tca tac tca tct tat 336

Asn Ser Arg Ile Gln Ser Lys Thr Glu Arg Gly Ser Tyr Ser Ser Tyr

            100                 105                 110

ggg aga gaa tca aac tta cag ggt tgc cac cag cag agt ctc ctt gga 384

Gly Arg Glu Ser Asn Leu Gln Gly Cys His Gln Gln Ser Leu Leu Gly

        115                 120                 125

ggt gac atg gat atg ggc aac cca gga acc ctt tcg ccc acc aaa cct 432

Gly Asp Met Asp Met Gly Asn Pro Gly Thr Leu Ser Pro Thr Lys Pro

    130                 135                 140

ggt tcc cag tac tat cag tat tct agc aat aat ccc cga agg agg cct 480

Gly Ser Gln Tyr Tyr Gln Tyr Ser Ser Asn Asn Pro Arg Arg Arg Pro

145                 150                 155                 160

ctt cac agt agt gcc atg gag gta cag aca aag aaa gtt cga aaa gtt 528
Leu His Ser Ser Ala Met Glu Val Gln Thr Lys Lys Val Arg Lys Val
                165                 170                 175

cct cca ggt ttg cca tct tca gtc tat gct cca tca gca agc act gcc 576
Pro Pro Gly Leu Pro Ser Ser Val Tyr Ala Pro Ser Ala Ser Thr Ala
            180                 185                 190

gac tac aat agg gac tcg cca ggc tat cct tcc tcc aaa cca gca acc 624
Asp Tyr Asn Arg Asp Ser Pro Gly Tyr Pro Ser Ser Lys Pro Ala Thr
        195                 200                 205

agc act ttc cct agc tcc ttc ttc atg caa gat ggc cat cac agc agt 672
Ser Thr Phe Pro Ser Ser Phe Phe Met Gln Asp Gly His His Ser Ser
    210                 215                 220

gac cct tgg agc tcc tcc agt ggg atg aat cag cct ggc tat gca gga 720
Asp Pro Trp Ser Ser Ser Ser Gly Met Asn Gln Pro Gly Tyr Ala Gly
225                 230                 235                 240

atg ttg ggc aac tct tct cat att cca cag tcc agc agc tac tgt agc 768
Met Leu Gly Asn Ser Ser His Ile Pro Gln Ser Ser Ser Tyr Cys Ser
                245                 250                 255

ctg cat cca cat gaa cgt ttg agc tat cca tca cac tcc tca gca gac 816
Leu His Pro His Glu Arg Leu Ser Tyr Pro Ser His Ser Ser Ala Asp
            260                 265                 270

atc aat tcc agt ctt cct ccg atg tcc act ttc cat cgt agt ggt aca 864
Ile Asn Ser Ser Leu Pro Pro Met Ser Thr Phe His Arg Ser Gly Thr
        275                 280                 285

aac cat tac agc acc tct tcc tgt acg cct cct gcc aac ggg aca gac 912
Asn His Tyr Ser Thr Ser Ser Cys Thr Pro Pro Ala Asn Gly Thr Asp
    290                 295                 300

agt ata atg gca aat aga gga agc ggg gca gcc ggc agc tcc cag act 960
Ser Ile Met Ala Asn Arg Gly Ser Gly Ala Ala Gly Ser Ser Gln Thr
305                 310                 315                 320

gga gat gct ctg ggg aaa gca ctt gct tcg atc tat tct cca gat cac 1008
Gly Asp Ala Leu Gly Lys Ala Leu Ala Ser Ile Tyr Ser Pro Asp His
                325                 330                 335

act aac aac agc ttt tca tca aac cct tca act cct gtt ggc tct cct 1056
Thr Asn Asn Ser Phe Ser Ser Asn Pro Ser Thr Pro Val Gly Ser Pro
            340                 345                 350

cca tct ctc tca gca ggc aca gct gtt tgg tct aga aat gga gga cag 1104
Pro Ser Leu Ser Ala Gly Thr Ala Val Trp Ser Arg Asn Gly Gly Gln
        355                 360                 365

gcc tca tcg tct cct aat tat gaa gga ccc tta cac tct ttg caa agc 1152
Ala Ser Ser Ser Pro Asn Tyr Glu Gly Pro Leu His Ser Leu Gln Ser

    370                 375                 380

cga att gaa gat cgt tta gaa aga ctg gat gat gct att cat gtt ctc 1200
Arg Ile Glu Asp Arg Leu Glu Arg Leu Asp Asp Ala Ile His Val Leu
385                 390                 395                 400

cgg aac cat gca gtg ggc cca tcc aca gct atg cct ggt ggt cat ggg 1248
Arg Asn His Ala Val Gly Pro Ser Thr Ala Met Pro Gly Gly His Gly
                405                 410                 415

gac atg cat gga atc att gga cct tct cat aat gga gcc atg ggt ggt 1296
Asp Met His Gly Ile Ile Gly Pro Ser His Asn Gly Ala Met Gly Gly
            420                 425                 430

ctg ggc tca ggg tat gga acc ggc ctt ctt tca gcc aac aga cat tca 1344
Leu Gly Ser Gly Tyr Gly Thr Gly Leu Leu Ser Ala Asn Arg His Ser
        435                 440                 445

ctc atg gtg ggg acc cat cgt gaa gat ggc gtg gcc ctg aga ggc agc 1392
Leu Met Val Gly Thr His Arg Glu Asp Gly Val Ala Leu Arg Gly Ser
    450                 455                 460

cat tct ctt ctg cca aac cag gtt ccg gtt cca cag ctt cct gtc cag 1440
His Ser Leu Leu Pro Asn Gln Val Pro Val Pro Gln Leu Pro Val Gln
465                 470                 475                 480

tct gcg act tcc cct gac ctg aac cca ccc cag gac cct tac aga ggc 1488

Ser Ala Thr Ser Pro Asp Leu Asn Pro Pro Gln Asp Pro Tyr Arg Gly
                485                 490                 495

atg cca cca gga cta cag ggg cag agt gtc tcc tct ggc agc tct gag 1536
Met Pro Pro Gly Leu Gln Gly Gln Ser Val Ser Ser Gly Ser Ser Glu
            500                 505                 510

atc aaa tcc gat gac gag ggt gat gag aac ctg caa gac acg aaa tct 1584
Ile Lys Ser Asp Asp Glu Gly Asp Glu Asn Leu Gln Asp Thr Lys Ser
        515                 520                 525

tcg gag gac aag aaa tta gat gac gac aag aag gat atc aaa tca att 1632
Ser Glu Asp Lys Lys Leu Asp Asp Asp Lys Lys Asp Ile Lys Ser Ile
    530                 535                 540

act agc aat aat gac gat gag gac ctg aca cca gag cag aag gca gag 1680
Thr Ser Asn Asn Asp Asp Glu Asp Leu Thr Pro Glu Gln Lys Ala Glu
545                 550                 555                 560

cgt gag aag gag cgg agg atg gcc aac aat gcc cga gag cgt ctg cgg 1728
Arg Glu Lys Glu Arg Arg Met Ala Asn Asn Ala Arg Glu Arg Leu Arg
                565                 570                 575

gtc cgt gac atc aac gag gct ttc aaa gag ctc ggc cgc atg gtg cag 1776
Val Arg Asp Ile Asn Glu Ala Phe Lys Glu Leu Gly Arg Met Val Gln
            580                 585                 590

ctc cac ctc aag agt gac aag ccc cag acc aag ctc ctg atc ctc cac 1824
Leu His Leu Lys Ser Asp Lys Pro Gln Thr Lys Leu Leu Ile Leu His
        595                 600                 605

cag gcg gtg gcc gtc atc ctc agt ctg gag cag caa gtc cga gaa agg 1872
Gln Ala Val Ala Val Ile Leu Ser Leu Glu Gln Gln Val Arg Glu Arg
    610                 615                 620

aat ctg aat ccg aaa gct gcg tgt ctg aaa aga agg gag gaa gag aag 1920
Asn Leu Asn Pro Lys Ala Ala Cys Leu Lys Arg Arg Glu Glu Glu Lys
625                 630                 635                 640

gtg tcc tca gag cct ccc cct ctc tcc ttg gcc ggc cca cac cct gga 1968
Val Ser Ser Glu Pro Pro Pro Leu Ser Leu Ala Gly Pro His Pro Gly
                645                 650                 655

atg gga gac gca tcg aat cac atg gga cag atg taa 2004
Met Gly Asp Ala Ser Asn His Met Gly Gln Met
            660                 665

<210> 10
<211> 667
<212> PRT
<213> Homo sapiens
<400> 10

Met His His Gln Gln Arg Met Ala Ala Leu Gly Thr Asp Lys Glu Leu

1               5                   10                  15

Ser Asp Leu Leu Asp Phe Ser Ala Met Phe Ser Pro Pro Val Ser Ser

            20                  25                  30

Gly Lys Asn Gly Pro Thr Ser Leu Ala Ser Gly His Phe Thr Gly Ser

        35                  40                  45

Asn Val Glu Asp Arg Ser Ser Ser Gly Ser Trp Gly Asn Gly Gly His

    50                  55                  60

Pro Ser Pro Ser Arg Asn Tyr Gly Asp Gly Thr Pro Tyr Asp His Met

65                  70                  75                  80

Thr Ser Arg Asp Leu Gly Ser His Asp Asn Leu Ser Pro Pro Phe Val

                85                  90                  95

Asn Ser Arg Ile Gln Ser Lys Thr Glu Arg Gly Ser Tyr Ser Ser Tyr

            100                 105                 110

Gly Arg Glu Ser Asn Leu Gln Gly Cys His Gln Gln Ser Leu Leu Gly

        115                 120                 125

Gly Asp Met Asp Met Gly Asn Pro Gly Thr Leu Ser Pro Thr Lys Pro

    130                 135                 140

Gly Ser Gln Tyr Tyr Gln Tyr Ser Ser Asn Asn Pro Arg Arg Arg Pro

145                 150                 155                 160

Leu His Ser Ser Ala Met Glu Val Gln Thr Lys Lys Val Arg Lys Val

                165                 170                 175

Pro Pro Gly Leu Pro Ser Ser Val Tyr Ala Pro Ser Ala Ser Thr Ala

            180                 185                 190

Asp Tyr Asn Arg Asp Ser Pro Gly Tyr Pro Ser Ser Lys Pro Ala Thr

        195                 200                 205

Ser Thr Phe Pro Ser Ser Phe Phe Met Gln Asp Gly His His Ser Ser

    210                 215                 220

Asp Pro Trp Ser Ser Ser Ser Gly Met Asn Gln Pro Gly Tyr Ala Gly

225                 230                 235                 240

Met Leu Gly Asn Ser Ser His Ile Pro Gln Ser Ser Ser Tyr Cys Ser

                245                 250                 255

Leu His Pro His Glu Arg Leu Ser Tyr Pro Ser His Ser Ser Ala Asp

            260                 265                 270

Ile Asn Ser Ser Leu Pro Pro Met Ser Thr Phe His Arg Ser Gly Thr

        275                 280                 285

Asn His Tyr Ser Thr Ser Ser Cys Thr Pro Pro Ala Asn Gly Thr Asp

    290                 295                 300

Ser Ile Met Ala Asn Arg Gly Ser Gly Ala Ala Gly Ser Ser Gln Thr
305                 310                 315                 320

Gly Asp Ala Leu Gly Lys Ala Leu Ala Ser Ile Tyr Ser Pro Asp His
                325                 330                 335

Thr Asn Asn Ser Phe Ser Ser Asn Pro Ser Thr Pro Val Gly Ser Pro
            340                 345                 350

Pro Ser Leu Ser Ala Gly Thr Ala Val Trp Ser Arg Asn Gly Gly Gln
        355                 360                 365

Ala Ser Ser Ser Pro Asn Tyr Glu Gly Pro Leu His Ser Leu Gln Ser
    370                 375                 380

Arg Ile Glu Asp Arg Leu Glu Arg Leu Asp Asp Ala Ile His Val Leu
385                 390                 395                 400

Arg Asn His Ala Val Gly Pro Ser Thr Ala Met Pro Gly Gly His Gly
                405                 410                 415

Asp Met His Gly Ile Ile Gly Pro Ser His Asn Gly Ala Met Gly Gly
            420                 425                 430

Leu Gly Ser Gly Tyr Gly Thr Gly Leu Leu Ser Ala Asn Arg His Ser

        435                 440                 445

Leu Met Val Gly Thr His Arg Glu Asp Gly Val Ala Leu Arg Gly Ser
    450                 455                 460

His Ser Leu Leu Pro Asn Gln Val Pro Val Pro Gln Leu Pro Val Gln
465                 470                 475                 480

Ser Ala Thr Ser Pro Asp Leu Asn Pro Pro Gln Asp Pro Tyr Arg Gly
                485                 490                 495

Met Pro Pro Gly Leu Gln Gly Gln Ser Val Ser Ser Gly Ser Ser Glu
            500                 505                 510

Ile Lys Ser Asp Asp Glu Gly Asp Glu Asn Leu Gln Asp Thr Lys Ser
        515                 520                 525

Ser Glu Asp Lys Lys Leu Asp Asp Asp Lys Lys Asp Ile Lys Ser Ile
    530                 535                 540

Thr Ser Asn Asn Asp Asp Glu Asp Leu Thr Pro Glu Gln Lys Ala Glu
545                 550                 555                 560

Arg Glu Lys Glu Arg Arg Met Ala Asn Asn Ala Arg Glu Arg Leu Arg
                565                 570                 575

Val Arg Asp Ile Asn Glu Ala Phe Lys Glu Leu Gly Arg Met Val Gln

            580                 585                 590

Leu His Leu Lys Ser Asp Lys Pro Gln Thr Lys Leu Leu Ile Leu His

        595                 600                 605

Gln Ala Val Ala Val Ile Leu Ser Leu Glu Gln Gln Val Arg Glu Arg

    610                 615                 620

Asn Leu Asn Pro Lys Ala Ala Cys Leu Lys Arg Arg Glu Glu Glu Lys

625                 630                 635                 640

Val Ser Ser Glu Pro Pro Pro Leu Ser Leu Ala Gly Pro His Pro Gly

                645                 650                 655

Met Gly Asp Ala Ser Asn His Met Gly Gln Met

            660                 665

<210> 11

<211> 2085

<212> DNA

<213> Homo sapiens

<220>

<221> CDS

<222> (1)..(2085)

<223>

<400> 11

atg ccg gag ttc ctg gaa gac ccc tcg gtc ctg aca aaa gac aag ttg 48

Met Pro Glu Phe Leu Glu Asp Pro Ser Val Leu Thr Lys Asp Lys Leu
1               5                   10                  15

aag agt gag ttg gtc gcc aac aat gtg acg ctg ccg gcc ggg gag cag     96
Lys Ser Glu Leu Val Ala Asn Asn Val Thr Leu Pro Ala Gly Glu Gln
            20                  25                  30

cgc aaa gac gtg tac gtc cag ctc tac ctg cag cac ctc acg gct cgc     144
Arg Lys Asp Val Tyr Val Gln Leu Tyr Leu Gln His Leu Thr Ala Arg
        35                  40                  45

aac cgg ccg ccg ctc ccc gcc ggc acc aac agc aag ggg ccc ccg gac     192
Asn Arg Pro Pro Leu Pro Ala Gly Thr Asn Ser Lys Gly Pro Pro Asp
    50                  55                  60

ttc tcc agt gac gaa gag cgc gag ccc acc ccg gtc ctc ggc tct ggg     240
Phe Ser Ser Asp Glu Glu Arg Glu Pro Thr Pro Val Leu Gly Ser Gly
65                  70                  75                  80

gcc gcc gcc gcg ggc cgg agc cga gca gcc gtc ggc agg aaa gcc aca     288
Ala Ala Ala Ala Gly Arg Ser Arg Ala Ala Val Gly Arg Lys Ala Thr
                85                  90                  95

aaa aaa act gat aaa ccc aga caa gaa gat aaa gat gat cta gat gta     336
Lys Lys Thr Asp Lys Pro Arg Gln Glu Asp Lys Asp Asp Leu Asp Val
            100                 105                 110

aca gag ctc act aat gaa gat ctt ttg gat cag ctt gtg aaa tac gga 384
Thr Glu Leu Thr Asn Glu Asp Leu Leu Asp Gln Leu Val Lys Tyr Gly
        115                 120                 125

gtg aat cct ggt cct att gtg gga aca acc agg aag cta tat gag aaa 432
Val Asn Pro Gly Pro Ile Val Gly Thr Thr Arg Lys Leu Tyr Glu Lys
    130                 135                 140

aag ctt ttg aaa ctg agg gaa caa gga aca gaa tca aga tct tct act 480
Lys Leu Leu Lys Leu Arg Glu Gln Gly Thr Glu Ser Arg Ser Ser Thr
145                 150                 155                 160

cct ctg cca aca att tct tct tca gca gaa aat aca agg cag aat gga 528
Pro Leu Pro Thr Ile Ser Ser Ser Ala Glu Asn Thr Arg Gln Asn Gly
                165                 170                 175

agt aat gat tct gac aga tac agt gac aat gaa gaa gga aag aag aaa 576
Ser Asn Asp Ser Asp Arg Tyr Ser Asp Asn Glu Glu Gly Lys Lys Lys
            180                 185                 190

gaa cac aag aaa gtg aag tcc act agg gat att gtt cct ttt tct gaa 624
Glu His Lys Lys Val Lys Ser Thr Arg Asp Ile Val Pro Phe Ser Glu
        195                 200                 205

ctt gga act act ccc tct ggt ggt gga ttt ttt cag ggt att tct ttt 672
Leu Gly Thr Thr Pro Ser Gly Gly Gly Phe Phe Gln Gly Ile Ser Phe
    210                 215                 220

cct gaa atc tcc acc cgt cct cct ttg ggc agt acc gaa cta cag gca 720
Pro Glu Ile Ser Thr Arg Pro Pro Leu Gly Ser Thr Glu Leu Gln Ala
225                 230                 235                 240

gct aag aaa gta cat act tct aag gga gac cta cct agg gag cct ctt 768
Ala Lys Lys Val His Thr Ser Lys Gly Asp Leu Pro Arg Glu Pro Leu
                245                 250                 255

gtt gcc aca aac ttg cct ggc agg gga cag ttg cag aag tta gcc tct 816
Val Ala Thr Asn Leu Pro Gly Arg Gly Gln Leu Gln Lys Leu Ala Ser
            260                 265                 270

gaa agg aat ttg ttt att tca tgc aag tct agc cat gat agg tgt tta 864
Glu Arg Asn Leu Phe Ile Ser Cys Lys Ser Ser His Asp Arg Cys Leu
        275                 280                 285

gag aaa agt tct tcg tca tct tct cag cct gaa cac agt gcc atg ttg 912
Glu Lys Ser Ser Ser Ser Ser Ser Gln Pro Glu His Ser Ala Met Leu
    290                 295                 300

gtc tct act gca gct tct cct tca ctg att aaa gaa acc acc act ggt 960
Val Ser Thr Ala Ala Ser Pro Ser Leu Ile Lys Glu Thr Thr Thr Gly
305                 310                 315                 320

tac tat aaa gac ata gta gaa aat att tgc ggt aga gag aaa agt gga 1008
Tyr Tyr Lys Asp Ile Val Glu Asn Ile Cys Gly Arg Glu Lys Ser Gly

                325                 330                 335

att caa cca tta tgt cct gag agg tcc cat att tca gat caa tcg cct 1056

Ile Gln Pro Leu Cys Pro Glu Arg Ser His Ile Ser Asp Gln Ser Pro

            340                 345                 350

ctc tcc agt aaa agg aaa gca cta gaa gag tct gag agc tca caa cta 1104

Leu Ser Ser Lys Arg Lys Ala Leu Glu Glu Ser Glu Ser Ser Gln Leu

        355                 360                 365

att tct ccg cca ctt gcc cag gca atc aga gat tat gtc aat tct ctg 1152

Ile Ser Pro Pro Leu Ala Gln Ala Ile Arg Asp Tyr Val Asn Ser Leu

    370                 375                 380

ttg gtc cag ggt ggg gta ggt agt ttg cct gga act tct aac tct atg 1200

Leu Val Gln Gly Gly Val Gly Ser Leu Pro Gly Thr Ser Asn Ser Met

385                 390                 395                 400

ccc cca ctg gat gta gaa aac ata cag aag aga att gat cag tct aag 1248

Pro Pro Leu Asp Val Glu Asn Ile Gln Lys Arg Ile Asp Gln Ser Lys

                405                 410                 415

ttt caa gaa act gaa ttc ctg tct cct cca aga aaa gtc cct aga ctg 1296

Phe Gln Glu Thr Glu Phe Leu Ser Pro Pro Arg Lys Val Pro Arg Leu

            420                 425                 430

agt gag aag tca gtg gag gaa agg gat tca ggt tcc ttt gtg gca ttt 1344

Ser Glu Lys Ser Val Glu Glu Arg Asp Ser Gly Ser Phe Val Ala Phe
        435                 440                 445

cag aac ata cct gga tcc gaa ctg atg tct tct ttt gcc aaa act gtt 1392
Gln Asn Ile Pro Gly Ser Glu Leu Met Ser Ser Phe Ala Lys Thr Val
    450                 455                 460

gtc tct cat tca ctc act acc tta ggt cta gaa gtg gct aag caa tca 1440
Val Ser His Ser Leu Thr Thr Leu Gly Leu Glu Val Ala Lys Gln Ser
465                 470                 475                 480

cag cat gat aaa ata gat gcc tca gaa cta tct ttt ccc ttc cat gaa 1488
Gln His Asp Lys Ile Asp Ala Ser Glu Leu Ser Phe Pro Phe His Glu
                485                 490                 495

tct att tta aaa gta att gaa gaa gaa tgg cag caa gtt gac agg cag 1536
Ser Ile Leu Lys Val Ile Glu Glu Glu Trp Gln Gln Val Asp Arg Gln
            500                 505                 510

ctg cct tca ctg gca tgc aaa tat cca gtt tct tcc agg gag gca aca 1584
Leu Pro Ser Leu Ala Cys Lys Tyr Pro Val Ser Ser Arg Glu Ala Thr
        515                 520                 525

cag ata tta tca gtt cca aaa gta gat gat gaa atc cta ggg ttt att 1632
Gln Ile Leu Ser Val Pro Lys Val Asp Asp Glu Ile Leu Gly Phe Ile
    530                 535                 540

tct gaa gcc act cca cta gga ggt att caa gca gcc tcc act gag tct 1680
Ser Glu Ala Thr Pro Leu Gly Gly Ile Gln Ala Ala Ser Thr Glu Ser
545                 550                 555                 560

tgc aat cag cag ttg gac tta gca ctc tgt aga gca tat gaa gct gca 1728
Cys Asn Gln Gln Leu Asp Leu Ala Leu Cys Arg Ala Tyr Glu Ala Ala
                565                 570                 575

gca tca gca ttg cag att gca act cac act gcc ttt gta gct aag gct 1776
Ala Ser Ala Leu Gln Ile Ala Thr His Thr Ala Phe Val Ala Lys Ala
            580                 585                 590

atg cag gca gac att agt caa gct gca cag att ctt agc tca gat cct 1824
Met Gln Ala Asp Ile Ser Gln Ala Ala Gln Ile Leu Ser Ser Asp Pro
        595                 600                 605

agt cgt acc cac caa gcg ctt ggg att ctg agc aaa aca tat gat gca 1872
Ser Arg Thr His Gln Ala Leu Gly Ile Leu Ser Lys Thr Tyr Asp Ala
    610                 615                 620

gcc tca tat att tgt gaa gct gca ttt gat gaa gtg aag atg gct gcc 1920
Ala Ser Tyr Ile Cys Glu Ala Ala Phe Asp Glu Val Lys Met Ala Ala
625                 630                 635                 640

cat acc atg gga aat gcc act gta ggt cgt cga tac ctc tgg ctg aag 1968
His Thr Met Gly Asn Ala Thr Val Gly Arg Arg Tyr Leu Trp Leu Lys
                645                 650                 655

gat tgc aaa att aat tta gct tct aag aat aag ctg gct tcc act ccc 2016
Asp Cys Lys Ile Asn Leu Ala Ser Lys Asn Lys Leu Ala Ser Thr Pro
            660                 665                 670

ttt aaa ggt gga aca tta ttt gga gga gaa gta tgc aaa gta att aaa 2064
Phe Lys Gly Gly Thr Leu Phe Gly Gly Glu Val Cys Lys Val Ile Lys
        675                 680                 685

aag cgt gga aat aaa cac tag 2085
Lys Arg Gly Asn Lys His
    690

<210> 12
<211> 694
<212> PRT
<213> Homo sapiens
<400> 12
Met Pro Glu Phe Leu Glu Asp Pro Ser Val Leu Thr Lys Asp Lys Leu
1               5                   10                  15

Lys Ser Glu Leu Val Ala Asn Asn Val Thr Leu Pro Ala Gly Glu Gln
            20                  25                  30

Arg Lys Asp Val Tyr Val Gln Leu Tyr Leu Gln His Leu Thr Ala Arg
        35                  40                  45

Asn Arg Pro Pro Leu Pro Ala Gly Thr Asn Ser Lys Gly Pro Pro Asp
    50                  55                  60
Phe Ser Ser Asp Glu Glu Arg Glu Pro Thr Pro Val Leu Gly Ser Gly
65                  70                  75                  80
Ala Ala Ala Ala Gly Arg Ser Arg Ala Ala Val Gly Arg Lys Ala Thr
                85                  90                  95
Lys Lys Thr Asp Lys Pro Arg Gln Glu Asp Lys Asp Asp Leu Asp Val
            100                 105                 110
Thr Glu Leu Thr Asn Glu Asp Leu Leu Asp Gln Leu Val Lys Tyr Gly
        115                 120                 125
Val Asn Pro Gly Pro Ile Val Gly Thr Thr Arg Lys Leu Tyr Glu Lys
    130                 135                 140
Lys Leu Leu Lys Leu Arg Glu Gln Gly Thr Glu Ser Arg Ser Ser Thr
145                 150                 155                 160
Pro Leu Pro Thr Ile Ser Ser Ser Ala Glu Asn Thr Arg Gln Asn Gly
                165                 170                 175
Ser Asn Asp Ser Asp Arg Tyr Ser Asp Asn Glu Glu Gly Lys Lys Lys
            180                 185                 190

Glu His Lys Lys Val Lys Ser Thr Arg Asp Ile Val Pro Phe Ser Glu
        195                 200                 205
Leu Gly Thr Thr Pro Ser Gly Gly Gly Phe Phe Gln Gly Ile Ser Phe
    210                 215                 220
Pro Glu Ile Ser Thr Arg Pro Pro Leu Gly Ser Thr Glu Leu Gln Ala
225                 230                 235                 240
Ala Lys Lys Val His Thr Ser Lys Gly Asp Leu Pro Arg Glu Pro Leu
                245                 250                 255
Val Ala Thr Asn Leu Pro Gly Arg Gly Gln Leu Gln Lys Leu Ala Ser
            260                 265                 270
Glu Arg Asn Leu Phe Ile Ser Cys Lys Ser Ser His Asp Arg Cys Leu
        275                 280                 285
Glu Lys Ser Ser Ser Ser Ser Ser Gln Pro Glu His Ser Ala Met Leu
    290                 295                 300
Val Ser Thr Ala Ala Ser Pro Ser Leu Ile Lys Glu Thr Thr Thr Gly
305                 310                 315                 320
Tyr Tyr Lys Asp Ile Val Glu Asn Ile Cys Gly Arg Glu Lys Ser Gly
                325                 330                 335

Ile Gln Pro Leu Cys Pro Glu Arg Ser His Ile Ser Asp Gln Ser Pro
            340                 345                 350
Leu Ser Ser Lys Arg Lys Ala Leu Glu Glu Ser Glu Ser Ser Gln Leu
        355                 360                 365
Ile Ser Pro Pro Leu Ala Gln Ala Ile Arg Asp Tyr Val Asn Ser Leu
    370                 375                 380
Leu Val Gln Gly Gly Val Gly Ser Leu Pro Gly Thr Ser Asn Ser Met
385                 390                 395                 400
Pro Pro Leu Asp Val Glu Asn Ile Gln Lys Arg Ile Asp Gln Ser Lys
                405                 410                 415
Phe Gln Glu Thr Glu Phe Leu Ser Pro Pro Arg Lys Val Pro Arg Leu
            420                 425                 430
Ser Glu Lys Ser Val Glu Glu Arg Asp Ser Gly Ser Phe Val Ala Phe
        435                 440                 445
Gln Asn Ile Pro Gly Ser Glu Leu Met Ser Ser Phe Ala Lys Thr Val
    450                 455                 460
Val Ser His Ser Leu Thr Thr Leu Gly Leu Glu Val Ala Lys Gln Ser
465                 470                 475                 480

Gln His Asp Lys Ile Asp Ala Ser Glu Leu Ser Phe Pro Phe His Glu
                485                 490                 495
Ser Ile Leu Lys Val Ile Glu Glu Glu Trp Gln Gln Val Asp Arg Gln
            500                 505                 510
Leu Pro Ser Leu Ala Cys Lys Tyr Pro Val Ser Ser Arg Glu Ala Thr
        515                 520                 525
Gln Ile Leu Ser Val Pro Lys Val Asp Asp Glu Ile Leu Gly Phe Ile
    530                 535                 540
Ser Glu Ala Thr Pro Leu Gly Gly Ile Gln Ala Ala Ser Thr Glu Ser
545                 550                 555                 560
Cys Asn Gln Gln Leu Asp Leu Ala Leu Cys Arg Ala Tyr Glu Ala Ala
                565                 570                 575
Ala Ser Ala Leu Gln Ile Ala Thr His Thr Ala Phe Val Ala Lys Ala
            580                 585                 590
Met Gln Ala Asp Ile Ser Gln Ala Ala Gln Ile Leu Ser Ser Asp Pro
        595                 600                 605
Ser Arg Thr His Gln Ala Leu Gly Ile Leu Ser Lys Thr Tyr Asp Ala
    610                 615                 620

Ala Ser Tyr Ile Cys Glu Ala Ala Phe Asp Glu Val Lys Met Ala Ala
625                 630                 635                 640

His Thr Met Gly Asn Ala Thr Val Gly Arg Arg Tyr Leu Trp Leu Lys
                645                 650                 655

Asp Cys Lys Ile Asn Leu Ala Ser Lys Asn Lys Leu Ala Ser Thr Pro
            660                 665                 670

Phe Lys Gly Gly Thr Leu Phe Gly Gly Glu Val Cys Lys Val Ile Lys
        675                 680                 685

Lys Arg Gly Asn Lys His
    690

<210>  13
<211>  21
<212>  PRT
<213>  Homo sapiens
<220>
<221>  MISC_FEATURE
<223>  Partial sequence of IPF1, showing high score in the local alignme
       nt between IPF1 and HNF3G, No.1
<400>  13
Pro Pro Gly Leu Ser Ala Ser Pro Gln Pro Ser Ser Val Ala Pro Arg
1               5                   10                  15

Arg Pro Gln Glu Pro
            20

<210> 14
<211> 8
<212> PRT
<213> Homo sapiens
<220>
<221> MISC_FEATURE
<223> Partial sequence of IPF1, showing high score in the local alignme
      nt between IPF1 and HNF3G, No.2
<400> 14

Pro Glu Gly Ala Glu Pro Gly Val
1               5

<210> 15
<211> 12
<212> PRT
<213> Homo sapiens
<220>
<221> MISC_FEATURE
<223> Partial sequence of IPF1, showing high score in the local alignme
      nt between IPF1 and HNF3G, No.3

<400> 15

Pro Pro Pro His Pro Phe Pro Gly Ala Leu Gly Ala
1               5                   10

<210>  16
<211>  22
<212>  PRT
<213>  Homo sapiens
<220>
<221>  MISC_FEATURE
<223>  Partial sequence of HNF3G, showing high score in the local alignm
       ent between IPF1 and HNF3G, No.1
<400>  16
Pro Gly Gly Leu Pro Ala Ser Pro Leu Pro Ser Gly Pro Leu Ala Pro
1               5                   10                  15

Pro Ala Pro Ala Ala Pro
            20

<210>  17
<211>  8
<212>  PRT
<213>  Homo sapiens
<220>
<221>  MISC_FEATURE
<223>  Partial sequence of HNF3G, showing high score in the local alignm
       ent between IPF1 and HNF3G, No.2
<400>  17

Ala Glu Gly Gly Glu Pro Gly Val
1               5

<210> 18
<211> 12
<212> PRT
<213> Homo sapiens
<220>
<221> MISC_FEATURE
<223> Partial sequence of HNF3G, showing high score in the local alignm
      ent between IPF1 and HNF3G, No.3
<400> 18
Pro Leu Ser Ser Pro Tyr Pro Gly Gly Leu Pro Ala
1               5                   10

<210> 19
<211> 23
<212> PRT
<213> Homo sapiens
<220>
<221> MISC_FEATURE
<223> Partial sequence of IPF1, showing high score in the local alignme
      nt between IPF1 and PHF1, No.1
<400> 19
His Pro Phe Pro Gly Ala Leu Gly Ala Leu Glu Gln Gly Ser Pro Pro
1               5                   10                  15

Asp Ile Ser Pro Tyr Glu Val

            20

<210> 20

<211> 9

<212> PRT

<213> Homo sapiens

<220>

<221> MISC_FEATURE

<223> Partial sequence of IPF1, showing high score in the local alignme

nt between IPF1 and PHF1, No.2

<400> 20

Ala Val Thr Ser Gly Glu Glu Leu Leu

1               5

<210> 21

<211> 23

<212> PRT

<213> Homo sapiens

<220>

<221> MISC_FEATURE

<223> Partial sequence of PHF1, showing high score in the local alignme

nt between IPF1 and PHF1, No.1

<400> 21

His Pro Ser Ala Ser Thr Ala Gly Thr Ser Gly Asp Ser Gly Pro Pro

1               5                   10                  15

Asp Arg Ser Pro Leu Glu Leu

            20

<210> 22

<211> 9

<212> PRT

<213> Homo sapiens

<220>

<221> MISC_FEATURE

<223> Partial sequence of PHF1, showing high score in the local alignme

nt between IPF1 and PHF1, No. 2

<400> 22

Ala Ala Leu Pro Gly Glu Glu Leu Leu

1               5

<210> 23

<211> 57

<212> PRT

<213> Homo sapiens

<220>

<221> MISC_FEATURE

<223> Partial sequence of IPF1, showing high score in the local alignme

nt between IPF1 and DLX4, No. 1

<400> 23

Lys Arg Thr Arg Thr Ala Tyr Thr Arg Ala Gln Leu Leu Glu Leu Glu

1               5                   10                  15

Lys Glu Phe Leu Phe Asn Lys Tyr Ile Ser Arg Pro Arg Arg Val Glu

            20                  25                  30

Leu Ala Val Met Leu Asn Leu Thr Glu Arg His Ile Lys Ile Trp Phe

        35                  40                  45

Gln Asn Arg Arg Met Lys Trp Lys Lys

    50                  55

<210> 24

<211> 15

<212> PRT

<213> Homo sapiens

<220>

<221> MISC_FEATURE

<223> Partial sequence of IPF1, showing high score in the local alignme

nt between IPF1 and DLX4, No.2

<400> 24

Ser Pro Gln Pro Ser Ser Val Ala Pro Arg Arg Pro Gln Glu Pro

1               5                   10                  15

<210> 25

<211> 57

<212> PRT

<213> Homo sapiens

<220>

<221> MISC_FEATURE

<223>  Partial sequence of DLX4, showing high score in the local alignme
       nt between IPF1 and DLX4, No.1

<400>  25

Arg Lys Pro Arg Thr Ile Tyr Ser Ser Leu Gln Leu Gln His Leu Asn
1               5                   10                  15

Gln Arg Phe Gln His Thr Gln Tyr Leu Ala Leu Pro Glu Arg Ala Gln
            20                  25                  30

Leu Ala Ala Gln Leu Gly Leu Thr Gln Thr Gln Val Lys Ile Trp Phe
        35                  40                  45

Gln Asn Lys Arg Ser Lys Tyr Lys Lys
    50                  55

<210>  26

<211>  12

<212>  PRT

<213>  Homo sapiens

<220>

<221>  MISC_FEATURE

<223>  Partial sequence of DLX4, showing high score in the local alignme
       nt between IPF1 and DLX4, No.2

<400>  26

Ser Pro Glu Pro Ser Glu Arg Arg Pro Gln Ala Pro
1               5                   10

<210> 27

<211> 15

<212> PRT

<213> Homo sapiens

<220>

<221> MISC_FEATURE

<223> Partial sequence of IPF1, showing high score in the local alignme

nt between IPF1 and TCF4, No.1

<400> 27

His His His Leu Pro Ala Gln Leu Ala Leu Pro His Pro Pro Ala

1               5                   10                  15

<210> 28

<211> 16

<212> PRT

<213> Homo sapiens

<220>

<221> MISC_FEATURE

<223> Partial sequence of IPF1, showing high score in the local alignme

nt between IPF1 and TCF4, No.2

<400> 28

Arg Leu Pro Pro Gly Leu Ser Ala Ser Pro Gln Pro Ser Ser Val Ala

1               5                   10                  15

<210> 29

<211> 26

<212> PRT

<213>  Homo sapiens

<220>

<221>  MISC_FEATURE

<223>  Partial sequence of IPF1, showing high score in the local alignme

       nt between IPF1 and TCF4, No.3

<400>  29

Gly Pro Ala Pro Glu Phe Ser Ala Ser Pro Pro Ala Cys Leu Tyr Met

1               5                   10                  15

Gly Arg Gln Pro Pro Pro Pro Pro Pro His

            20                  25

<210>  30

<211>  15

<212>  PRT

<213>  Homo sapiens

<220>

<221>  MISC_FEATURE

<223>  Partial sequence of TCF4 showing high score in the local alignmen

       t between IPF1 and TCF4, No.1

<400>  30

His Ser Leu Leu Pro Asn Gln Val Pro Val Pro Gln Leu Pro Val

1               5                   10                  15

<210>  31

<211>  16

<212>  PRT

<213>  Homo sapiens

<220>

<221>  MISC_FEATURE

<223>  Partial sequence of TCF4 showing high score in the local alignmen

       t between IPF1 and TCF4, No.2

<400>  31

Lys Val Pro Pro Gly Leu Pro Ser Ser Val Tyr Ala Pro Ser Ala Ser

1               5                   10                  15


<210>  32

<211>  26

<212>  PRT

<213>  Homo sapiens

<220>

<221>  MISC_FEATURE

<223>  Partial sequence of TCF4 showing high score in the local alignmen

       t between IPF1 and TCF4, No.3

<400>  32

Gly Ser Pro Pro Ser Leu Ser Ala Gly Thr Ala Val Trp Ser Arg Asn

1               5                   10                  15


Gly Gly Gln Ala Ser Ser Ser Pro Asn Tyr

            20                  25


<210>  33

<211>  14

<212>  PRT

<213>  Homo sapiens

<220>

<221>  MISC_FEATURE

<223>  Partial sequence of IPF1 showing high score in the local alignmen

       t between IPF1 and TMPO

<400>  33

Phe Gln Arg Gly Pro Ala Pro Glu Phe Ser Ala Ser Pro Pro

1               5                   10

<210>  34

<211>  14

<212>  PRT

<213>  Homo sapiens

<220>

<221>  MISC_FEATURE

<223>  Partial sequence of TMPO showing high score in the local alignmen

       t between IPF1 and TMPO

<400>  34

Phe Gln Gly Ile Ser Phe Pro Glu Ile Ser Thr Arg Pro Pro

1               5                   10

<210>  35

<211>  37

<212>  DNA

<213>  Artificial Sequence

<220>

<223>  Desined oligonucleotide based on sequence of SEQ ID NO: 3 for use

as a primer (Human HNF3G)

<400> 35

gctagcctgg gctcagtgaa gatggaggcc catgacc 37

<210> 36

<211> 37

<212> DNA

<213> Artificial Sequence

<220>

<223> Desined oligonucleotide based on sequence of SEQ ID NO: 3 for use

as a reverse primer (Human HNF3G)

<400> 36

ctcgagctag gatgcattaa gcaaagagcg ggaatag 37

<210> 37

<211> 26

<212> DNA

<213> Artificial Sequence

<220>

<223> Desined oligonucleotide based on sequence of SEQ_ID NO:5 for use

as a primer (Human PHF1)

<400> 37

gcgctagcgc gcagcccccc cggctg 26

<210> 38

<211> 26

<212> DNA

<213>  Artificial Sequence

<220>

<223>  Desined oligonucleotide based on sequence of SEQ_ID NO:5 for use

       as a reverse primer (Human PHF1)

<400>  38

gcctcgagtc agaagatgcc ccctcc                                          26

<210>  39

<211>  29

<212>  DNA

<213>  Artificial Sequence

<220>

<223>  Desined oligonucleotide based on sequence of SEQ ID NO: 7 for use

        as a primer (Human DLX4)

<400>  39

acctgcagag caccctcagg aactcgagg                                       29

<210>  40

<211>  29

<212>  DNA

<213>  Artificial Sequence

<220>

<223>  Desined oligonucleotide based on sequence of SEQ ID NO: 7 for use

        as a reverse primer (Human DLX4)

<400>  40

gacagtctca actccagcca atggctctc                                       29

<210> 41

<211> 31

<212> DNA

<213> Artificial Sequence

<220>

<223> Desined oligonucleotide based on mouse HNF3G gene for use as a pr

imer

<400> 41

tgcctgtaga gagaccgaag cactcggttc c 31

<210> 42

<211> 30

<212> DNA

<213> Artificial Sequence

<220>

<223> Desined oligonucleotide based on mouse HNF3G gene for use as a re

verse primer

<400> 42

agctaatggg tcatatcaca gtaatagacc 30

<210> 43

<211> 30

<212> DNA

<213> Artificial Sequence

<220>

<223> Desined oligonucleotide based on mouse PHF1 gene for use as a pri

mer

<400> 43

gagactcatc caggtctctt gagtctgacc 30

<210> 44

<211> 30

<212> DNA

<213> Artificial Sequence

<220>

<223> Desined oligonucleotide based on mouse PHF1 gene for use as a rev

erse primer

<400> 44

tccaggtaca tcccagctgt aggtgaggtc 30

<210> 45

<211> 29

<212> DNA

<213> Artificial Sequence

<220>

<223> Desined oligonucleotide based on mouse DLX4 gene for use as a pri

mer

<400> 45

gctggcagaa agagtagaca gcgtgcggg 29

<210> 46

<211> 27

<212> DNA

<213>  Artificial Sequence

<220>

<223>  Desined oligonucleotide based on mouse DLX4 gene for use as a rev

       erse primer

<400>  46

agcaggtgct cgggtcctgg gcttgac                                          27

<210>  47

<211>  11

<212>  PRT

<213>  Homo sapiens

<220>

<221>  MISC_FEATURE

<223>  Partial sequence of IPF1 showing high score in the local alignmen

       t between IPF1 and HNF3G, No.4

<400>  47

Pro Pro Gly Leu Ser Ala Ser Pro Gln Pro Ser

1               5                   10

<210>  48

<211>  7

<212>  PRT

<213>  Homo sapiens

<220>

<221>  MISC_FEATURE

<223>  Partial sequence of IPF1 showing high score in the local alignmen

       t between IPF1 and HNF3G, No.5

<400> 48

Glu Gly Ala Glu Pro Gly Val

1               5

<210> 49

<211> 8

<212> PRT

<213> Homo sapiens

<220>

<221> MISC_FEATURE

<223> Partial sequence of IPF1 showing high score in the local alignmen

t between IPF1 and HNF3G, No.6

<400> 49

Pro Phe Pro Gly Ala Leu Gly Ala

1               5

<210> 50

<211> 11

<212> PRT

<213> Homo sapiens

<220>

<221> MISC_FEATURE

<223> Partial sequence of HNF3G showing high score in the local alignme

nt between IPF1 and HNF3G, No.4

<400> 50

Pro Gly Gly Leu Pro Ala Ser Pro Leu Pro Ser

1               5                   10

<210> 51

<211> 7

<212> PRT

<213> Homo sapiens

<220>

<221> MISC_FEATURE

<223> Partial sequence of HNF3G showing high score in the local alignme

nt between IPF1 and HNF3G, No.5

<400> 51

Glu Gly Gly Glu Pro Gly Val

1               5

<210> 52

<211> 8

<212> PRT

<213> Homo sapiens

<220>

<221> MISC_FEATURE

<223> Partial sequence of HNF3G showing high score in the local alignme

nt between IPF1 and HNF3G, No.6

<400> 52

Pro Tyr Pro Gly Gly Leu Pro Ala

1               5

<210> 53

<211>  8

<212>  PRT

<213>  Homo sapiens

<220>

<221>  MISC_FEATURE

<223>  Partial sequence of IPF1 showing high score in the local alignmen

       t between IPF1 and PHF1, No.3

<400>  53

Pro Pro Asp Ile Ser Pro Tyr Glu

1               5


<210>  54

<211>  5

<212>  PRT

<213>  Homo sapiens

<220>

<221>  MISC_FEATURE

<223>  Partial sequence of IPF1 showing high score in the local alignmen

       t between IPF1 and PHF1, No.4

<400>  54

Gly Glu Glu Leu Leu

1               5


<210>  55

<211>  8

<212>  PRT

<213>  Homo sapiens

<220>

<221> MISC_FEATURE

<223> Partial sequence of PHF1 showing high score in the local alignmen

t between IPF1 and PHF1, No.3

<400> 55

Pro Pro Asp Arg Ser Pro Leu Glu

1               5

<210> 56

<211> 5

<212> PRT

<213> Homo sapiens

<220>

<221> MISC_FEATURE

<223> Partial sequence of PHF1 showing high score in the local alignmen

t between IPF1 and PHF1, No.4

<400> 56

Gly Glu Glu Leu Leu

1               5

<210> 57

<211> 14

<212> PRT

<213> Homo sapiens

<220>

<221> MISC_FEATURE

<223> Partial sequence of IPF1 showing high score in the local alignmen

t between IPF1 and DLX4, No.3

<400> 57

Ile Lys Ile Trp Phe Gln Asn Arg Arg Met Lys Trp Lys Lys

1               5                   10

<210> 58

<211> 5

<212> PRT

<213> Homo sapiens

<220>

<221> MISC_FEATURE

<223> Partial sequence of IPF1 showing high score in the local alignmen

t between IPF1 and DLX4, No.4

<400> 58

Ser Pro Gln Pro Ser

1               5

<210> 59

<211> 6

<212> PRT

<213> Homo sapiens

<220>

<221> MISC_FEATURE

<223> Partial sequence of IPF1 showing high score in the local alignmen

t between IPF1 and DLX4, No.5

<400> 59

Arg Arg Pro Gln Glu Pro

1                 5

<210>  60

<211>  14

<212>  PRT

<213>  Homo sapiens

<220>

<221>  MISC_FEATURE

<223>  Partial sequence of DLX4 showing high score in the local alignmen

       t between IPF1 and DLX4, No.3

<400>  60

Val Lys Ile Trp Phe Gln Asn Lys Arg Ser Lys Tyr Lys Lys

1                 5                   10

<210>  61

<211>  5

<212>  PRT

<213>  Homo sapiens

<220>

<221>  MISC_FEATURE

<223>  Partial sequence of DLX4 showing high score in the local alignmen

       t between IPF1 and DLX4, No.4

<400>  61

Ser Pro Glu Pro Ser

1                 5

<210>  62

<211> 6

<212> PRT

<213> Homo sapiens

<220>

<221> MISC_FEATURE

<223> Partial sequence of DLX4 showing high score in the local alignmen

t between IPF1 and DLX4, No.5

<400> 62

Arg Arg Pro Gln Ala Pro

1               5

<210> 63

<211> 7

<212> PRT

<213> Homo sapiens

<220>

<221> MISC_FEATURE

<223> Partial sequence of IPF1 showing high score in the local alignmen

t between IPF1 and TCF4, No.4

<400> 63

His His His Leu Pro Ala Gln

1               5

<210> 64

<211> 7

<212> PRT

<213> Homo sapiens

<220>

<221> MISC_FEATURE

<223> Partial sequence of IPF1 showing high score in the local alignmen

t between IPF1 and TCF4, No.5

<400> 64

Pro Pro Gly Leu Ser Ala Ser

1               5

<210> 65

<211> 8

<212> PRT

<213> Homo sapiens

<220>

<221> MISC_FEATURE

<223> Partial sequence of IPF1 showing high score in the local alignmen

t between IPF1 and TCF4, No.6

<400> 65

Gly Pro Ala Pro Glu Phe Ser Ala

1               5

<210> 66

<211> 7

<212> PRT

<213> Homo sapiens

<220>

<221> MISC_FEATURE

<223> Partial sequence of TCF4 showing high score in the local alignmen

t between IPF1 and TCF4, No.4

<400> 66

His Ser Leu Leu Pro Asn Gln

1               5

<210> 67

<211> 7

<212> PRT

<213> Homo sapiens

<220>

<221> MISC_FEATURE

<223> Partial sequence of TCF4 showing high score in the local alignmen

t between IPF1 and TCF4, No.5

<400> 67

Pro Pro Gly Leu Pro Ser Ser

1               5

<210> 68

<211> 8

<212> PRT

<213> Homo sapiens

<220>

<221> MISC_FEATURE

<223> Partial sequence of TCF4 showing high score in the local alignmen

t between IPF1 and TCF4, No.6

<400> 68

Gly Ser Pro Pro Ser Leu Ser Ala

1                    5

<210>  69

<211>  14

<212>  PRT

<213>  Homo sapiens

<220>

<221>  MISC_FEATURE

<223>  Partial sequence of IPF1 showing high score in the local alignmen

       t between IPF1 and TMPO, No.2

<400>  69

Phe Gln Arg Gly Pro Ala Pro Glu Phe Ser Ala Ser Pro Pro

1                    5                        10

<210>  70

<211>  14

<212>  PRT

<213>  Homo sapiens

<220>

<221>  MISC_FEATURE

<223>  Partial sequence of TMPO showing high score in the local alignmen

       t between IPF1 and TMPO, No.2

<400>  70

Phe Gln Gly Ile Ser Phe Pro Glu Ile Ser Thr Arg Pro Pro

1                    5                        10

## Description

### Technical Field

The present invention relates to a method for regulating insulin gene transcription. More specifically, the present invention relates to a method for regulating insulin gene transcription, which comprises the step of inhibiting binding of insulin promoter factor 1 (hereinafter, abbreviated as "IPF1" in the specification) and a protein that binds to IPF1.

### Background Art

IPF1, a transcription factor expressed in β-cells of the pancreas, is a factor for promoting expressions of genes important for glycometabolism such as insulin, glucokinase, and GLUT2 (as reviews, see, Diabetologia, 44, 1203-1214, 2001; Eur. J. Endocrinol., 146, 129-141, 2002; Diabetologia, 45, 309-326, 2002). Deficiency of IPF1 function causes abnormal glycometabolism and results in onset of hereditary type 2 diabetes, i.e., maturity-onset diabetes of the young (MODY4, J. Clin. Invest., 104, R41-R48, 1999). Therefore, IPF1 is considered to be an important factor for glycometabolic system and functional maintenance of the pancreas such as insulin secretion.

It has been reported that IPF1 is phosphorylated via the signal transduction systems of phosphatidylinositol 3-kinase and stress-activated protein kinase, which is triggered by glucose stimulation, and then translocated into the nucleus to accelerate insulin gene promoter activity (J. Biol. Chem. 272, 20936-20944, 1997; J. Biol. Chem. 274, 1011-1016, 1999). However, any enzyme that catalyzes the phosphorylation of IPF1 has not yet been identified so far. Further, it has been reported that IPF1-dependent insulin gene promoter activity is inhibited by hepatocyte nuclear factor-1α (HNF-1α, Endocr. Res. 27, 63-74, 2001), and this inhibition is considered to be caused by competitive inhibition by HNF-1α against the binding of IPF1 to the insulin gene promoter region. However, it has not yet been clarified so far whether or not IPF1 and HNF-1 α directly bind to each other.

Several factors regulating gene transcription are known. For example, hepatocyte nuclear factor 3-gamma (HNF3G) is a transcription factor having a forkhead box and is considered to be a transcription-regulating factor of liver-specific genes (Genomics, 20, 377-385, 1994; Genomics, 39, 417-419, 1997; Mol. Cell. Biol., 18, 4245-4251, 1998). Distal-less homeobox 4 (DLX4, also sometimes called as BP1) is a transcription factor containing a homeodomain and is known to inhibit transcription of β -globin gene (Mol. Cell. Biol., 22, 2505-2514, 2002). Transcription factor 4 (TCF4) is a transcription factor having a helix-loop-helix structure and is known to bind to an initiator element of somatostatin receptor II gene or an enhancer elements of immunoglobulin genes to activate transcription (EMBO J., 15, 6680-6690; Science, 247, 467-470). Further, PHD finger protein 1 (PHF1) is a protein having a PHD finger domain and is speculated to be a transcription-regulating factor, although functions thereof remain unknown (Genomics, 48, 381-383, 1998). However, it has never been known so far that these transcription factors are factors for regulating insulin gene transcription. Furthermore, thymopoietin (TMPO), a class of nucleoprotein, is considered to possibly participate in maintenance of a nuclear structure and cell cycle. However, details of functions thereof remained unclarified (Genomics, 28, 198-205, 1995; Genome Res., 6, 361-370, 1996). It has not been reported that this protein is involved in insulin gene transcription.

### Disclosure of the Invention

As explained above, IPF1 is an important transcription promoting factor in insulin gene transcription. Accordingly, identification of a protein that binds to IPF1 is extremely important for providing a means for prophylactic and/or therapeutic treatment of diabetes. Further, a means for regulating insulin gene transcription may possibly be provided on the basis of inhibition of the binding of IPF1 and the protein. Therefore, an object of the present invention is to provide a protein that binds to IPF1, and further provide a means for regulating insulin gene transcription by inhibiting the binding of IPF1 and the protein.

In order to identify a protein that binds to IPF1 and elucidate functions thereof, the inventors of the present invention divided the amino acid sequence of IPF1 into oligopeptides each having given lengths, and searched databases for proteins that have the amino acid sequences of those oligopeptides or amino acid sequences homologous to the amino acid sequences, and then according to the prediction method described in International Patent Publication WO01/67299, they performed local alignments of the selected proteins and IPF1 and predicted that proteins giving a high local alignment score are proteins that successfully bind to IPF1. As a result, they found several types of proteins which contains oligopeptides having homology to the oligopeptides consisting of IPF1-derived amino acid sequences. Further, the inventors of the present invention found that these proteins successfully bind to IPF1, and found a method for regulating insulin gene transcription on the basis of the binding. They also found that these proteins have inhibitory actions on insulin promoter activity. The present invention was achieved on the basis of the above findings.

The present invention thus provides a method for promoting insulin gene transcription, which comprises the step of inhibiting binding of IPF1 and any one of protein selected from the following group:
(i) hepatocyte nuclear factor 3-gamma (HNF3G),
(ii) PHD finger protein 1 (PHF1), and
(iii) distal-less homeobox 4 (DLX4).

Further, the present invention also provides a method for screening a substance that inhibits binding of IPF1 and any one of protein selected from the following group, which comprises the step of bringing a test substance into contact with IPF1 and/or the protein under a condition that allows binding of IPF1 and said protein and then determining whether or not the test substance inhibits the binding of IPF1 and said protein by detecting the presence or absence, or change of a signal and/or marker generated by binding of IPF1 and said protein in a system in which the signal and/or the marker can be detected:
(i) HNF3G,
(ii) PHF1,
(iii) DLX4,
(iv) transcription factor4 (TCF4), and
(v) thymopoietin (TMPO).

Further, the present invention also provides a method for screening a substance that promotes insulin gene transcription, which comprises the step of bringing a test substance into contact with IPF1 and/or any one of protein selected from the following group under a condition that allows binding of IPF1 and said protein and then determining whether or not the test substance inhibits the binding of IPF1 and said protein by detecting the presence or absence, or change of a signal and/or marker generated by binding of IPF1 and said protein in a system in which the signal and/or the marker can be detected:
(i) HNF3G,
(ii) PHF1, and
(iii) DLX4.

The present invention also provides a method for screening a substance that promotes insulin gene transcription, which comprises the step of bringing a test substance into contact with IPF1 and/or any one of protein selected from the following group under a condition that allows binding of IPF1 and said protein to determine whether or not insulin gene transcription is promoted:
(i) HNF3G,
(ii) PHF1, and
(iii) DLX4.

From another aspect, the present invention provides a substance screened by any of the above screening methods.

The present invention also provides a medicament for prophylactic treatment and/or therapeutic treatment of a disease caused by a reduced amount of gene product of the insulin gene, which medicament inhibits binding of IPF1 and any one of protein selected from the following group:
(i) HNF3G,
(ii) PHF1, and
(iii) DLX4; and
a medicament for prophylactic treatment and/or therapeutic treatment of diabetes, which inhibits binding of IPF1 and any one of protein selected from the following group :
(i) HNF3G,
(ii) PHF1, and
(iii) DLX4.

According to a preferred embodiment of these medicaments, the present invention provides a medicament containing, as an active ingredient, a substance screened by any of the aforementioned screening methods.

The present invention further provides a method for prophylactic treatment and/or therapeutic treatment of a disease caused by a reduced amount of gene product of the insulin gene, which comprises the step of inhibiting binding of IPF1 and any one of protein selected from the following group:
(i) HNF3G,
(ii) PHF1, and
(iii) DLX4; and
a method for prophylactic treatment and/or therapeutic treatment of diabetes, which comprises the step of inhibiting binding of IPF1 and any one of protein selected from the following group:
(i) HNF3G,
(ii) PHF1, and
(iii) DLX4.

According to a preferred embodiment of these methods, the present invention provides a method comprising the step of administering a substance screened by any of the aforementioned screening methods.

From a further aspect, the present invention provides a kit of reagents used for any of the aforementioned screening methods, which comprises:
(a) IPF1 and/or a DNA encoding IPF1, and
(b) a protein binding to IPF1 and/or a DNA encoding said protein.

According to a preferred embodiment, the present invention provides a kit of reagents, which comprises:
(a) IPF1 and/or a DNA encoding IPF1, and
(b) one or more kinds of proteins selected from the following group and/or DNAs encoding said proteins:
   (i) HNF3G,
   (ii) PHF1, and
   (iii) DLX4.

### Brief Description of the Drawings

Fig. 1 shows results of local alignments of oligopeptides PPGLSASPQPS, EGAEPGV and PFPGALGA consisting of IPF1-derived amino acid residues with PGGLPASPLPS, EGGEPGV and PYPGGLPA which are homologous oligopeptides in HNF3G.
Fig. 2 shows results of local alignments of oligopeptides PPDISPYE and GEELL consisting of IPF1-derived amino acid residues with PPDRSPLE and GEELL which are homologous oligopeptides in PHF1,
Fig. 3 shows results of local alignments of oligopeptides IKIWFQNRRMKWKK, SPQPS and RRPQEP consisting of IPF1-derived amino acid residues with VKIWFQNKRSKYKK, SPEPS and RRPQAP which are homologous oligopeptides in DLX4.
Fig. 4 shows results of local alignments of oligopeptides HHHLPAQ, PPGLSAS and GPAPEFSA consisting of IPF1-derived amino acid residues with HSLLPNQ, PPGLPSS and GSPPSLSA which are homologous oligopeptides in TCF4.
Fig. 5 shows results of local alignment of an oligopeptide FQRGPAPEFSASPP consisting of IPF1-derived amino acid residues with FQGISFPEISTRPP which is a homologous oligopeptide in TMPO.
Fig. 6 shows results of a binding test of IPF1 to HNF3G, PHF1, DLX4, TCF4 or TMPO.
Fig. 7 shows results of detection of IPF1-dependent human insulin promoter activity in a HeLa cell system.
Fig. 8 shows effect of overexpression of HNF3G, PHF1 and DLX4 on IPF1-dependent human insulin promoter activity in a HeLa cell system.
Fig. 9 shows effect of overexpression of HNF3G, PHF1 and DLX4 on human insulin promoter activity in an MIN6 cell system.
Fig. 10 shows results of confirmation of expressions of human HNF3G, human PHF1 and human DLX4 in human pancreas (RT-PCR).
Fig. 11 shows results of confirmation of expressions of mouse HNF3G, mouse PHF1 and mouse DLX4 in MIN6 cells (RT-PCR).

### Best Mode for Carrying out the Invention

The proteins (i) to (v) used in the present invention (also referred to as "wild type interactive proteins") are proteins represented by the amino acid sequences of SEQ ID NOS: 4, 6, 8, 10 and 12 in the sequence listing, respectively. IPF1 used in the present invention (also referred to as "wild type IPF1") is a protein represented by the amino acid sequence of SEQ ID NO: 2 in the sequence listing. Those skilled in the art can easily obtain the wild type interactive proteins and wild type IPF1 in view of these descriptions. For example, the proteins can be recovered and purified from samples in which production of these proteins are observed (for example, cells derived from the human pancreas) by a purification method known per se (affinity chromatography by using a monoclonal antibody recognizing each protein as an antigen).

Further, in the present specification, the proteins (i) to (v) encompass proteins which have amino acid sequences including substitution, insertion or deletion of one to several amino acid in the amino acid sequence of the aforementioned wild type interactive proteins and have substantially the same insulin gene transcription regulating action as that of the aforementioned wild type interactive proteins, or having substantially the same insulin promoter activity inhibitory action as that of the aforementioned wild type interactive proteins in mammals in vivo including a human (these proteins are also referred to as "mutant type interactive proteins").

Further, in the specification, IPF1 encompasses proteins which have amino acid sequences including substitution, insertion or deletion of one to several amino acids in the amino acid sequence of the aforementioned wild type IPF1 and have substantially the same insulin promoter activation action as that of the aforementioned wild type IPF1 or have substantially the same insulin gene transcription promoting action as that of the aforementioned wild type IPF1 in mammals in vivo including a human (these proteins are also referred to as "mutant type IPF1").

In general, the mutant type interactive proteins preferably have an amino acid sequence having homology higher than a given level to the amino acid sequences of the wild type interactive proteins (for example, 70% or higher, preferably 80% or higher, more preferably 85% or higher, further preferably 90% or higher, and most preferably 95% or higher). Similarly, the mutant IPF1 preferably has an amino acid sequence having homology higher than a given level to the amino acid sequence of the wild type IPF1 (for example, 70% or higher, preferably 80% or higher, more preferably 85% or higher, further preferably 90% or higher, and most preferably 95% or higher).

Methods for obtaining genes encoding these mutant proteins are known. For example, the genes can be appropriately obtained by the methods described in Molecular Cloning: A Laboratory Manual (Ed. by Sambrook et al., Cold Spring Harbor Laboratory Press, Cold Spring Harbor, New York, 1989) and the like or similar methods thereto, and desired mutant proteins can be easily obtained. Whether or not a mutant protein obtained has substantially the same insulin gene transcription regulating action substantially as that of the wild type interactive proteins can be easily examined by those skilled in the art by using the method for detecting inhibitory action on insulin promoter activity specifically described in Example 3 of the present specification. Further, whether or not a mutant protein obtained has the same insulin promoter activation action as that of the wild type IPF1 can be easily examined by those skilled in the art by using the method for determining insulin promoter activity specifically described in Example 3 of the present specification.

The insulin gene transcription promotion method provided by the present invention is characterized to comprise the step of inhibiting binding of IPF1 with any one of protein selected from the group consisting of the aforementioned proteins (i), (ii) and (iii). Although it is not intended to be bound by any specific theory, the aforementioned proteins (i), (ii) and (iii) that bind to IPF1 are generally expected to inhibit the insulin promoter activity of IPF1. Therefore, a substance inhibiting said binding can activate the insulin promoter and thereby promote the insulin gene transcription by inhibiting the insulin promoter activity suppressing action of the aforementioned protein. The type of the aforementioned substance inhibiting said binding is not particularly limited, and examples thereof include low molecular compounds such as organic compounds, inorganic compounds, and sugar compounds, as well as high molecular compounds such as proteins including antibodies, nucleic acids including antisense nucleic acids, polysaccharides, and lipids. Further, the substance may be a natural or a non-natural substance.

In the specification, the binding of IPF1 with a certain protein means an interaction of IPF1 with the protein via a non-covalent bond such as hydrogen bond, hydrophobic bond, and electrostatic interaction so that IPF1 and the protein can form a complex. As for the "binding" herein referred to, the binding of IPF1 with said protein as a whole is sufficient. For example, amino acids constituting IPF1 or the protein may include amino acids which are not involved in the binding of IPF1 and said protein.

The binding of IPF1 with said protein can be detected by methods known per se such as identification of coprecipitates by an immunoprecipitation technique, two-hybrid analysis, pull-down analysis, Western blotting, and fluorescence resonance energy transfer, or any combination of these methods.

A substance having the aforementioned inhibitory action can be screened by, for example, the following method provided by the present invention. This method is for screening a substance that inhibits the binding of IPF1 with any one of proteins selected from the group consisting of the aforementioned proteins (i) to (v), and the method is characterized to comprise the step of bringing a test substance into contact with IPF1 and/or the protein under a condition that allows the binding of IPF1 and the protein, and then detecting the presence or absence, or change of a signal and/or marker generated by the binding of IPF1 with the protein in a system in which the signal and/or the marker can be detected to determine whether or not the test substance inhibits the binding of IPF1 and the protein. According to a preferred embodiment of the aforementioned method, a substance that inhibits the binding of IPF1 with any one of proteins selected from the group consisting of the aforementioned proteins (i), (ii) and (iii) can be screened.

The condition that allows the binding of IPF1 with said protein in the aforementioned step means, for example, a condition wherein IPF1 and the protein are co-expressed in a cell. Such a condition can be satisfied by transfecting a suitable vector, which is incorporated with polynucleotides coding for IPF1 and the protein, into a cell by a conventional genetic engineering technique.

The signal generated by the binding of IPF1 and the protein in the aforementioned step means a signal that is generated by the binding of IPF1 with the protein and can be directly detected on the basis of a physical or chemical property of the signal, per se. The marker generated by the binding of IPF1 and the protein means a marker that is generated by binding of IPF1 with the protein and can be indirectly detected by using a physical or biological property of the marker, per se, as an index. Examples of the signaling substances include luciferase, radioactive isotopes and the like. Examples of the markers include reporter genes such as the chloramphenicol acetyltransferase gene or the like, and epitope tags for detection such as 6×His-tag. Methods for detecting these signals or markers are well known to those skilled in the art.

When a test substance is allowed to coexist with IPF1 and the protein under a condition that allows the binding of IPF1 with the protein in the aforementioned step, it can be judged that the test substance inhibits the binding of IPF 1 with the protein if a signal and/or marker generated by the binding of IPF1 and the protein is reduced as compared with a step where the test substance is not allowed to coexist, or the signal and/or marker disappears.

The type of the test substance used in the screening method of the present invention is not particularly limited, and any compound can be used as the test substance. The test substance may be any of low molecular compounds such as organic compounds, inorganic compounds, and sugar compounds, as well as high molecular compounds such as proteins, nucleic acids, polysaccharides, and lipids. Further, the substance may be a natural or a non-natural substance. Examples of a library that is subjected to the screening include a low molecular compound library, phage display library, combinatorial library and the like. However, the libraries are not limited to these examples.

In the aforementioned method, the step of detecting the presence or absence, or change of a signal and/or marker generated by the binding of IPF1 with the protein in a system in which the signal and/or the marker can be detected is performed as a step for determining whether or not the insulin gene transcription is promoted. As a result, a substance that promotes the insulin gene transcription can be screened according to the aforementioned method.

The present invention further provides a kit for performing the aforementioned screening. The kit is characterized to comprise (a) IPF1 and/or a DNA encoding IPF1, and (b) a protein that interacts with IPF1 and/or a DNA encoding the protein. The elements of the kit may be provided as proteins, or they may be provided in the forms of genes that can express the proteins, preferably recombinant vectors containing the genes or the like.

A medicament containing a substance screened by the method of the present invention as an active ingredient can be administered to a mammal including a human as a medicament for prophylactic treatment and/or therapeutic treatment of a disease caused by a reduced amount of a gene product of the insulin gene. An example of the disease caused by the reduced amount of the gene product of the insulin gene includes diabetes (including complications of diabetes). The administration route of the medicament of the present invention is not particularly limited, and the medicament can be orally or parenterally administered. As the medicament of the present invention, a substance as the active ingredient, per se, may be used. However, a pharmaceutical composition containing a pharmacologically and pharmaceutically acceptable additive together with the substance as an active ingredient is preferably prepared and administered.

For example, the medicament of the present invention containing a protein as an active ingredient can be prepared by an ordinary method for preparing a protein preparation. The medicament of the present invention containing a nucleic acid as an active ingredient can also be prepared by a means available in this field. In the specification, the term "nucleic acid" encompasses DNA and RNA. The medicament of the present invention containing a nucleic acid can be used, for example, in the form of a recombinant vector containing the nucleic acid as an active ingredient. The aforementioned recombinant vector may be incorporated with various sequences required to express the gene or promote gene expression in a suitable order so that a gene product can be efficiently expressed in vivo from the nucleic acid as an active ingredient. When the medicament of the present invention contains an antisense nucleic acid, the nucleic acid may be either DNA or RNA, and a total length thereof is not particularly limited. The antisense nucleic acid may be, for example, an oligonucleotide of 10 nucleotides or more, preferably 15 nucleotides or more, so as to allow complementary binding. When an antibody, preferably a monoclonal antibody, which can bind to the aforementioned protein is used as an active ingredient of the medicament of the present invention, the antibody can be produced by an ordinary method, and a monoclonal antibody that can specifically bind to the aforementioned protein can also be produced by a method generally used by those skilled in the art.

Examples of the pharmacologically and pharmaceutically acceptable additives include excipients, disintegrating agents or disintegrating aids, binders, lubricants, coating agents, dyes, diluents, bases, dissolving agents or dissolving aids, isotonic agents, pH modifiers, stabilizers, propellants, adhesion agents and the like. Examples of pharmaceutical compositions suitable for oral administration include tablets, capsules, powders, subtilized granules, granules, solutions, syrups and the like. Examples of pharmaceutical compositions suitable for parenteral administration include injections, drip infusions, suppositories, inhalants, transdermal preparations, eye drops, ear drops, ointments, creams, patches and the like. Doses of the medicament of the present invention are not particularly limited, and can be suitably selected depending on various conditions such as the type of the substance as an active ingredient, the purpose of therapeutic or preventive treatment, the age and symptom of a patient, and the route of administration. In general, doses can be selected from the range of about 0.001 to 1000 mg per day for an adult.

The present invention also provides a method for prophylactic and/or therapeutic treatment of a disease caused by a reduced amount of a gene product of the insulin gene. The method is characterized by inhibiting the binding of IPF1 and a protein that binds to the IPF1 (preferably, HNF3G, PHF1 or DLX4). An example of the disease caused by the reduced amount of the gene product of the insulin gene includes diabetes. The method can be implemented by using the aforementioned medicament.

### Examples

The present invention will be explained more specifically with reference to the following examples. However, the scope of the present invention is not limited to these examples.

### Example 1: Prediction of proteins interacting with IPF1

Proteins interacting with IPF1 were predicted according to the prediction method described in International Patent Publication WO01/67299. The amino acid sequence of IPF1 was divided into oligopeptides having suitable lengths. Proteins having amino acid sequences of these oligopeptides or amino acid sequences with homology to the amino acid sequences of the above oligopeptides were searched in databases, and local alignment was performed for each of the obtained proteins and IPF1. The proteins that gave a high local alignment score was predicted to interact with IPF1. The high local alignment score was defined as a score of 25.0 or higher as in the method described in International Patent Publication WO01/67299.

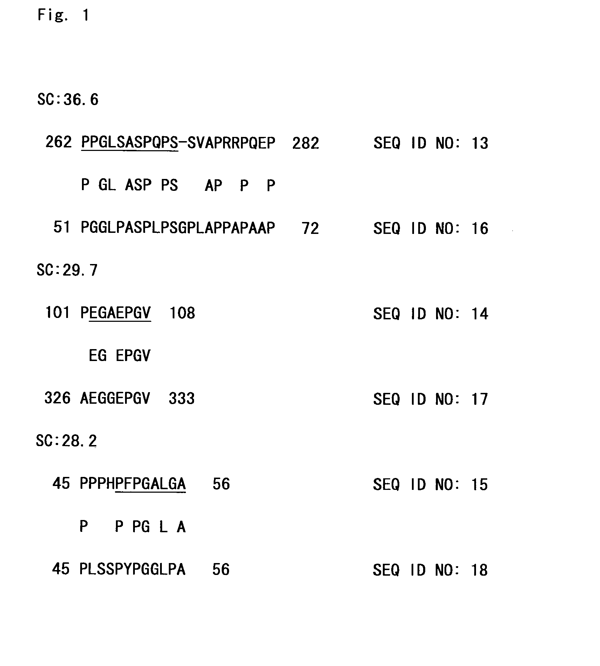

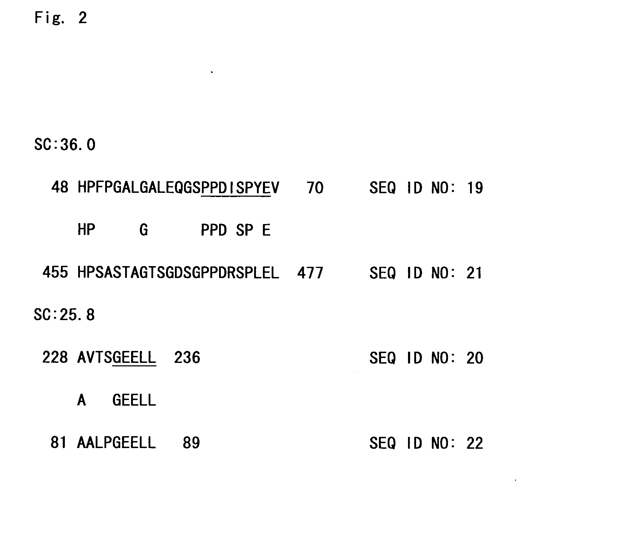

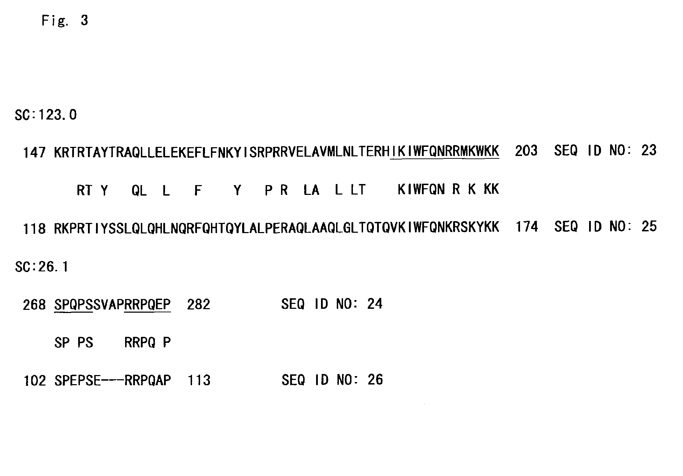

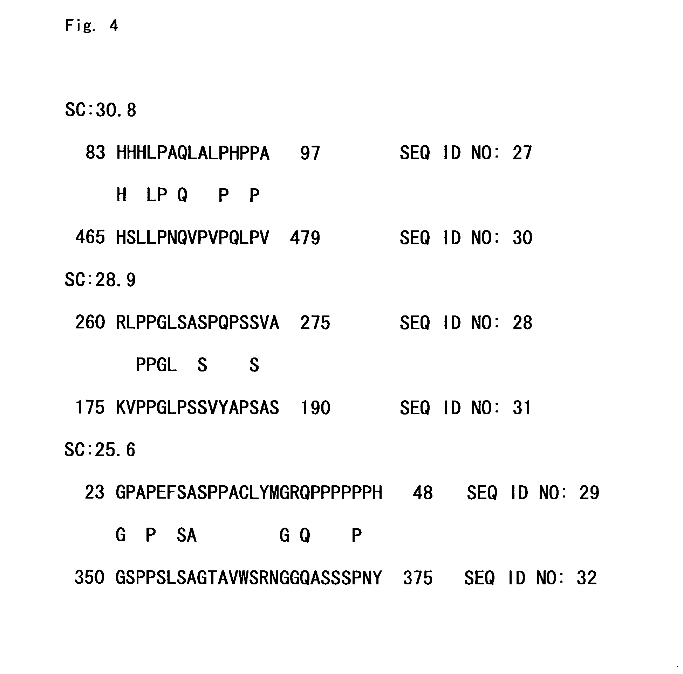

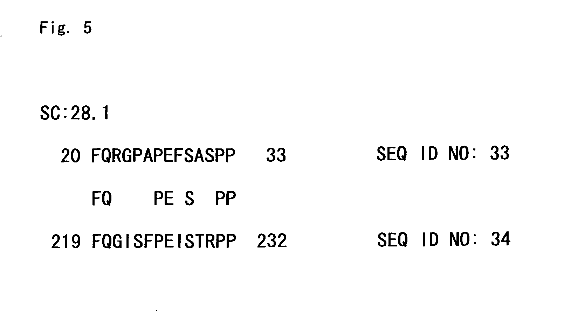

As a result of the prediction, the oligopeptides of SEQ ID NOS: 50, 51 and 52 having homology to the oligopeptides of SEQ ID NOS: 47, 48 and 49, each consisting of IPF1-derived amino acid residues, were found to exist in the amino acid sequence of HNF3G. The result of the local alignment of IPF1 and HNF3G is shown in Fig. 1. Further, the oligopeptides of SEQ ID NOS: 55 and 56 having homology to the oligopeptides of SEQ ID NOS: 53 and 54, each consisting of IPF1-derived amino acid residues, were found to exist in the amino acid sequence of PHF1 (Fig. 2), the oligopeptides of SEQ ID NOS: 60, 61 and 62 having homology to the oligopeptides of SEQ ID NOS: 57, 58 and 59, each consisting of IPF1-derived amino acid residues, were found to exist in the amino acid sequence of DLX4 (Fig. 3), the oligopeptides of SEQ ID NOS: 66, 67 and 68 having homology to the oligopeptides of SEQ ID NOS: 63, 64 and 65, each consisting of IPF1-derived amino acid residues, were found to exist in the amino acid sequence of TCF4 (Fig. 4), and the oligopeptide of SEQ ID NO: 70 having homology to the oligopeptide of SEQ ID NO: 69, consisting of IPF1-derived amino acid residues, were found to exist in the amino acid sequence of TMPO (Fig. 5).

### Example 2: Binding test to human IPF1

Studies were made by applying the GST-pull down assay to examine whether or not human IPF1 binds to human HNF3G, human PHF1, human DLX4, human TCF4, or human TMPO.

### <Materials>

### (1) Cloning of each cDNA

Human IPF1 cDNA was cloned from human liver-derived cDNA (Clontech), human HNF3G cDNA was cloned from human liver-derived cDNA (Clontech), human PHF1 cDNA, human TCF4 cDNA, and human TMPO cDNA were cloned from human brain-derived cDNA (Clontech), and human DLX4 cDNA was cloned from human placenta-derived cDNA (Clontech) by PCR.

### (2) Various expression plasmids

Human IPF1 cDNA was introduced into pGEX-4T (Amersham Biosciences), a GST fusion protein expression vector, to construct pGEX-4T/IPF1 as an N-terminus GST-fused IPF1 expression plasmid. Further, each cDNA of human HNF3G, human PHF1, human DLX4, human TCF4, and human TMPO was introduced into pcDNA3.1(+) (Invitrogen), as being a vector for in vitro protein synthesis and expression in animal cells, to construct expression plasmids (pcDNA-HA-HNF3G, pcDNA-HA-PHF1, pcDNA-HA-DLX4, pcDNA-HA-TCF4, and pcDNA-HA-TMPO). In this construction, an HA tag-coding sequence was inserted at the 5' end of each cDNA so as to have each protein expressed as an N-terminus HA-tagged protein. As a LacZ expression plasmid as a negative control, pCruzHA-LacZ (N-terminus HA-tagged LacZ expression plasmid, Santa Cruz) was used.

### (3) Purification of N-terminus GST-fused IPF1 (GST-IPF1)

GST-IPF1 was expressed in *Escherichia coli* containing pGEX-4T/IPF1 and then purified by using Glutathione Sepharose 4B (Amersham Biosciences).

### <Method>

### (1) Binding test by GST-pull down assay

Human HNF3G, human PHF1, human DLX4, human TCF4, human TMPO, and LacZ were synthesized in vitro as ³⁵S-methionine-labeled proteins by using TNT Quick Coupled Transcription/Tanslation Systems (Promega). In a volume of 20 µ l of a reaction mixture for synthesis and 5 µ g of GST-IPF1 or GST were left standing in 500 µ l of a binding buffer (40 mM HEPES, pH 7.5/50 mM KCl/5 mM MgCl₂/0.2 mM EDTA/1 mM DTT/0.5% NP-40) on ice for 1 hour. Then, the mixture was added with 20 µ l (bed volume) of Glutathione Sepharose 4B and mixed gently overnight at 4°C on a rocker, and then the beads were recovered by centrifugation. The beads were washed 4 times with 500 µl of the binding buffer, added with 20 µl of 2 × SDS sample buffer (125 mM Tris-HCl, pH 6.8/4% SDS/20% glycerol/0.01% bromophenol blue) and boiled for 3 minutes, and then the supernatant was separated by 5-20% SDS-PAGE. Then, the binding proteins were detected by using BAS2000 (Fuji Photo Film).

### <Results>

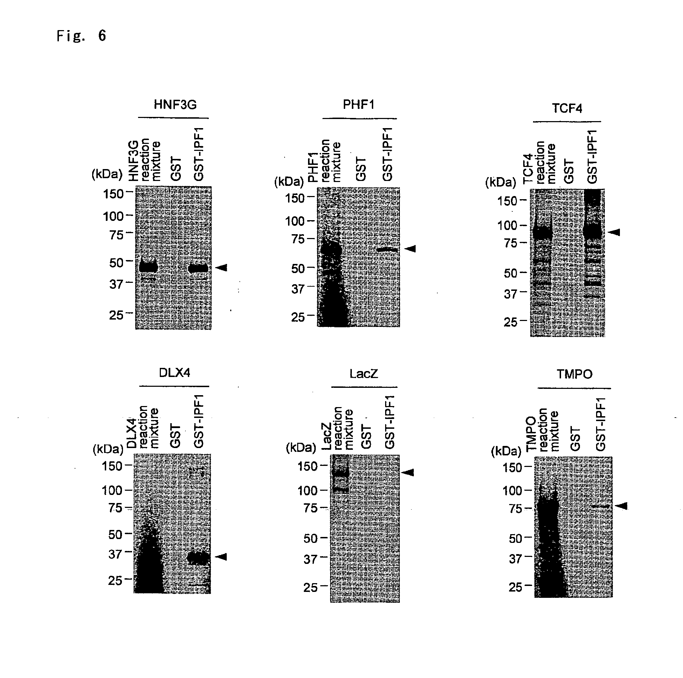

The bindings of IPF1 with HNF3G, PHF1, DLX4, TCF4, and TMPO were observed (Fig. 6). Whilst the binding of LacZ and IPF1 was not observed, which means that the detected binding of IPF1 with each protein was not non-specific (Fig. 6). In Fig. 6, GST (lane of "GST") or GST-IPF1 (lane of "GST-IPF1") was mixed with each protein synthesized as ³²S-methionine-labeled protein (HNF3G, PHF1, DLX4, TCF4, TMPO, and LacZ) (lanes of "input") in an in vitro transcription/translation system, and GST or GST-IPF1 was recovered by using Glutathione Sepharose and separated by SDS-PAGE. Then, the bound proteins were detected by autoradiography. Each arrowhead points at the position of each protein (HNF3G, PHF1, DLX4, TCF4, TMPO, and LacZ) synthesized in the in vitro system.

### Example 3: Inhibition of human insulin gene promoter activity by human HNF3G, human PHF1, and human DLX4

Among human HNF3G, human PHF1, human DLX4, human TCF4, and human TMPO which were found to bind to IPF1, human HNF3G, human PHF1, and human DLX4 were used to examine inhibitory action against human insulin gene promoter activity by using a reporter assay system.

### <Materials>

### (1) Cloning of human insulin gene promoter region and construction of luciferase reporter plasmid

The human insulin gene promoter region (-392/+237, transcription initiation point being defined "+1") was cloned from Human genomic DNA (Clontech) by PCR. The cloned promoter region was introduced into pGL3-Basic (Promega) as a luciferase reporter vector to construct pInsPro(-392/+237)-GL3 as a reporter plasmid for determining human insulin gene promoter activity.

### (2) Various expression plasmids

Human IPF1 cDNA was introduced into pcDNA3.1(+) (Invitrogen) to construct an IPF1 expression plasmid (pcDNA-FLAG-IPF1). In this construction, a FLAG tag coding sequence was inserted into the 5' end of IPF1 cDNA so as to have the protein expressed as an N-terminus FLAG-tagged protein. As expression plasmids for human HNF3G, human PHF1, and human DLX4, pcDNA-HA-HNF3G, pcDNA-HA-PHF1 and pcDNA-HA-DLX4 were used, respectively.

### <Methods>

### (1) Transfection and reporter assay

2 × 10⁵ HeLa cells were inoculated on a 6-well plate on the previous day and the cells were cultured overnight, and then transfection was performed by using FuGENE6 (Roche Diagnostics). As plasmids, 200 ng of pInsPro(-392/+237)-GL3, 400 ng of pcDNA-FLAG-IPF1, and 1 µg each of the expression plasmids (pcDNA-HA-HNF3G, pcDNA-HA-PHF1, and pcDNA-HA-DLX4) as well as 0.5 ng of pRL-SV40 (Promega) as an internal control were used. The total amount of DNA was adjusted with pcDNA3.1(+) (Invitrogen) to 1.6 µg. The cells were cultured for 48 hours, and luciferase activity was determined by using Dual-Luciferase Reporter Assay System (Promega). The measured values were corrected on the basis of the Renilla luciferase activity. When MIN6 cells, cells of mouse insulinoma-derived β -cell strain, were used, 5 × 10⁵ MIN6 cells were inoculated on a 6-well plate on the previous day and cultured overnight, and transfection was performed by using FuGENE6 (Roche Diagnostics). As plasmids, 200 ng of pInsPro(-392/+237)-GL3 and 1 µ g each of the expression plasmids (pcDNA-HA-HNF3G, pcDNA-HA-PHF1, and pcDNA-HA-DLX4) as well as 5 ng of pRL-SV40 as an internal control were used. The total amount of DNA was adjusted with pcDNA3.1(+) (Invitrogen) to 1.2 µg. The cells were cultured for 48 hours, and then the luciferase activity was determined by the same method as described above.

### <Results>

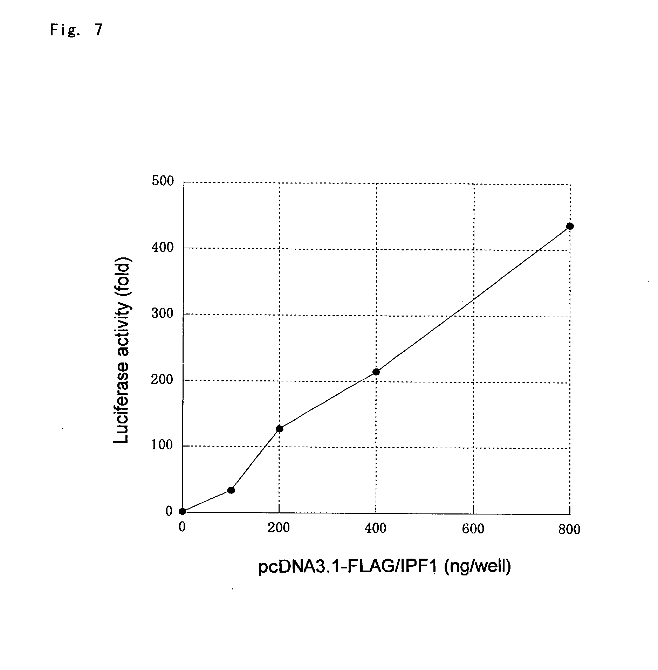

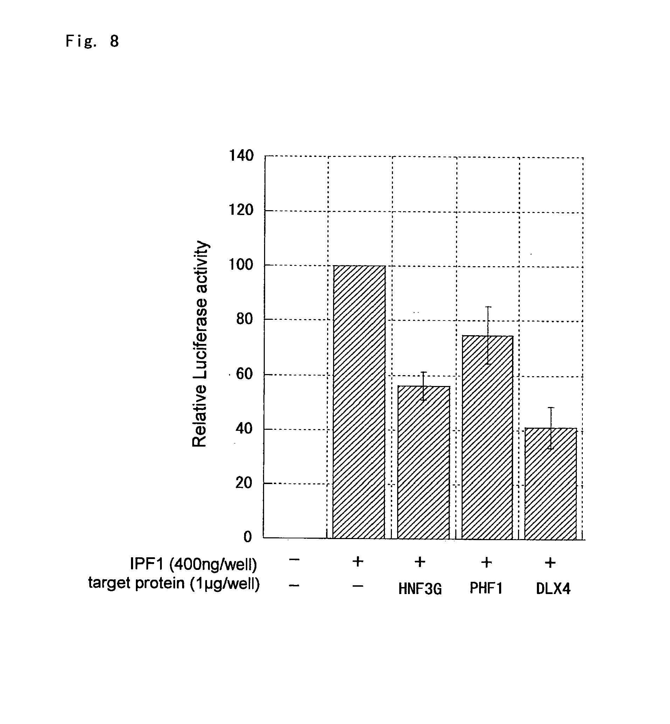

A system for detecting IPF1-dependent human insulin promoter activity was first constructed in which a luciferase reporter system in HeLa cells not expressing IPF1 was used. As a result, increase in human insulin promoter activity dependent on the amount of IPF1 expression plasmid was observed as shown in Fig. 7. In Fig. 7, the vertical axis represents relative luciferase activity based on the luciferase activity without pcDNA-FLAG-IPF1, which is taken as 1, and the horizontal axis represents the amount of introduced pcDNA-FLAG-IPF1. Then, effect of each protein was examined by using this system. As a result, it was revealed that IPF1-dependent human insulin promoter activity was inhibited by about 45%, about 25%, and about 60% due to overexpression of HNF3G, PHF1 and DLX4, respectively (Fig. 8). In Fig. 8, the vertical axis represents relative luciferase activity based on the luciferase activity obtained with introduction of pcDNA-FLAG-IPF1 alone, which was taken as 100. The symbol "-" means that any expression plasmid was not introduced, and "+" means that an expression plasmid was introduced. Further, HNF3G, PHF1, and DLX4 mean that each corresponding expression plasmid was introduced.

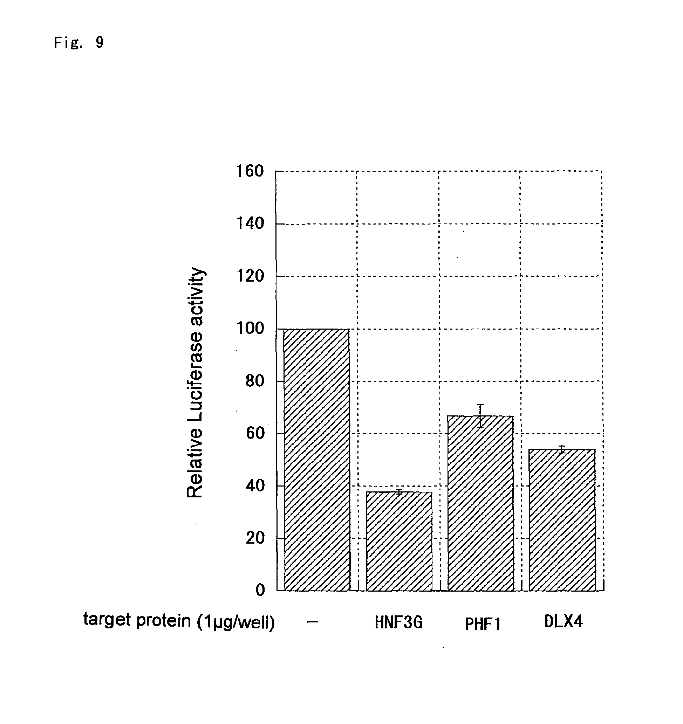

Then, effect of HNF3G, PHF1, and DLX4 on the human insulin promoter activity was examined by a reporter assay using MIN6 cells which are cells of a mouse insulinoma-derived β -cell strain intrinsically expressing IPF1. As a result, it was revealed that human insulin promoter activity was inhibited by about 60%, about 30%, and about 45% due to overexpression of HNF3G, PHF1, and DLX4, respectively (Fig. 9). In Fig. 9, the vertical axis represents relative luciferase activity based on the luciferase activity obtained without introduction of each expression plasmid, which was taken as 100. The symbol "-" means that any expression plasmid was not introduced, and "HNF3G," "PHF1," and "DLX4" mean that each corresponding expression plasmid was introduced. The above results revealed that HNF3G, PHF1, and DLX4 inhibited both of the IPF1-dependent human insulin gene promoter activity in the HeLa cell system and human insulin promoter activity in the MIN6 cell system.

### Example 4: Confirmation of expression of HNF3G, PHF1, and DLX4 in human pancreas and MIN6 cells (RT-PCR)

Studies were made by applying the RT-PCR method to examine whether or not HNF3G, PHF1, and DLX4 were expressed in the human pancreas as the insulin secreting tissue, and in MIN6 cells as cells of a mouse insulinoma derived β -cell strain.

### <Method>

### (1) Confirmation of each mRNA by RT-PCR

cDNAs derived from the human pancreas, human brain, and human placenta were purchased from Invitrogen. As for the mouse MIN6 cells, total RNAs were prepared by using RNeasy Mini Kit (Qiagen) and then used to synthesize cDNAs derived from the MIN6 cells by using RNA PCR Kit (AMV) Ver.2.1 (Takara). PCR was performed by using each cDNA as a template, KOD Plus DNA Polymerase (Toyobo), and primers specific to each gene. Each reaction mixture was subjected to electrophoresis in a 2% agarose gel and stained with ethidium bromide to detect target PCR products.

The primers used were:
for human HNF3G,
oligonucleotide consisting of the nucleotide sequence of SEQ ID NO: 35, and
oligonucleotide consisting of the nucleotide sequence of SEQ ID NO: 36,
for human PHF1,
oligonucleotide consisting of the nucleotide sequence of SEQ ID NO: 37, and
oligonucleotide consisting of the nucleotide sequence of SEQ ID NO: 38,
and for human DLX4.,
oligonucleotide consisting of the nucleotide sequence of SEQ ID NO: 39, and
oligonucleotide consisting of the nucleotide sequence of SEQ ID NO: 40.

Further used are:
for mouse HNF3G,
oligonucleotide consisting of the nucleotide sequence of SEQ ID NO: 41, and
oligonucleotide consisting of the nucleotide sequence of SEQ ID NO: 42,
for mouse PHF1,
oligonucleotide consisting of the nucleotide sequence of SEQ ID NO: 43, and
oligonucleotide consisting of the nucleotide sequence of SEQ ID NO: 44,
and for mouse DLX4,
oligonucleotide consisting of the nucleotide sequence of SEQ ID NO: 45, and
oligonucleotide consisting of the nucleotide sequence of SEQ ID NO: 46.

### <Results>

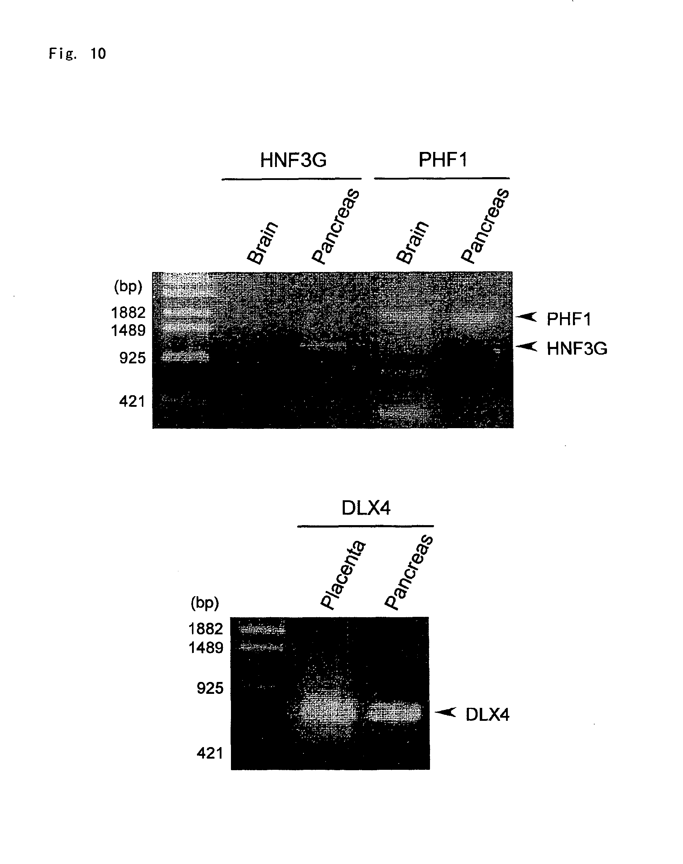

Expressions of HNF3G, PHF1, and DLX4 in the human pancreas as the insulin secreting tissue were examined by RT-PCR. As a result, the presence of mRNA was detected for each gene. Fig. 10 shows the results of PCR using cDNAs derived from the human pancreas (lane of "pancreas"), human brain (lane of "brain") and human placenta (lane of "placenta") as templates and primers specific to each gene (panels of "HNF3G," "PHF1," and "DLX4," respectively). Each reaction mixture was separated by 2% agarose gel electrophoresis, and then target PCR products were detected by ethidium bromide staining. The numbers on the left represent values of size markers (bp), and each arrowhead points at the position of the amplification product derived from each mRNA. "Brain," "Pancreas," and "Placenta" mean that cDNA derived from each organ was used as a template.

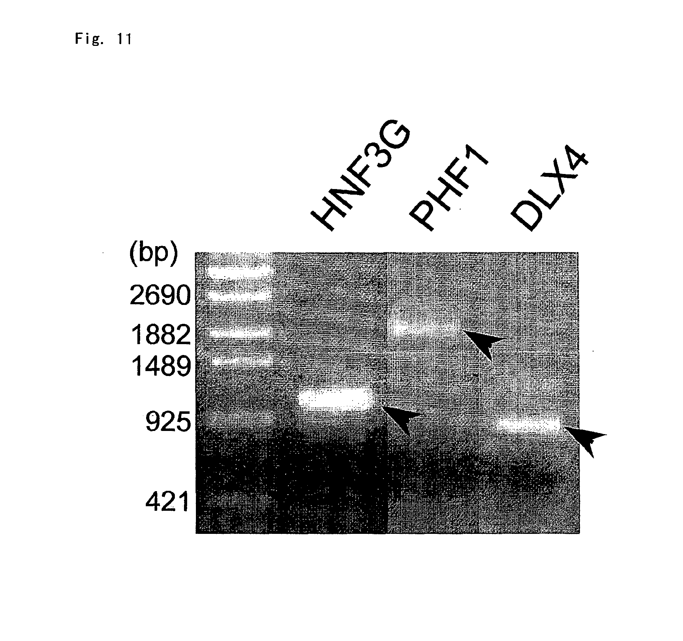

Insulin is secreted from β -cells existing in the pancreas. Expression of each mouse gene in the MIN6 cells, cells of mouse insulinoma derived β -cell strain, was examined by RT-PCR. As a result, the presence of mRNA was detected for each mouse gene. Fig. 11 shows the results of PCR using mouse cDNA derived from MIN6 cells as a template and primers specific to each gene (lanes of "HNF3G," "PHF1," and "DLX4," respectively). Each reaction mixture was separated by 2% agarose gel electrophoresis, and the target PCR products were detected by ethidium bromide staining. The numbers on the left represent values of size markers (bp), and each arrowhead points at the position of the amplification product derived from each mRNA. The primers for detecting each gene were designed so that each of the primers includes intro-exon boundary, and accordingly, the amplified PCR products were not those derived from genomic DNAs.

### Sequence listing free text

SEQ ID NO: 13: Partial IPF1 oligopeptide which gave a high score in the local alignment of IPF1 and HNF3G
SEQ ID NO: 14: Partial IPF1 oligopeptide which gave a high score in the local alignment of IPF1 and HNF3G
SEQ ID NO: 15: Partial IPF1 oligopeptide which gave a high score in the local alignment of IPF1 and HNF3G
SEQ ID NO: 16: Partial HNF3G oligopeptide which gave a high score in the local alignment of IPF1 and HNF3G
SEQ ID NO: 17: Partial HNF3G oligopeptide which gave a high score in the local alignment of IPF1 and HNF3G
SEQ ID NO: 18: Partial HNF3G oligopeptide which gave a high score in the local alignment of IPF1 and HNF3G
SEQ ID NO: 19: Partial IPF1 oligopeptide which gave a high score in the local alignment of IPF1 and PHF1
SEQ ID NO: 20: Partial IPF1 oligopeptide which gave a high score in the local alignment of IPF1 and PHF1
SEQ ID NO: 21: Partial PHF1 oligopeptide which gave a high score in the local alignment of IPF 1 and PHF1
SEQ ID NO: 22: Partial PHF1 oligopeptide which gave a high score in the local alignment of IPF1 and PHF1
SEQ ID NO: 23: Partial IPF1 oligopeptide which gave a high score in the local alignment of IPF1 and DLX4
SEQ ID NO: 24: Partial IPF1 oligopeptide which gave a high score in the local alignment of IPF1 and DLX4
SEQ ID NO: 25: Partial DLX4 oligopeptide which gave a high score in the local alignment of IPF1 and DLX4
SEQ ID NO: 26: Partial DLX4 oligopeptide which gave a high score in the local alignment of IPF1 and DLX4
SEQ ID NO: 27: Partial IPF1 oligopeptide which gave a high score in the local alignment of IPF1 and TCF4
SEQ ID NO: 28: Partial IPF1 oligopeptide which gave a high score in the local alignment of IPF1 and TCF4
SEQ ID NO: 29: Partial IPF1 oligopeptide which gave a high score in the local alignment of IPF1 and TCF4
SEQ ID NO: 30: Partial TCF4 oligopeptide which gave a high score in the local alignment of IPF1 and TCF4
SEQ ID NO: 31: Partial TCF4 oligopeptide which gave a high score in the local alignment of IPF1 and TCF4
SEQ ID NO: 32: Partial TCF4 oligopeptide which gave a high score in the local alignment of IPF1 and TCF4
SEQ ID NO: 33: Partial IPF1 oligopeptide which gave a high score in the local alignment of IPF1 and TMPO
SEQ ID NO: 34: Partial TMPO oligopeptide which gave a high score in the local alignment of IPF1 and TMPO
SEQ ID NO: 35: Primer oligonucleotide designed on the basis of the nucleotide sequence of SEQ ID NO: 3
SEQ ID NO: 36: Primer oligonucleotide designed on the basis of the nucleotide sequence of SEQ ID NO: 3
SEQ ID NO: 37: Primer oligonucleotide designed on the basis of the nucleotide sequence of SEQ ID NO: 5
SEQ ID NO: 38: Primer oligonucleotide designed on the basis of the nucleotide sequence of SEQ ID NO: 5
SEQ ID NO: 39: Primer oligonucleotide designed on the basis of the nucleotide sequence of SEQ ID NO: 7
SEQ ID NO: 40: Primer oligonucleotide designed on the basis of the nucleotide sequence of SEQ ID NO: 7
SEQ ID NO: 41: Primer oligonucleotide designed on the basis of nucleotide sequence of mouse HNF3G gene
SEQ ID NO: 42: Primer oligonucleotide designed on the basis of nucleotide sequence of mouse HNF3G gene
SEQ ID NO: 43: Primer oligonucleotide designed on the basis of nucleotide sequence of mouse PHF1 gene
SEQ ID NO: 44: Primer oligonucleotide designed on the basis of nucleotide sequence of mouse PHF1 gene
SEQ ID NO: 45: Primer oligonucleotide designed on the basis of nucleotide sequence of mouse DLX4 gene
SEQ ID NO: 46: Primer oligonucleotide designed on the basis of nucleotide sequence of mouse DLX4 gene
SEQ ID NO: 47: Partial IPF1 oligopeptide which gave a high score in the local alignment of IPF1 and HNF3G
SEQ ID NO: 48: Partial IPF1 oligopeptide which gave a high score in the local alignment of IPF1 and HNF3G
SEQ ID NO: 49: Partial IPF1 oligopeptide which gave a high score in the local alignment of IPF1 and HNF3G
SEQ ID NO: 50: Partial HNF3G oligopeptide which gave a high score in the local alignment of IPF1 and HNF3G
SEQ ID NO: 51: Partial HNF3G oligopeptide which gave a high score in the local alignment of IPF1 and HNF3G
SEQ ID NO: 52: Partial HNF3G oligopeptide which gave a high score in the local alignment of IPF1 and HNF3G
SEQ ID NO: 53: Partial IPF1 oligopeptide which gave a high score in the local alignment of IPF1 and PHF1
SEQ ID NO: 54: Partial IPF1 oligopeptide which gave a high score in the local alignment of IPF1 and PHF1
SEQ ID NO: 55: Partial PHF1 oligopeptide which gave a high score in the local alignment of IPF1 and PHF1
SEQ ID NO: 56: Partial PHF1 oligopeptide which gave a high score in the local alignment of IPF1 and PHF1
SEQ ID NO: 57: Partial IPF1 oligopeptide which gave a high score in the local alignment of IPF1 and DLX4
SEQ ID NO: 58: Partial IPF1 oligopeptide which gave a high score in the local alignment of IPF1 and DLX4
SEQ ID NO: 59: Partial IPF1 oligopeptide which gave a high score in the local alignment of IPF1 and DLX4
SEQ ID NO: 60: Partial DLX4 oligopeptide which gave a high score in the local alignment of IPF1 and DLX4
SEQ ID NO: 61: Partial DLX4 oligopeptide which gave a high score in the local alignment of IPF1 and DLX4
SEQ ID NO: 62: Partial DLX4 oligopeptide which gave a high score in the local alignment of IPF1 and DLX4
SEQ ID NO: 63: Partial IPF1 oligopeptide which gave a high score in the local alignment of IPF1 and TCF4
SEQ ID NO: 64: Partial IPF1 oligopeptide which gave a high score in the local alignment of IPF1 and TCF4
SEQ ID NO: 65: Partial IPF1 oligopeptide which gave a high score in the local alignment of IPF1 and TCF4
SEQ ID NO: 66: Partial TCF4 oligopeptide which gave a high score in the local alignment of IPF1 and TCF4
SEQ ID NO: 67: Partial TCF4 oligopeptide which gave a high score in the local alignment of IPF1 and TCF4
SEQ ID NO: 68: Partial TCF4 oligopeptide which gave a high score in the local alignment of IPF1 and TCF4
SEQ ID NO: 69: Partial IPF1 oligopeptide which gave a high score in the local alignment of IPF1 and TMPO
SEQ ID NO: 70: Partial TMPO oligopeptide which gave a high score in the local alignment of IPF1 and TMPO

### Industrial Applicability

The present invention provides the proteins (i) to (v) that bind to IPF1, and also provides a means for regulating the insulin gene transcription based on the inhibition of the binding of IPF1 and the aforementioned proteins. By the screening method of the present invention using this means, for example, a substance promoting the insulin gene transcription can be easily screened, and the resulting substance can be used as an active ingredient of a medicament for prophylactic and/or therapeutic treatment of diseases such as diabetes.

## Claims

1. A method for promoting insulin gene transcription, which comprises the step of inhibiting binding of IPF1 and any one of proteins selected from the following group:
(i) HNF3G,
(ii) PHF1,
and
(iii) DLX4.

2. A method for screening a substance that inhibits binding of IPF1 and any one of proteins selected from the following group, which comprises the step of bringing a test substance into contact with IPF1 and/or said protein under a condition that allows the binding of IPF1 and said protein and then determining whether or not the test substance inhibits the binding of IPF1 and said protein by detecting presence or absence, or change of a signal and/or a marker generated by the binding of IPF1 and said protein in a system in which the signal and/or the marker can be detected:
(i) HNF3G,
(ii) PHF1,
(iii) DLX4,
(iv) TCF4,
and
(v) TMPO.

3. A method for screening a substance that promotes insulin gene transcription, which comprises the step of bringing a test substance into contact with IPF1 and/or any one of proteins selected from the following group under a condition that allows the binding of IPF1 and said protein and then determining whether or not the test substance inhibits the binding of IPF1 and said protein by detecting presence or absence, or change of a signal and/or a marker generated by the binding of IPF1 and said protein in a system in which the signal and/or the marker can be detected:
(i) HNF3G,
(ii) PHF1,
and
(iii) DLX4.

4. A method for screening a substance that promotes insulin gene transcription, which comprises the step of bringing a test substance into contact with IPF1 and/or any one of proteins selected from the following group under a condition that allows the binding of IPF1 and said protein to determine whether or not insulin gene transcription is promoted:
(i) HNF3G,
(ii) PHF1,
and
(iii) DLX4.

5. A substance screened by the screening method according to any one of claims 2 to 4.

6. A medicament for prophylactic and/or therapeutic treatment of a disease caused by a reduced amount of a gene product of insulin gene, which inhibits binding of IPF1 and any one of proteins selected from the following group:
(i) HNF3G,
(ii) PHF1,
and
(iii) DLX4.

7. A medicament for prophylactic and/or therapeutic treatment of diabetes, which inhibits binding of IPF1 and any one of proteins selected from the following group:
(i) HNF3G,
(ii) PHF1,
and
(iii) DLX4.

8. The medicament according to claim 6 or 7, which contains the substance according to claim 5 as an active ingredient.

9. A method for prophylactic and/or therapeutic treatment of a disease caused by a reduced amount of a gene product of insulin gene, which comprises the step of inhibiting binding of IPF1 and any one of proteins selected from the following group:
(i) HNF3G,
(ii) PHF1,
and
(iii) DLX4.

10. A method for prophylactic and/or therapeutic treatment of diabetes, which comprises the step of inhibiting binding of IPF1 and any one of proteins selected from the following group:
(i) HNF3G,
(ii) PHF1,
and
(iii) DLX4.

11. The method according to claim 9 or 10, which comprises the step of administering an effective amount of the substance according to claim 5 to a mammal including a human.

12. A kit of reagents used in the screening method according to any one of claims 2 to 4, which comprises:
(a) IPF1 and/or DNA coding for IPF1, and
(b) one or more kinds of proteins selected from the following group and/or DNAs encoding the proteins:
(i) HNF3G,
(ii) PHF1,
and
(iii) DLX4.
